# FASCICULE DE BREVET EUROPEEN

(11) **EP 1 692 114 B1**
(45) Date de publication et mention de la délivrance du brevet: **22.09.2010**
(21) Numéro de dépôt: 04805536.2
(22) Date de dépôt: 24.11.2004
(51) Int. Cl.: C07D 235/12, C07D 409/06, A61K 31/4184, A61P 3/04, A61P 15/00, A61P 25/00, C07D 403/04, C07D 409/12, C07D 405/04, C07D 403/10, C07D 401/04, C07D 403/06, C07D 405/06, C07D 401/06, C07D 413/04

(54) **NOUVEAUX DERIVES DE LA BENZIMIDAZOLE ET D’IMIDAZO-PYRIDINE ET LEUR UTILISATION EN TANT QUE MEDICAMENT**
NEUE BENZIMIDAZOL- UND IMIDAZOPYRIDINDERIVATE UND DEREN VERWENDUNG ALS MEDIKAMENT
NOVEL BENZIMIDAZOLE AND IMIDAZOPYRIDINE DERIVATIVES AND USE THEREOF AS A MEDICAMENT

(30) Priorité: 28.11.2003 FR 0313988
(43) Date de publication de la demande: 23.08.2006
(73) Titulaire: IPSEN PHARMA, 92100 Boulogne-Billancourt (FR)
(72) Inventeur: POITOUT, Lydie, F-94270 le Kremlin Bicêtre (FR); BRAULT, Valérie, F-78730 Saint-Arnoult-en-Yvelines (FR); SACKUR, Carole, F-75004 Paris (FR); ROUBERT, Pierre, F-75014 Paris (FR); PLAS, Pascale, F-92320 Châtillon (FR)
(74) Mandataire: Audonnet, Nathalie
(86) Numéro de dépôt international: PCT/FR2004/003007
(87) Numéro de publication internationale: WO 2005/056533

(56) Documents cités:
- WO-A-01/10842
- WO-A1-02/092575

## Description

La présente demande a pour objet de nouveaux dérivés de benzimidazole et d'imidazo-pyridine. Ces produits ont une bonne affinité pour certains sous-types de récepteurs des mélanocortines, en particulier des récepteurs MC4. Ils sont particulièrement intéressants pour traiter les états pathologiques et les maladies dans lesquels un ou plusieurs récepteurs des mélanocortines sont impliqués. L'invention concerne également des compositions pharmaceutiques contenant lesdits produits et leur utilisation pour la préparation d'un médicament.

Les mélanocortines représentent un groupe de peptides qui dérivent d'un même précurseur, la proopiomélanocortine (POMC), et qui sont structurellement proches : l'hormone adrénocorticotrope (ACTH), l'hormone stimulante des mélanocytes α (α-MSH), la β-MSH et la γ-MSH (Eipper B.A. et Mains R.E., Endocr. Rev. 1980, 1, 1-27). Les mélanocortines exercent de nombreuses fonctions physiologiques. Elles stimulent la synthèse des stéroïdes par la cortico-surrénale et la synthèse d'eumélanine par les mélanocytes. Elles régulent la prise de nourriture, le métabolisme énergétique, la fonction sexuelle, la régénération neuronale, la pression sanguine et la fréquence cardiaque, ainsi que la perception de la douleur, l'apprentissage, l'attention et la mémoire. Les mélanocortines possèdent également des propriétés anti-inflammatoires et anti-pyrétiques et contrôlent la sécrétion de plusieurs glandes endocrines ou exocrines telles les glandes sébacées, lacrymales, mammaires, la prostate et le pancréas (Wikberg J.E. et al. Pharmacol. Res. 2000, 42, 393-420 ; Abdel-Malek Z.A., Cell. Mol. Life. Sci. 2001,58,434-441).

Les effets des mélanocortines sont médiés par une famille de récepteurs membranaires spécifiques à sept domaines transmembranaires et couplés aux protéines G. Cinq sous-types de récepteurs, nommés MC1 à MC5, ont été clonés et caractérisés à ce jour. Ces récepteurs diffèrent par leur distribution tissulaire et par l'affinité des différentes mélanocortines, les récepteurs MC2 ne reconnaissant que l'ACTH. La stimulation des récepteurs des mélanocortines active l'adénylate cyclase avec production d'AMP cyclique. Si les rôles fonctionnels spécifiques de chacun des récepteurs ne sont pas totalement élucidés, le traitement de désordres pathologiques ou de maladies peut être associé à une affinité pour certains sous-types de récepteurs. Ainsi l'activation des récepteurs MC1 a été associée au traitement des inflammations, alors que leur blocage a été associé au traitement de cancers cutanés. Le traitement des troubles de la nutrition a été associé aux récepteurs MC3 et MC4, le traitement de l'obésité par les agonistes et le traitement de la cachexie et de l'anorexie par les antagonistes. D'autres indications associées à l'activation des récepteurs MC3 et MC4 sont les troubles de l'activité sexuelle, les douleurs neuropathiques, l'anxiété, la dépression et les toxicomanies. L'activation des récepteurs MC5 a été associée au traitement de l'acné et des dermatoses.

La demande internationale de brevet WO 2001/10842 décrit des dérivés du tétrahydrobenzimidazole possédant une affinité pour les récepteurs MC4 des mélanocortines.

Les déposants ont trouvé que les nouveaux composés de formule générale (I) décrits ci-après possèdent une bonne affinité pour les récepteurs des mélanocortines. Ils agissent préférentiellement sur les récepteurs MC4. Lesdits composés, agonistes ou antagonistes des récepteurs des mélanocortines, peuvent être utilisés pour traiter les états pathologiques ou les maladies métaboliques, du système nerveux ou dermatologiques dans lesquels un ou plusieurs récepteurs des mélanocortines sont impliqués tels que les exemples suivants : les états inflammatoires, les troubles de l'homéostasie énergétique, de la prise de nourriture, les désordres pondéraux (l'obésité, la cachexie, l'anorexie), les désordres de l'activité sexuelle (les troubles de l'érection), la douleur neuropathique. On peut également citer les troubles mentaux (l'anxiété, la dépression), les toxicomanies, les maladies de la peau (l'acné, les dermatoses, les cancers cutanés, les mélanomes). Ces composés peuvent également être utilisés pour stimuler la régénération nerveuse.

L'invention a donc pour objet un composé de formule générale (**I**) 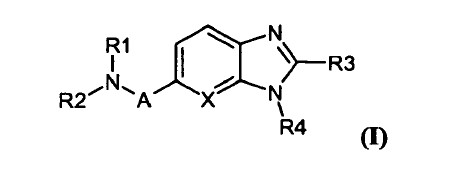 sous forme racémique, d'énantiomère ou toutes combinaisons de ces formes et dans laquelle :
A représente -CH₂- ,-C(O)-, -C(O)-C(Rₐ)(R_{b})- ;
X représente le radical -CH- ou l'atome d'azote ;
Rₐ et R_{b} représentent, indépendamment, l'atome d'hydrogène ou un radical (C₁-C₆)alkyle ;
R₁ représente l'atome d'hydrogène ou un radical (C₁-C₈)alkyle;
R₂ représente un radical (C₁-C₈)alkyle ;
ou bien R₁ et R₂ forment ensemble, avec l'atome d'azote auquel ils sont rattachés, un hétérobicycloalkyle ou un hétérocycloalkyle éventuellement substitué par un ou plusieurs substituants (C₁-C₆)alkyle identiques ou différents ;
R₃ représente -(CH₂)ₚ-Z₃, -C(O)-Z'₃, -CH(OH)-Z'₃ ou -C(O)-NH-Z"₃ ;
   Z₃ représente un radical (C₁-C₆)alkyle, (C₂-C₆)alkényle, (C₁-C₆)alkoxy, (C₁-C₆)alkyl-carbonyle, (C₁-C₆)alkoxy-carbonyle, (C₁-C₆)alkyl-N(R_{N})-carbonyle, (C₃-C₇)cycloalkyle, hétérocycloalkyle, aryle, aryl-thio ou hétéroaryle, Z₃ étant lié au radical -(CH₂)ₚ- par un atome de carbone,
   les radicaux (C₃-C₇) cycloalkyle et hétérocycloalkyle étant éventuellement substitués par un ou plusieurs radicaux identiques ou différents choisis parmi (C₁-C₆)alkyle et oxy ;
   le radical hétéroaryle étant éventuellement substitué par un ou plusieurs substituants identiques ou différents choisis parmi : halo, nitro ou -(CH₂)_{p'}-V₃₀-Y₃;
   le radical aryle étant éventuellement substitué par un ou plusieurs substituants identiques ou différents choisis parmi : halo, nitro, cyano, (C₂-C₆)alkényle, hétérocycloalkyle, aryle, aryloxy, aralkyl-oxy, hétéroaryle et -(CH₂)_{p'}-V₃₁-Y₃ ;
   V₃₀ représente -O-, -C(O)-, -C(O)-O- ou une liaison covalente ;
   V₃₁ représente -O-, -S-, -SO₂-,-C(O)-, -C(O)-O-, -N(R_{N})-, -NH-C(O)-, -C(O)-NR'₃-, -NH-C(O)-NR'₃- ou une liaison covalente ;
   Y₃ représente l'atome d'hydrogène ou un radical (C₁-C₆)alkyle éventuellement substitué par un ou plusieurs radicaux halo identiques ou différents ;
   R_{N} représente un atome d'hydrogène ou un radical (C₁-C₆)alkyle ;
   ou bien Z₃ représente un radical de formule 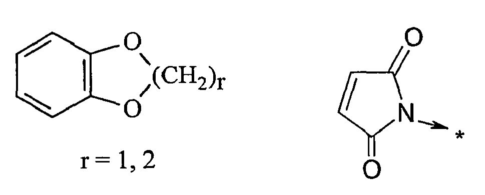 Z'₃ représente un radical aryle éventuellement substitué par un ou plusieurs substituants identiques ou différents choisis parmi : halo, nitro et -(CH₂)_{p"}-V'₃-Y'₃;
   V'₃ représente -O-, -C(O)-, -C(O)-O-, -NH-C(O)-, -C(O)-NR'₃- ou une liaison covalente ;
   Y'₃ représente l'atome d'hydrogène ou un radical (C₁-C₆)alkyle éventuellement substitué par un ou plusieurs radicaux halo identiques ou différents ;
   R'₃ représente l'atome d'hydrogène, un radical (C₁-C₆)alkyle ou (C₁₋C₆)alkoxy ;
   A"₃ représente un radical (C₁-C₆)alkyle, aryle ou hétéroaryle ;
   les radicaux alkyle et aryle étant éventuellement substitués par un ou plusieurs substituants identiques ou différents choisis parmi halo et -V"₃₋Y"₃ ;
   V"₃ représente -O-, -C(O)-, -C(O)-O-, -C(O)-NH- ou une liaison covalente ;
   Y"₃ représente l'atome d'hydrogène ou un radical (C₁-C₆)alkyle éventuellement substitué par un ou plusieurs radicaux halo identiques ou différents ;
   p représente un entier de 0 à 6 ; p' et p" représentent, indépendamment, un entier de 0 à 4 ; q représente un entier de 0 à 2 ;
R₄ représente un radical de formule -(CH₂)ₛ-R'₄ ;
R'₄ représente un hétérocycloalkyle contenant au moins un atome d'azote et éventuellement substitué par (C₁-C₆)alkyle ; un hétéroaryle contenant au moins un atome d'azote et éventuellement substitué par (C₁-C₆)alkyle ; ou un radical de formule -NW₄W'₄ ;
   W₄ représente l'atome d'hydrogène ou (C₁-C₈)alkyle ;
   W'₄ représente un radical de formule -(CH₂)_{s'}-Z₄ dans laquelle Z₄ représente l'atome d'hydrogène, un radical (C₁-C₈)alkyle ou (C₃-C₇)cycloalkyle ;
   s et s' représentent, indépendamment, un entier de 0 à 6 ;
ou un sel pharmaceutiquement acceptable de ce dernier.

Dans les définitions indiquées ci-dessus, l'expression halo représente le radical fluoro, chloro, bromo ou iodo, de préférence chloro, fluoro ou bromo. L'expression alkyle (lorsqu'il n'est pas donné plus de précision), représente de préférence un radical alkyle ayant de 1 à 6 atomes de carbone, linéaire ou ramifié, tels que les radicaux méthyle, éthyle, propyle, isopropyle, butyle, isobutyle, *sec*-butyle et *tert*-butyle, pentyle ou amyle, isopentyle, neopentyle, 2,2-diméthyl-propyle, hexyle, isohexyle ou 1,2,2-triméthyl-propyle. Le terme (C₁-C₈)alkyle désigne un radical alkyle ayant de 1 à 8 atomes de carbone, linéaire ou ramifié, tels les radicaux contenant de 1 à 6 atomes de carbone tels que définis ci-dessus mais également heptyle, octyle, 1,1,2,2-tétraméthyl-propyle, 1,1,3,3-tétraméthyl-butyle. Le terme alkyl-carbonyle désigne les radicaux dans lesquels le radical alkyle est tel que défini ci-dessus comme par exemple méthyl-carbonyle, éthyl-carbonyle. Le terme alkyl-N(R_{N})-carbonyle désigne les radicaux dans lesquels le radical alkyle est tel que défini ci-dessus comme par exemple méthylaminocarbonyle, éthyl-aminocarbonyle, *N-*propyl-*N-*méthylaminocarbonyle, *N,N*-diéthylaminocarbonyle.

Par alkényle, lorsqu'il n'est pas donné plus de précision, on entend un radical alkyle linéaire ou ramifié comptant de 2 à 6 atomes de carbone et présentant au moins une insaturation (double liaison), comme par exemple vinyle, allyle, propényle, butènyle ou pentènyle.

Le terme alkoxy désigne les radicaux dans lesquels le radical alkyle est tel que défini ci-dessus comme par exemple les radicaux méthoxy, éthoxy, propyloxy ou isopropyloxy mais également butoxy linéaire, secondaire ou tertiaire, pentyloxy. Le terme alkoxy-carbonyle désigne de préférence les radicaux dans lesquels le radical alkoxy est tel que défini ci-dessus comme par exemple méthoxycarbonyle, éthoxycarbonyle.

Le terme (C₃-C₇)cycloalkyle désigne un système monocyclique carboné saturé comprenant de 3 à 7 atomes de carbone, et de préférence les cycles cyclopropyle, cyclobutyle, cyclopentyle, cyclohexyle ou cycloheptyle. L'expression hétérocycloalkyle désigne un système saturé monocyclique ou bicyclique condensé contenant de 2 à 7 atomes de carbone et au moins un hétéroatome. Ce radical peut contenir plusieurs hétéroatomes identiques ou différents. De préférence, les hétéroatomes sont choisis parmi l'oxygène, le soufre ou l'azote. Comme exemple d'hétérocycloalkyle, on peut citer les cycles contenant au moins un atome d'azote tels que pyrrolidine, imidazolidine, pyrrazolidine, isothiazolidine, thiazolidine, isoxazolidine, oxazolidine, pipéridine, pipérazine, azépane (azacycloheptane), azacyclooctane, diazépane, morpholine, décahydroisoquinoline (ou décahydroquinoline) mais également les cycles ne contenant aucun atome d'azote tels que tétrahydrofurane ou tétrahydrothiophène. Comme exemple d'hétérocycloalkyle éventuellement substitué par oxy et alkyle, on peut citer les lactones et les lactames.

Le terme hétérobicycloalkyle désigne un système bicyclique hydrocarboné saturé non condensé contenant de 5 à 8 atomes de carbone et au moins un hétéroatome choisi parmi l'azote, l'oxygène et le soufre. Comme exemple d'hétérobicycloalkyle, on peut citer les aza-bicycloheptane et aza-bicyclooctane tels que 7-aza-bicyclo[2,2,1]heptane, 2-aza-bicyclo[2,2,2]octane ou 6-aza-bicyclo[3,2,1]octane.

L'expression aryle représente un radical aromatique, constitué d'un cycle ou de cycles condensés, comme par exemple le radical phényle, naphtyle ou fluorényle. L'expression arylthio représente un radical dont le radical aryle tel que défini ci-dessus comme par exemple phénylthio. L'expression aryloxy représente les radicaux dans lesquels le radical aryle est tel que défini ci-dessus comme par exemple phényloxy, napthyloxy.

Le terme aralkyle (arylalkyle) désigne de préférence les radicaux dans lesquels les radicaux aryle et alkyle sont tels que définis ci-dessus comme par exemple benzyle ou phénéthyle. L'expression aralkyloxy désigne les radicaux dans lesquels les radicaux aralkyle sont tels que définis ci-dessus comme par exemple benzyloxy, phénéthyloxy.

L'expression hétéroaryle désigne un radical aromatique, constitué d'un cycle ou de cycles condensés, avec au moins un cycle contenant un ou plusieurs hétéroatomes identiques ou différents choisis parmi le soufre, l'azote ou l'oxygène. Comme exemple de radical hétéroaryle, on peut citer les radicaux contenant au moins un atome d'azote tels que pyrrolyle, imidazolyle, pyrazolyle, isothiazolyle, thiazolyle, isoxazolyle, oxazolyle, triazolyle, thiadiazolyle, pyridyle, pyrazinyle, pyrimidyle, quinolyle, isoquinolyle, quinoxalinyle, indolyle, benzoxadiazoyle, benzothiazolyle, carbazolyle mais également les radicaux ne contenant pas d'atome d'azote tels que thiényle, benzothiényle, furyle, benzofuryle ou pyranyle.

Dans la présente demande également, le radical (CH₂)ᵢ (i entier pouvant représenter p, p', p", s et s' tels que définis ci-dessus), représente une chaîne hydrocarbonée, linéaire ou ramifiée, de i atomes de carbone. Ainsi le radical -(CH₂)₃- peut représenter -CH₂-CH₂-CH₂- mais également -CH(CH₃)-CH₂-, -CH₂-CH(CH₃)- ou -C(CH₃)₂-.

L'invention concerne de préférence des composés de formule I telle que définie ci-dessus et caractérisés en ce que X représente le radical -CH- ; ou un sel pharmaceutiquement acceptable de ce dernier.

De préférence également, l'invention concerne des composés de formule I telle que définie ci-dessus et caractérisés en ce que R₁ représente l'atome d'hydrogène ou un radical (C₁-C₈)alkyle, et R₂ représente un radical (C₁-C₈)alkyle ; et de manière très préférentielle R₁ représente un radical (C₁-C₆)alkyle et R₂ représente un radical (C₁-C₆)alkyle ; ou un sel pharmaceutiquement acceptable de ce dernier.

De préférence également, l'invention concerne des composés de formule I telle que définie ci-dessus et caractérisés en ce que A représente -CH₂-; ou un sel pharmaceutiquement acceptable de ce dernier.

De préférence également, l'invention concerne des composés de formule I telle que définie ci-dessus et caractérisés en ce que A représente -C(O)-C(Rₐ)(R_{b})- et Rₐ et R_{b} représentent, indépendamment, le radical méthyle ; ou un sel pharmaceutiquement acceptable de ce dernier.

De préférence également, l'invention concerne des composés de formule I telle que définie ci-dessus et caractérisés en ce que A représente -C(O)- ; ou un sel pharmaceutiquement acceptable de ce dernier.

De préférence également, l'invention concerne des composés de formule I telle que définie ci-dessus et caractérisés en ce que
R₄ représente un radical de formule-(CH₂)ₛ-R'₄ ;
R'₄ représente un hétérocycloalkyle contenant au moins un atome d'azote éventuellement substitué par (C₁-C₆)alkyle ; ou un radical de formule -NW₄W'₄ ;
W₄ représente l'atome d'hydrogène ou (C₁-C₈)alkyle ;
W'₄ représente un radical de formule -(CH₂)_{s'}-Z₄ dans laquelle Z₄ représente l'atome d'hydrogène ou un radical (C,-C₈)alkyle ;
s et s' représentent, indépendamment, un entier de 1 à 6 ; ou un sel pharmaceutiquement acceptable de ce dernier.

De manière très préférentielle, l'invention concerne des composés de formule I telle que définie ci-dessus et caractérisés en ce que R₄ représente un radical de formule-(CH₂)ₛ-R'₄ ; R'₄ représente un radical de formule -NW₄W'₄; W₄ représente un radical (C₁-C₈)alkyle ;
W'₄ représente un radical de formule -(CH₂)_{s'}-Z₄ dans laquelle Z₄ représente l'atome d'hydrogène ou un radical (C₁-C₈)alkyle ;
s et s' représentent, indépendamment, un entier de 2 à 6 ; ou un sel pharmaceutiquement acceptable de ce dernier.

De manière très préférentielle également, l'invention concerne des composés de formule I telle que définie ci-dessus et caractérisés en ce que R₄ représente un radical de formule-(CH₂)ₛ-R'₄ ;
R'₄ représente un hétérocycloalkyle contenant au moins un atome d'azote et éventuellement substitué par (C₁-C₆)alkyle ; et s représente un entier de 2 à 6 ;
et plus particulièrement R'₄ représente le cycle pipéridine ou pyrrolidine ; s représente un entier de 1 à 4 ; ou un sel pharmaceutiquement acceptable de ce dernier.

De préférence également, l'invention concerne des composés de formule I telle que définie ci-dessus et caractérisés en ce que R₃ représente -(CH₂)ₚ-Z₃ et
Z₃ représente un radical (C₁-C₆)alkyle, (C₂-C₆)alkényle (C₁-C₆)alkoxy, (C₁-C₆)alkyl-carbonyle, (C₁-C₆)alkoxy-carbonyle, (C₁-C₆)alkyl- N(R_{N})carbonyle, (C₃-C₇)cycloalkyle, hétérocycloalkyle, aryle, aryl-thio ou hétéroaryle,
les radicaux (C₃-C₇) cycloalkyle et hétérocycloalkyle étant éventuellement substitués par un ou plusieurs radicaux identiques ou différents choisis parmi (C₁-C₆)alkyle et oxy ;
le radical hétéroaryle étant éventuellement substitué par un ou plusieurs substituants identiques ou différents choisis parmi : halo, nitro ou -(CH₂)_{p'}-V₃₀-Y₃;
le radical aryle étant éventuellement substitué par un ou plusieurs substituants identiques ou différents choisis parmi : halo, nitro, (C₂-C₆)alkényle, hétérocycloalkyle, aryle, aryloxy, aralkyl-oxy, hétéroaryle et -(CH₂)_{p'}-V₃₁-Y₃ ;
V₃₀ représente -O-, -C(O)-, -C(O)-O- ou une liaison covalente ;
V₃₁ représente -O-, -S-, -SO₂-, -C(O)-, -C(O)-O-, -N(R_{N})-, -NH-C(O)-, -C(O)-NR'₃- ou une liaison covalente ;
Y₃ représente l'atome d'hydrogène ou un radical (C₁-C₆)alkyle éventuellement substitué par un ou plusieurs radicaux halo identiques ou différents ;
R_{N} représente un atome d'hydrogène ou un radical (C₁-C₆)alkyle ;
ou bien Z₃ représente un radical de formule 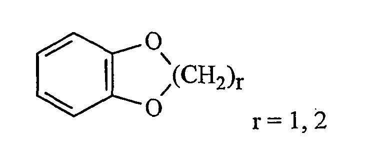 ou un sel pharmaceutiquement acceptable de ce dernier ;

De préférence également, l'invention concerne des composés de formule I telle que définie ci-dessus et caractérisés en ce que Z₃ représente un radical (C₁-C₆)alkyle, (C₁-C₆)alkyl-carbonyle, (C₁-C₆)alkoxy-carbonyle, (C₁-C₆)alkyl-N(R_{N})-carbonyle, (C₃-C₇)cycloalkyle, aryle ou hétéroaryle, les radicaux aryle et hétéroaryle étant éventuellement substitués ;
et de manière très préférentielle
le radical hétéroaryle est éventuellement substitué par un ou plusieurs substituants identiques ou différents choisis parmi : halo et -(CH₂)_{p'}-V₃₀-Y₃ ;
le radical aryle est éventuellement substitué par un ou plusieurs substituants identiques ou différents choisi parmi : nitro et -(CH₂)_{p'}-V₃₁-Y₃ ;
V₃₀ représente -O-, -C(O)-, -C(O)-O- ou une liaison covalente ;
V₃₁ représente -O-, -C(O)-, -C(O)-O- ou -SO₂-;
Y₃ représente un radical (C₁-C₆)alkyle ;
p et p' représentent, indépendamment, un entier de 0 à 4 ; ou un sel pharmaceutiquement acceptable de ce dernier.

De manière très préférentielle également, l'invention concerne des composés de formule I telle que définie ci-dessus et caractérisés en ce que Z₃ représente un radical (C₁-C₆)alkyle ; ou un sel pharmaceutiquement acceptable de ce dernier.

De manière très préférentielle également, l'invention concerne des composés de formule I telle que définie ci-dessus et caractérisés en ce que Z₃ représente un radical (C₁-C₆)alkyl-carbonyle, (C₁-C₆)alkoxy-carbonyle ou (C₁-C₆)alkyl-N(R_{N})-carbonyle ; ou un sel pharmaceutiquement acceptable de ce dernier.

De manière très préférentielle également, l'invention concerne des composés de formule I telle que définie ci-dessus et caractérisés en ce que Z₃ représente un hétéroaryle éventuellement substitué par un ou plusieurs substituants identiques ou différents choisis parmi : halo et -(CH₂)_{p'}-V₃₀-Y₃;
V₃₀ représente -O-, -C(O)-, -C(O)-O- ou une liaison covalente ;
Y₃ représente un radical (C₁-C₆)alkyle ;
p' représente un entier de 0 à 4 ;
et plus particulièrement Z₃ représente le radical thiényle, furyle, benzofuryle, benzothiényle, thiazolyle, pyrazolyle, imidazolyle, pyridinyle, indolyle ; ou un sel pharmaceutiquement acceptable de ce dernier.

De manière très préférentielle également, l'invention concerne des composés de formule I telle que définie ci-dessus et caractérisés en ce que Z₃ représente un radical (C₃-C₇)cycloalkyle ou aryle, le radical aryle étant éventuellement substitué par un ou plusieurs substituants identiques ou différents choisi parmi : nitro ou -(CH₂)_{p'}-V₃₁-Y₃ ;
V₃₁ représente -O-, -C(O)-, -C(O)-O- ou -SO₂- ;
Y₃ représente un radical (C₁-C₆)alkyle ;
p' représente un entier de 0 à 4 ;
et plus particulièrement le radical (C₃-C₇)cycloalkyle est choisi parmi cyclopentyle et cyclohexyle ; le radical aryle est le radical phényle ; ou un sel pharmaceutiquement acceptable de ce dernier.

De manière très préférentielle également, l'invention concerne des composés de formule I telle que définie ci-dessus et caractérisés en ce que R₃ représente -C(O)-Z'₃ ; ou un sel pharmaceutiquement acceptable de ce dernier.

De manière très préférentielle également, l'invention concerne des composés de formule I telle que définie ci-dessus et caractérisés en ce que Z'₃ représente un radical phényle éventuellement substitué par un ou plusieurs substituants identiques ou différents de formule -(CH₂)_{p"}- V'₃-Y'₃;
V'₃ représente -O- ;
Y'₃ représente un radical (C₁-C₆)alkyle ;
p" représente un entier de 0 à 4 ; ou un sel pharmaceutiquement acceptable de ce dernier.

De manière très préférentielle également, l'invention concerne des composés de formule I telle que définie ci-dessus et caractérisés en ce que R₃ représente -C(O)-NH-Z"₃
Z"₃ représente un radical -(CH₂)_{q}-A"₃;
A"₃ représente un radical (C₁-C₆)alkyle, phényle ou thiényle ;
les radicaux alkyle et aryle étant éventuellement substitués par un ou plusieurs substituants identiques ou différents de formule -V"₃-Y"₃ ;
V"₃ représente -O-, -C(O)-, -C(O)-O- ou une liaison covalente ;
Y"₃ représente l'atome d'hydrogène ou un radical (C₁-C₆)alkyle ;
q représente un entier de 0 à 1 ; ou un sel pharmaceutiquement acceptable de ce dernier.

Dans la présente demande, le symbole -> * correspond au point de rattachement du radical. Lorsque le site de rattachement n'est pas précisé sur le radical, cela signifie que le rattachement s'effectue sur un des sites disponibles de ce radical pour un tel rattachement.

Suivant les définitions des groupes variables A, X, R₁, R₂, R₃ et R₄, les composés selon l'invention peuvent être préparés en phase liquide selon les différentes procédures A à D décrites ci-dessous.

### A. Préparation selon le schéma réactionnel A :

Les composés de formule I selon l'invention dans laquelle A représente -C(O)-, peuvent être préparés selon le schéma A suivant :

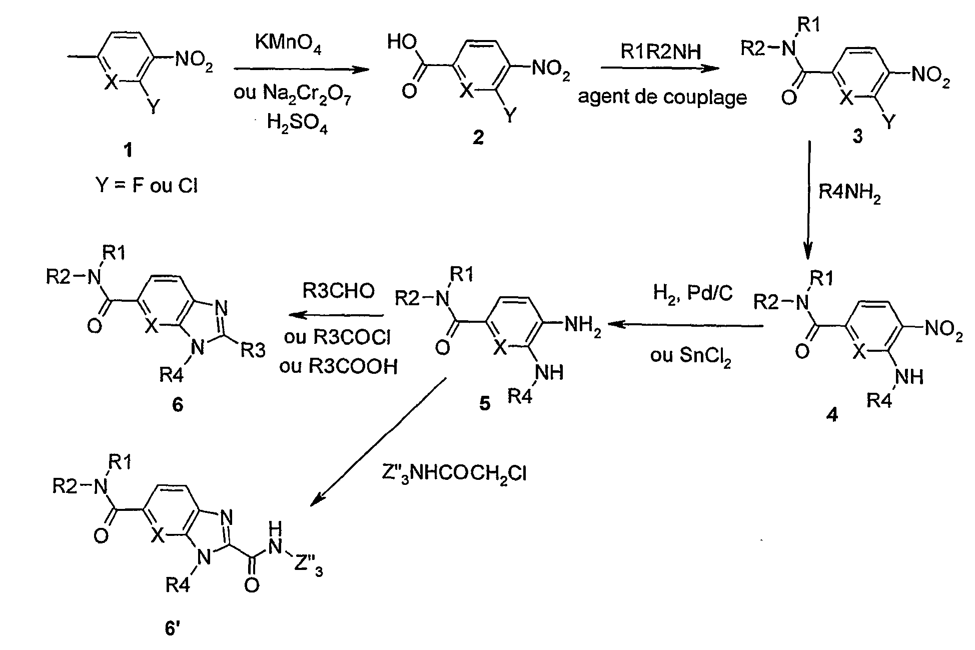

Comme décrit dans le schéma A, le dérivé méthylé (1) (pour X = CH composé commercial ; pour X = N composé préparé selon la procédure de Baumgarten et al, J.Am. Chem. Soc, 1952, 74, 3828-3831, à partir du 6-méthyl-3-nitro-pyridine-amine) peut être oxydé en acide carboxylique (2) par une solution aqueuse de permanganate de potassium à une température de 100° C pendant 3 à 6 heures (selon procédure de Schmelkes et al, J. Am. Chem. Soc, 1944, 1631), ou par une solution aqueuse de dichromate de sodium en présence d'acide sulfurique à une température de 20-90° C pendant 1 à 3 heures (selon procédure de Howes et al, European J. Med. Chem, 1999, 34, 225-234). L'acide carboxylique (2) peut être couplé avec une amine primaire ou secondaire en présence d'un agent de couplage tel que le diisopropylcarbodiimide (DIC), le dicyclohexylcarbodiimide (DCC), le chlorhydrate de 1-(3-diméthylaminopropyl)-3-éthylcarbodiimide (EDC) ou le carbonyldiimidazole (CDI) avec ou sans du 1-hydroxybenzotriazole (HOBt) dans un solvant organique inerte tel que du chlorure de méthylène, tétrahydrofuranne ou diméthylformamide à température ambiante pendant 3 à 24 heures pour conduire à l'amide correspondante (3). Le traitement du dérivé fluoré ou chloré (3) par une amine primaire en présence d'une base inorganique telle que du carbonate de césium ou de potassium dans un solvant organique inerte tel que le diméthylformamide ou l'acétontrile à une température de 20-100° C pendant 2 à 48 heures conduit au dérivé (4). La fonction nitro du composé (4) est réduite par traitement avec du chlorure d'étain dihydrate dans un solvant inerte tel que l'acétate d'éthyle ou le diméthylformamide à une température de 60-80° C pendant 3 à 15 heures, ou par hydrogénation catalytique en présence de palladium sur charbon 10 % dans un solvant inerte tel que le méthanol, éthanol, acétate d'éthyle ou un mélange de ces solvants, à une température de 18-25° C, pendant 2 à 8 heures pour conduire à la dianiline (5). Le dérivé (5) peut ensuite être traité par un aldéhyde en présence d'un oxydant tel que le nitrobenzène, le DDQ dans un solvant aprotique tel que le diméthylformamide à une température de 60-180° C pendant 2 à 24 heures, ou au micro-onde à une température de 150-200°C pendant 5 à 30 minutes, pour conduire au benzimidazole (6). Alternativement, le dérivé (5) peut réagir soit avec un chlorure d'acide, soit avec un acide carboxylique en présence d'un agent de couplage tel que le diisopropylcarbodiimide (DIC), le dicyclohexylcarbodiimide (DCC), le chlorhydrate de 1-(3-dimethylaminopropyl)-3-éthylcarbodiimide (EDC), le carbonyldiimidazole (CDI), le 2-(1H-benzotriazole-1-yl)-1,1,3,3-tetramethyluronium hexafluorophosphate (HBTU), le 2-(1H-benzotriazole-1-yl)-1,1,3,3-tetramethyluronium tetrafluoroborate (TBTU) ou le O-(7-azobenzotriazol-1-yl)-1,1,3,3-tétraméthyluronium hexafluorophosphate (HATU) dans un solvant organique inerte tel que du chlorure de méthylène, tétrahydrofuranne ou diméthylformamide à température ambiante pendant 3 à 24 heures pour conduire à l'amide correspondante. L'amide ainsi obtenue conduit au benzimidazole (6) par traitement avec un acide, tel que l'acide acétique, l'acide chlorhydrique, l'acide polyphosphorique à une température de 20-100° C pendant 2 à 24 heures ou au micro-onde à une température de 80-150°C pendant 5 à 30 minutes. Le dérivé (5) peut également réagir avec un imidate d'ester ou un dérivé chloroacétamide dans un solvant organique tel que du diméthylformamide ou du méthanol ou éthanol, en présence ou non d'une base tertiaire, de soufre, à une température de 20-100° C pendant 3 à 24 heures, ou au micro-onde à une température de 80-130°C pendant 5 à 30 minutes pour conduire au dérivé benzimidazole (6').

### Exemple A1 : chlorhydrate de 2-(4-méthoxyphényl)-N,N-bis(3-méthylbutyl)-1-(3-pipéridin-1-ylpropyl)-1H-benzimidazole-6-carboxamide

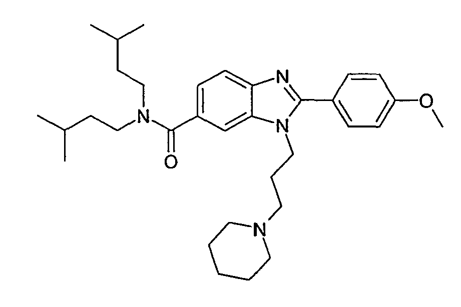

### Etape 1 : acide 3-fluoro-4-nitrobenzoïque

Un mélange de 3-fluoro-4-nitrotoluène (10 g, 1 eq) et de permanganate de potassium (25,5 g, 2,5 eq) dans l'eau (1 L) est chauffé à reflux pendant 6 heures puis refroidi à température ambiante. Le mélange est filtré sur célite et la phase aqueuse est lavée deux fois à l'éther diéthylique (2 x 300 mL). La phase aqueuse est acidifiée avec une solution aqueuse d'acide chlorhydrique concentrée (12N) puis concentrée sous pression réduite à 40° C jusqu'à un volume d'environ 300 mL. Le précipité formé est filtré puis lavé à l'éther de pétrole et séché pour donner le composé attendu sous forme d'un solide blanc (6,9 g ; 58 % rendement).

RMN ¹H (400 MHz, DMSO-*d₆*) : δ 7,93 (m, 2H), 8,25 (m, 1H), 13,95 (m, 1H).

### Etape 2 : 3-fluoro-N,N-bis(3-méthylbutyl)-4-nitrobenzamide

A l'acide 3-fluoro-4-nitrobenzoïque (3,8 g, 1 eq) en solution dans le THF anhydre (30 mL) sont successivement additionnés le chlorhydrate de 1-(3-diméthylaminopropyl)-3-éthylcarbodiimide (EDC) (4,4 g, 1,1 eq) en solution dans le chloroforme (25 mL) et le 1-hydroxybenzotriazole (HOBt) (3,05 g, 1,1 eq) en solution dans le THF (40 mL). Le mélange est agité 1 heure à une température d'environ 20° C puis la diisoamylamine (3,6 g, 1,1 eq) en solution dans le THF (30 mL) est additionnée. Après 16 heures d'agitation à une température d'environ 20° C, le mélange réactionnel est concentré sous pression réduite à 40° C. Le résidu est repris par du dichlorométhane (200 mL) et de l'eau (70 mL). Après décantation et extraction, les phases organiques combinées sont lavées avec de la saumure, séchées sur Na₂SO₄ puis concentrées sous pression réduite à 40° C. La purification du composé par chromatographie éclair sur gel de silice (éluant : heptane / acétate d'éthyle 9 :1) donne le composé attendu sous forme d'une huile jaune (4,3 g ; 65 % rendement).

SM/CL : MM calculée = 324,4 ; m/z = 325,2 (MH+)

RMN ¹H (400 MHz, DMSO-*d₆*): δ 0,69 (m, 6H), 0,93 (m, 6H), 1,35-1,60 (m, 6H), 3,09 (m, 2H), 3,41 (m, 2H), 7,38 (d,1H), 7,63 (d,1H), 8,21 (t, 1H).

### Etape 3 : N,N-bis(3-méthylbutyl)-4-nitro-3-[(3-pipéridin-1-ylpropyl)amino]benzamide

Un mélange de 3-fluoro-*N,N*-bis(3-méthylbutyl)-4-nitrobenzamide (430 mg, 1 eq), de 3-pipéridino-propylamine (212 mg, 1,1 eq) et de carbonate de potassium (365 mg, 2 eq) dans l'acétonitrile (10 mL) est chauffé au reflux pendant 3 heures puis concentré sous pression réduite à 40° C. Le résidu est repris par du dichlorométhane (50 mL) et de l'eau (20 mL). Après décantation et extraction, les phases organiques combinées sont lavées avec de la saumure, séchées sur Na₂SO₄ puis concentrées sous pression réduite à 40° C. La purification du résidu par chromatographie éclair sur gel de silice (éluant : heptane/acétate d'éthyle 1:1 à acétate d'éthyle 100 %) donne le composé attendu sous forme d'une huile jaune (460 mg ; 78 % rendement).

SM/CL : MM calculée = 446,6 ; m/z = 447,3 (MH+)

RMN ¹H (400 MHz, DMSO-*d₆*) : δ 0,68 (d, 6H), 0,92 (d, 6H), 1,31-1,69 (m, 12H), 1,74 (m, 2H), 2,32 (m, 6H), 3,10 (m, 2H), 3,38 (m, 4H), 6,53 (d, 1H), 6,91 (m, 1H), 8,09 (d, 1H), 8,44 (t, 1H).

### Etape 4 : 4-amino-N,N-bis(3-méthylbutyl)-3-[(3-pipéridin-1-ylpropyl)amino]benzamide

Dans un autoclave sont additionnés le *N,N*-bis(3-méthylbutyl)-4-nitro-3-[(3-pipéridin-1-ylpropyl)amino]benzamide (1 g) en solution dans un mélange d'acétate d'éthyle / éthanol 2:1 (100 mL) et le palladium sur charbon 10 % (100 mg). Après 3 heures d'agitation sous atmosphère d'hydrogène (3 bars) à une température d'environ 20° C, le catalyseur est éliminé par filtration sur célite et le filtrat est concentré sous pression réduite à 40° C pour donner le composé attendu sous forme d'une huile (910 mg, 97 % rendement).

SM/CL : MM calculée = 416,6 ; m/z = 417,3 (MH+)

RMN ¹H (400 MHz, DMSO-*d₆*) : δ 0,81 (d, 12H), 1,39-1,69 (m, 12H), 1,73 (m, 2H), 2,32 (m, 6H), 3,03 (m, 2H), 3,38 (m, 4H), 4,62 (s, 1H), 4,76 (s, 2H), 6,36 (s, 1H), 6,42 (AB, 1H), 6,50 (AB, 1H).

### Etape 5 : chlorhydrate de 2-(4-méthoxyphényl)-N,N-bis(3-méthylbutyl)-1-(3-pipéridin-1-ylpropyl)-1H-benzimidazole-6-carboxamide

A une solution du 4-amino-*N,N*-bis(3-méthylbutyl)-3-[(3-pipéridin-1-ylpropyl)amino] benzamide (62 mg) dans le nitrobenzène (2 mL) est additionné le *p-*anisaldéhyde (27 mg, 1,3 eq). Le mélange est chauffé à 130° C pendant 6 heures. La purification du mélange par chromatographie éclair sur gel de silice (éluant : dichlorométhane 100 % à dichlorométhane / méthanol 9:1) donne le composé attendu sous forme de base libre. Le sel de chlorhydrate correspondant est formé par addition d'une solution d'acide chlorhydrique 1N dans l'éther diéthylique. Le précipité obtenu est filtré et séché pour donner le composé monochlorhydrate attendu (58 mg, 68 % rendement).

SM/CL : MM calculée = 532,8 ; m/z = 533,5 (MH+)

RMN ¹H (400 MHz, DMSO-*d₆*):δ 0,68 (d, 6H), 0,95 (d, 6H), 1,26-1,69 (m, 12H), 2,27 (m, 2H), 2,72 (m, 2H), 3,03 (m, 2H), 3,25 (m, 4H), 3,45 (m, 2H), 3,91 (s, 3H), 4,56 (t, 2H), 7,27 (AB, 2H), 7,50 (AB, 1H), 7,87 (AB, 1H), 7,92 (AB, 1H), 8,15 (s, 1H), 10,89 (s, 1H).

### Exemple A2 : chlorhydrate de 2-(4-méthoxybenzyl)-N,N-bis(3-méthylbutyl)-1-(3-pipéridin-1-ylpropyl)-1H benzimidazole-6-carboxamide

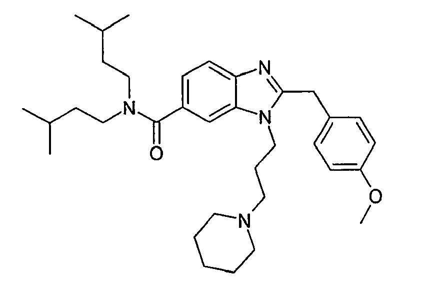

A une solution du 4-amino-*N,N*-bis(3-méthylbutyl)-3-[(3-pipéridin-1-ylpropyl)amino] benzamide (66 mg) dans l'acide acétique (2 mL) est additionné le 4-méthoxyphénylacétylchloride (32 mg, 1,1 eq). Le mélange est chauffé à 100° C pendant 18 heures puis refroidi et concentré sous pression réduite. Au résidu obtenu dissous dans le dichlorométhane est additionnée une solution aqueuse saturée en hydrogénocarbonate de sodium. Après décantation et extractions, les phases organiques combinées sont lavées à la saumure, séchées sur Na₂SO₄ et concentrées sous pression réduite. La purification du résidu obtenu par chromatographie éclair sur gel de silice (éluant : dichlorométhane 100 % à dichlorométhane / méthanol 9:1) donne le composé attendu sous forme de base libre. Le sel de chlorhydrate correspondant est formé par addition d'une solution d'acide chlorhydrique 1N dans l'éther diéthylique. Le précipité obtenu est filtré et séché pour donner le composé monochlorhydrate attendu (51 mg, 59 % rendement).

SM/CL : MM calculée = 546,8 ; m/z = 547,5 (MH+)

RMN ¹H (400 MHz, DMSO-*d₆*) : δ 0,63 (d, 6H), 0,94 (d, 6H), 1,24-1,92 (m, 12H), 2,11 (m, 2H), 2,73 (m, 2H), 3,03-3,29 (m, 6H), 3,40 (m, 2H), 3,74 (s, 3H), 4,56 (t, 2H), 4,62 (s, 2H), 6,96 (AB, 2H), 7,43 (m, 3H), 7,79 (AB, 1H), 8,03 (s, 1H), 11,02 (s, 1H).

### Exemple A3 : chlorhydrate de 2-[3-(méthylamino)-3-oxopropyl]-N,N bis(3-méthylbutyl)-1-(3-pipéridin-1-ylpropyl)-1H-benzimidazole-6-carboxamide

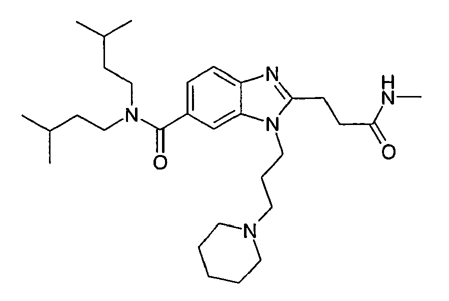

A une solution d'acide *N-*méthylsuccinimique (26 mg, 1 eq) dans le DMF (1 mL) sont successivement additionnés le TBTU (67 mg, 1 eq) et la diisopropyléthylamine (70 µL, 2 eq). Après 30 minutes d'agitation à température ambiante, une solution de 4-amino-*N,N*-bis(3-méthylbutyl)-3-[(3-pipéridin-1-ylpropyl)amino] benzamide (66 mg) dans le DMF (1 mL) est additionnée au mélange. Le mélange est agité 15 heures à une température voisine de 20° C puis dilué dans l'acétate d'éthyle (10 mL) et additionné d'une solution aqueuse saturée en hydrogénocarbonate de sodium (4 mL). Après décantation et extractions, les phases organiques combinées sont lavées à la saumure, séchées sur Na₂SO₄ et concentrées sous pression réduite à 40° C. L'huile ainsi obtenue est solubilisée dans l'acide acétique (2 mL). Le mélange est chauffé à 100° C pendant 18 heures puis refroidi à température ambiante et concentré sous pression réduite à 40° C. Au résidu obtenu dissous dans le dichlorométhane est additionnée une solution aqueuse saturée en hydrogénocarbonate de sodium. Après décantation et extractions, les phases organiques combinées sont lavées à la saumure, séchées sur Na₂SO₄ et concentrées sous pression réduite. La purification du résidu obtenu par chromatographie éclair sur gel de silice (éluant : dichlorométhane 100 % à dichlorométhane / méthanol 85:15) donne le composé attendu sous forme de base libre. Le sel de chlorhydrate correspondant est formé par addition d'une solution d'acide chlorhydrique 1N dans l'éther diéthylique. Le précipité obtenu est filtré et séché pour donner le composé monochlorhydrate attendu (64 mg, 54 % rendement).

SM/CL : MM calculée = 511,8 ; m/z = 512,4 (MH+)

RMN ¹H (400 MHz, DMSO-*d₆*): δ 0,65 (d, 6H), 0,94 (d, 6H), 1,24-1,90 (m, 12H), 2,29 (m, 2H), 2,56 (d, 3H), 2,82 (m, 2H), 2,93 (t, 2H), 3,16 (m, 4H), 3,33-3,52 (m, 6H), 4,63 (t, 2H), 7,46 (AB, 1H), 7,43 (m, 3H), 7,82 (AB, 1H), 8,10 (s, 1H), 8,20 (m, 1H), 10,86 (s, 1H).

### Exemple A4 : chlorhydrate de 2-(1-benzofuran-2-yl)-N,N-dibutyl-1-(3-pipéridin-1-ylpropyl)-1H-benzimidazole-6-carboxamide

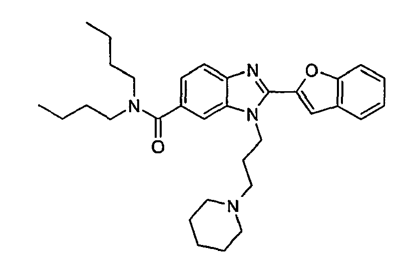

A une solution de 4-amino-*N,N*-dibutyl-3-[(3-pipéridin-1-ylpropyl)amino]benzamide (1 g) dans le nitrobenzène (5 mL), placée dans un tube réactionnel "Personal Chemistry®" est additionné le 1-benzofuran-2-carbaldéhyde (450 mg). Le tube est scellé par une capsule, placé dans le micro-onde "Personal Chemistry®" et chauffé sous agitation magnétique à 200° C pendant 20 minutes. La purification du mélange obtenu par chromatographie éclair sur gel de silice (éluant : dichlorométhane 100 % à dichlorométhane / méthanol 95:5) donne le composé attendu sous forme de base libre. Le sel de chlorhydrate correspondant est formé par addition d'une solution d'acide chlorhydrique 1N dans l'éther diéthylique. Le précipité obtenu est filtré et séché pour donner le composé chlorhydrate attendu (780 mg, 54 % rendement).

SM/CL : MM calculée = 514,7 ; m/z = 515,5 (MH+)

RMN ¹H (400 MHz, DMSO-*d₆*): δ 0,70 (s large, 3H), 0,95 (s large, 3H), 1,28-1,88 (m, 12H), 2,36 (m, 2H), 2,83 (m, 2H), 2,93 (t, 2H), 3,22 (m, 4H), 3,36 (d, 2H), 3,42 (m, 2H), 4,78 (t, 2H), 7,33 (AB, 1H), 7,42 (t, 1H), 7,52 (t, 1H), 7,79 (AB, 1H), 7,87 (AB, 1H), 7,91 (s, 1H), 7,96 (s, 1H).

### Exemple A5 : chlorhydrate du 4-({[6-{[bis(3-méthylbutyl)amino]carbonyl}-1-(3-pipéridin-1-ylpropyl)-1H-benzimidazol-2-yl]carbonyl}amino)benzoate d'éthyle

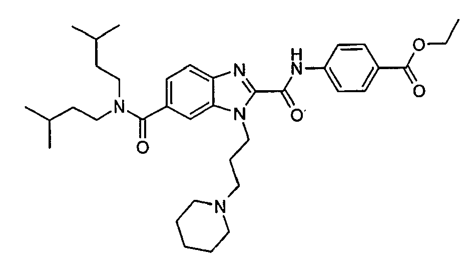

A une solution du 4-amino-*N,N*-bis(3-méthylbutyl)-3-[(3-pipéridin-1-ylpropyl)amino] benzamide (100 mg) dans l'éthanol (3 mL), placée dans un tube réactionnel « Personal Chemistry®" sont successivement additionnés la triéthylamine (100 µL), l'éthyl 4-[(chloroacétyl)amino]benzoate (173 mg) et le soufre (12 mg). Le tube est scellé par une capsule, placé dans le micro-onde "Personal Chemistry®" et chauffé sous agitation magnétique à 130° C pendant 20 minutes. L'éthanol est ensuite évaporé et le résidu additionné d'eau et de dichlorométhane. Après décantation et extraction, les phases organiques combinées sont lavées avec de la saumure, séchées sur Na₂SO₄ puis concentrées sous pression réduite à 40° C. La purification du composé par chromatographie éclair sur gel de silice (éluant : dichlorométhane 100 % à dichlorométhane / éthanol 85 :15) donne le composé attendu sous forme de base libre. Le sel de chlorhydrate correspondant est formé par addition d'une solution d'acide chlorhydrique 1N dans l'éther diéthylique. Le précipité obtenu est filtré et séché pour donner le composé monochlorhydrate attendu (80 mg, 51 % rendement).

SM/CL : MM calculée = 617,8 ; m/z = 618,5 (MH+)

RMN ¹H (400 MHz, DMSO-*d₆*) : δ 0,62 (s large, 6H), 0,95 (s large, 6H), 1,32 (t, 3H), 1,37-1,75 (m, 12H), 2,30 (m, 2H), 2,83 (m, 2H), 2,93 (t, 2H), 3,17 (m, 4H), 3,37-3,48 (m, 4H), 4,30 (q, 2H), 4,77 (t, 2H), 7,30 (AB, 1H), 7,87 (AB, 1H), 7,88 (s, 1H), 7,97 (AB, 1H), 8,06 (AB, 1H), 10,14 (s, 1H), 11,25 (s, 1H).

Selon le schéma réactionnel A et de façon analogue aux procédures décrites pour la synthèse du chlorhydrate de 2-(4-méthoxyphényl)-*N,N*-bis(3-méthylbutyl)-1-(3-pipéridin-1-ylpropyl)-1*H-*benzimidazole-6-carboxamide, de 2-(4-méthoxybenzyl)-*N,N*-bis(3-méthylbutyl)-1-(3-pipéridin-1-ylpropyl)-1*H-*benzimidazole-6-carboxamide, de 2-[3-(méthylamino)-3-oxopropyl]-*N,N*-bis(3-méthylbutyl)-1-(3-pipéridin-1-ylpropyl)-1*H-*benzimidazole-6-carboxamide, de 2-(1-benzofuran-2-yl)--*N,N*-dibutyl-1-(3-pipéridin-1-ylpropyl)-1*H-*benzimidazole-6-carboxamide ou du 4-({[6-{[bis(3-méthylbutyl)amino]carbonyl}-1-(3-piperidin-1-ylpropyl)-1*H-*benzimidazol-2-yl] carbonyl}amino)benzoate d'éthyle, les composés suivants ont été préparés : 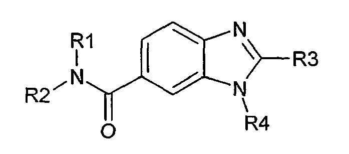 dans lesquels R₁R₂N représente l'un des radicaux ci-après : 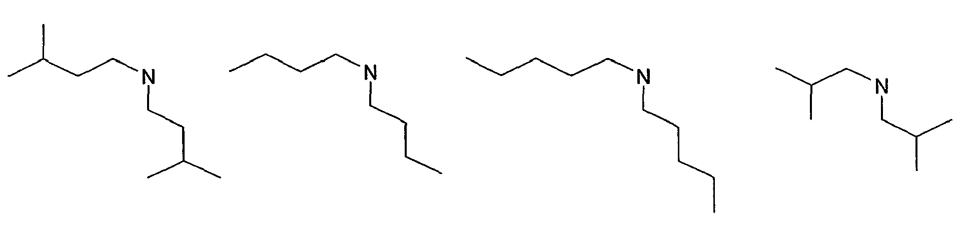 R₃ représente l'un des radicaux ci-après : 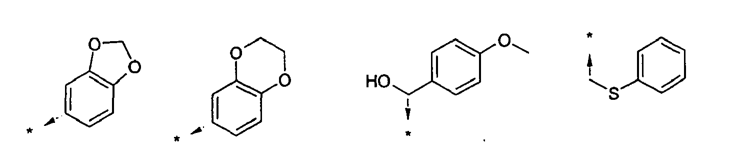 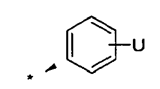 1 ou plusieurs substitutions choisies parmi :
= H, F, Cl, Br, I, NO₂, OMe, OEt, OPh, SMe, SEt, SCF₃, Me, Et, iPr, tBu, CN, CF₃, OCF₃, C(O)OMe, C(O)OEt, C(O)Me, C(O)Et, C(O)NHMe, C(O)NH₂, NMe₂, NEt₂, NHCOMe, Phe, OCH₂Ph SO₂Me 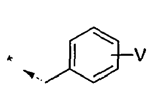 V = H, F, Cl, Br, I, NO₂, OMe, Me, Et, iPr, CF₃,OCF₃, C(O)OMe, C(O)Me, C(O)NHMe, SO₂Me 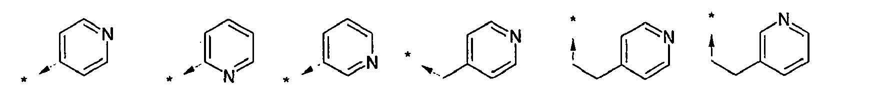 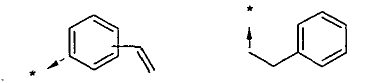 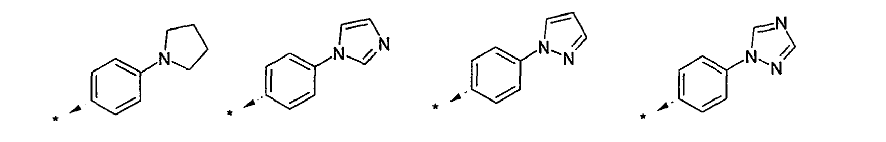 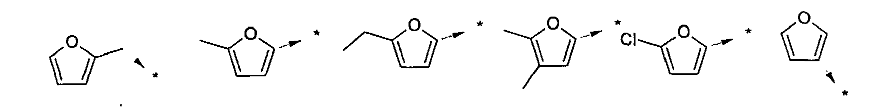 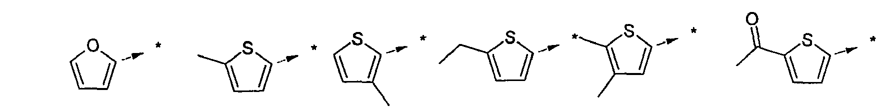 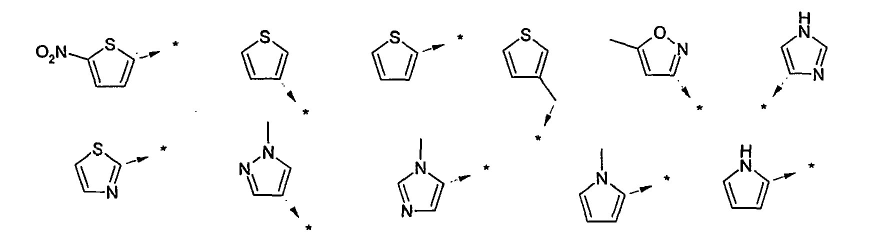 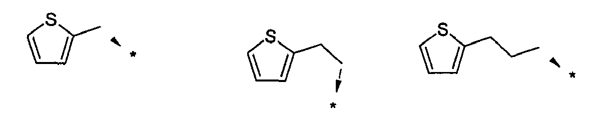 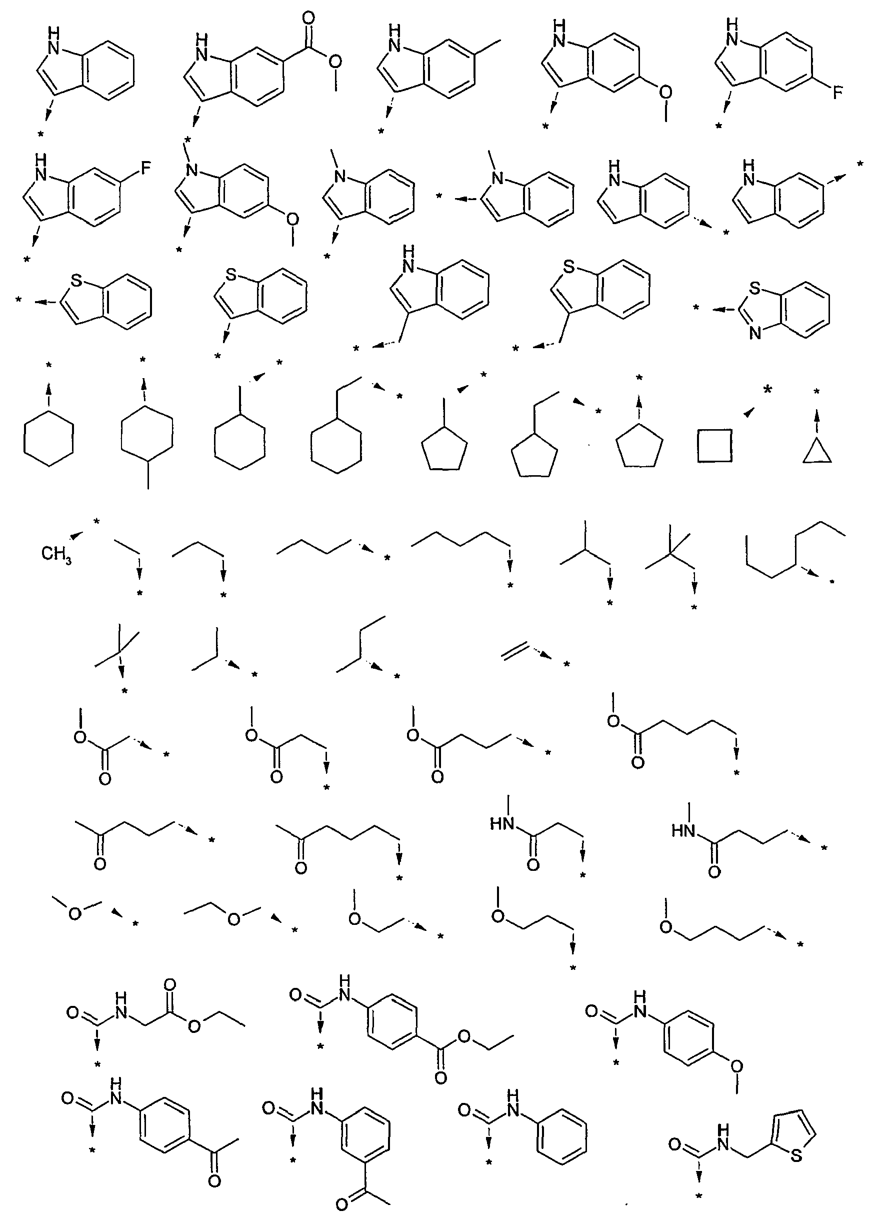 et R₄ représente l'un des radicaux ci-après :

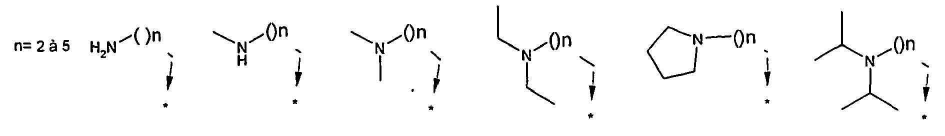

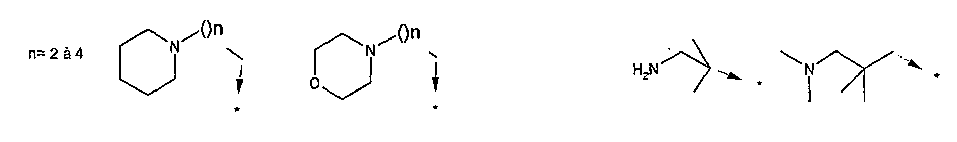

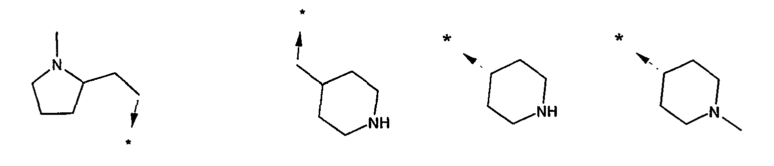

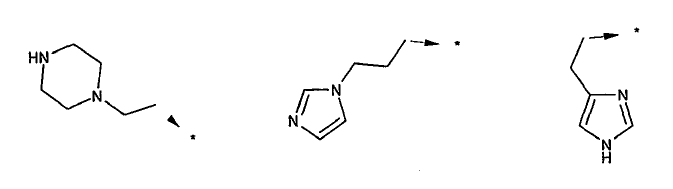

### B. Préparation selon le schéma réactionnel B :

Les composés de formule I selon l'invention dans laquelle A représente -(CO)- et R₃ représente -C(O)-Z'₃ (Z'₃ représentant un radical aryle symbolisé par Ar) peuvent être préparés selon le schéma B suivant :

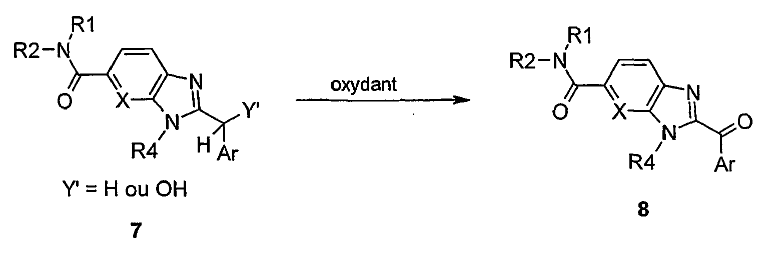

Comme décrit dans le schéma B, le dérivé (7) peut être oxydé par du manganèse dioxide dans un solvant aprotique tel que le tétrahydrofuranne, le dioxanne ou par du trioxide de chromium dans un acide tel que l'acide acétique, à une température de 20-80° C pendant 10-96 heures pour conduire au dérivé (8).

### Exemple B1 : chlorhydrate de 2-(4-méthoxybenzoyl)-N,N-bis(3-méthylbutyl)-1-(3-pipéridin-1-ylpropyl)-1H-benzimidazole-6-carboxamide

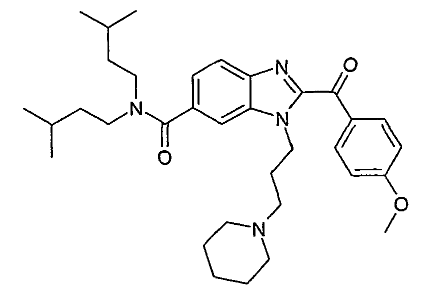

A une solution de 2-(4-méthoxybenzyl)-*N,N*-bis(3-méthylbutyl)-1-(3-pipéridin-1-ylpropyl)-1*H-*benzimidazole-6-carboxamide (171 mg, préparé selon l'exemple A2) dans le 1,4 dioxanne (5 mL) est additionné le dioxyde de manganèse (500 mg). Le mélange est chauffé à 70° C pendant 24 heures puis une nouvelle portion de dioxyde de manganèse (500 mg) est additionnée. Après 24 heures d'agitation supplémentaires à 70° C, une portion de dioxyde de manganèse (500 mg) est de nouveau additionnée et l'agitation à 70° C est poursuivie encore pendant 24 heures puis le mélange est refroidi à température ambiante, concentré sous pression réduite et filtré sur célite. Le filtrat est concentré sous pression réduite à 40° C pour donner le composé attendu sous forme de base libre. Le sel de chlorhydrate correspondant est formé par addition d'une solution d'acide chlorhydrique 1N dans l'éther diéthylique. Le précipité obtenu est filtré, lavé à l'éther diéthylique puis recristallisé dans un mélange dichlorométhane/éther diéthylique et séché pour donner le composé chlorhydrate attendu (50 mg, 26 % rendement).

SM/CL : MM calculée = 560,8 ; m/z = 561,4 (MH+)

RMN ¹H (400 MHz, DMSO-*d₆*) : δ 0,65 (d, 6H), 0,95 (d, 6H), 1,25-1,79 (m, 12H), 2,28 (m, 2H), 2,82 (m, 2H), 3,16 (m, 4H), 3,32-3,48 (m, 4H), 3,89 (s, 3H), 4,61 (t, 2H), 7,13 (AB, 2H), 7,30 (AB, 1H), 7,89 (AB, 1H), 8,33 (AB, 2H), 10,48 (s, 1H).

Selon le schéma réactionnel B et de façon analogue à la procédure décrite pour la synthèse du chlorhydrate de 2-(4-méthoxybenzoyl)-*N,N*-bis(3-méthylbutyl)-1-(3-pipéridin-1-ylpropyl)-1*H-*benzimidazole-6-carboxamide, les composés suivants ont été préparés : 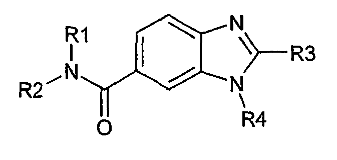 dans lesquels R₁R₂N représente l'un des radicaux ci-après : 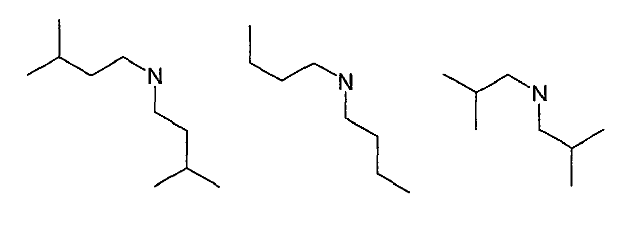 R₃ représente l'un des radicaux ci-après : 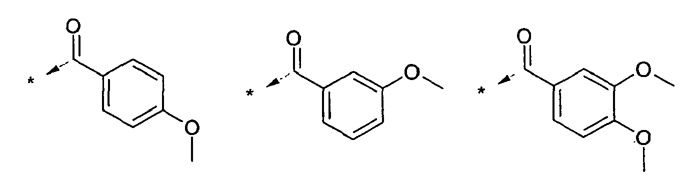 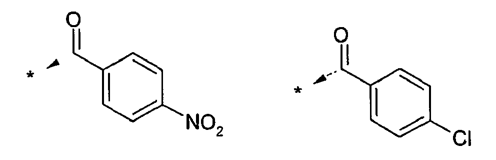 et R₄ représente le radical ci-après :

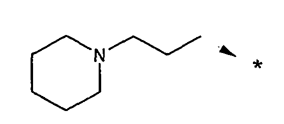

### C. Préparation selon le schéma réactionnel C :

Les composés de formule I selon l'invention dans laquelle A représente -CH₂- peuvent être préparés selon le schéma C suivant :

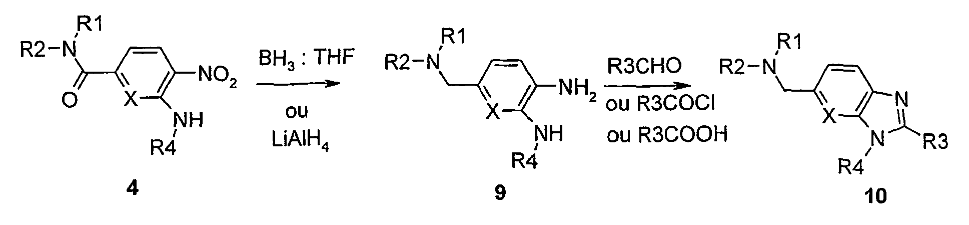

Comme décrit dans le schéma C, le dérivé (4) préparé selon le schéma réactionnel A, peut être réduit en composé (9) à l'aide de borane ou d'hydrure de lithium aluminium dans un solvant aprotique tel que le tétrahydrofuranne ou l'éther diéthylique à une température de 0 à 70° C, pendant 3 à 24 heures. La dianiline (9) peut être ensuite traitée par un aldéhyde en présence d'un oxydant tel que le nitrobenzène, à une température de 60-140° C pendant 2 à 24 heures dans un solvant aprotique tel que le diméthylformamide, pour conduire au benzimidazole (10). Alternativement, le dérivé (9) peut réagir soit avec un chlorure d'acide, soit avec un acide carboxylique en présence d'un agent de couplage tel que le diisopropylcarbodiimide (DIC), le dicyclohexylcarbodiimide (DCC), le chlorhydrate de 1-(3-diméthylaminopropyl)-3-éthylcarbodiimide (EDC), le carbonyldiimidazole (CDI), le 2-(1H-benzotriazole-1-yl)-1,1,3,3-tétraméthyluronium hexafluorophosphate (HBTU), le 2-(1H-benzotriazole-1-yl)-1,1,3,3-tétraméthyluronium tétrafluoroborate (TBTU) ou le O-(7-azobenzotriazol-1-yl)-1,1,3,3-tétraméthyluronium hexafluorophosphate (HATU) dans un solvant organique inerte tel que du chlorure de méthylène, tétrahydrofuranne ou diméthylformamide à température ambiante pendant 3 à 24 heures pour conduire à l'amide correspondante. L'amide ainsi obtenue conduit au benzimidazole (10) par traitement avec un acide, tel que l'acide acétique, l'acide chlorhydrique, l'acide polyphosphorique à une température de 20-100° C pendant 2 à 24 heures. Le dérivé (9) peut également réagir avec un imidate d'ester ou un dérivé chloroacétamide dans un solvant organique inerte tel que du diméthylformamide à une température de 20-100° C pendant 3 à 24 heures pour conduire au dérivé benzimidazole (10).

### Préparation selon le schéma réactionnel C' :

Les composés (10) peuvent également être préparés selon le schéma C' suivant :

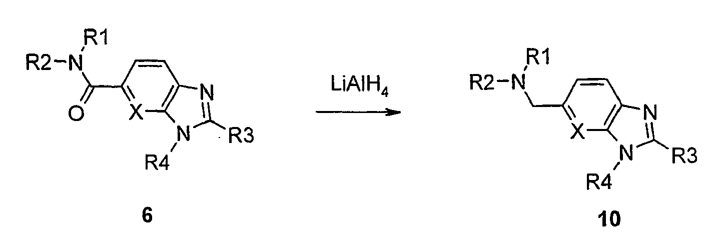

Comme décrit dans le schéma C', l'amide (6) préparée selon le schéma réactionnel A, peut être réduite en amine correspondante (10) à l'aide de borane ou d'hydrure de lithium aluminium dans un solvant aprotique tel que le tétrahydrofuranne ou l'éther diéthylique à une température de 0 à 70° C, pendant 1 à 6 heures.

### Exemple C1 : dichlorhydrate du 4-[6-{[bis(3-méthylbutyl)amino]méthyl}-1-(3-pipéridin-1-ylpropyl)-1H-benzimidazol-2-yl]benzoate de méthyle

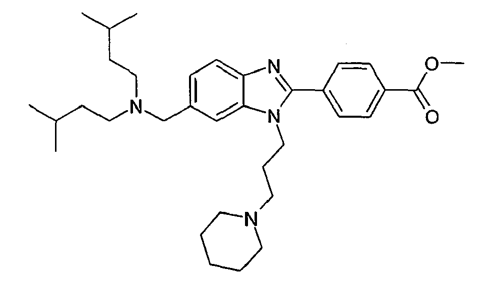

### Etape 1 : 4-{[bis(3-méthylbutyl)amino]méthyl}-N²-(3-pipéridin-1-ylpropyl)benzène-1,2-diamine

A une solution refroidie à 0° C de *N,N*-bis(3-méthylbutyl)-4-nitro-3-[(3-pipéridin-1-ylpropyl)amino]benzamide (1,6 g) est additionnée goutte à goutte une solution d'hydrure de lithium aluminium (36 mL ; 1N dans le THF). Le mélange est ramené à une température de 20° C puis chauffé au reflux pendant 6 heures et hydrolysé par de l'eau refroidie à 0° C suivie d'une solution de soude 1N. Après addition de dichlorométhane, le mélange est filtré sur célite. Après décantation du filtrat et extractions, les phases organiques réunies sont lavées à la soude 1N puis à la saumure, séchées sur Na₂SO₄ et concentrées sous pression réduite à 40° C pour donner le composé attendu sous forme d'une huile (1,23 g, 85 % rendement).

SM/CL : MM calculée = 402,7 ; m/z = 403,3 (MH+)

RMN ¹H (400 MHz, DMSO-*d₆*) : δ 0,81 (d, 12H), 1,28 (m, 4H), 1,38 (m, 2H), 1,48 (m, 6H), 1,71 (m, 2H), 2,31 (m, 10H), 3,01 (m, 2H), 3,29 (m, 2H), 4,28 (m, 2H), 4,6 (m, 1H), 6,30 (AB, 1H), 6,38 (s, 1H), 6,43 (AB, 1H).

### Etape 2 : Dichlorhydrate de 4-[6-{[bis(3-méthylbutyl)amino]méthyl}-1-(3-pipéridin-1-ylpropyl)-1H-benzimidazol-2-yl]benzoate de méthyle

A une solution du 4-{[bis(3-méthylbutyl)amino]méthyl}-*N*²-(3-pipéridin-1-ylpropyl)benzène-1,2-diamine (80 mg) dans le nitrobenzène (2 mL) est additionné le méthyl-4-formylbenzoate (33 mg, 1 eq). Le mélange est chauffé à 130° C pendant 18 heures. La purification du mélange par chromatographie éclair sur gel de silice (éluant : dichlorométhane 100 % à dichlorométhane / méthanol 7:3) donne le composé attendu sous forme de base libre. Le sel de chlorhydrate correspondant est formé par addition d'une solution d'acide chlorhydrique 1N dans l'éther diéthylique. Le précipité obtenu est filtré et séché pour donner le composé monochlorhydrate attendu (47 mg, 41 % rendement).

SM/CL : MM calculée = 546,8 ; m/z = 547,3 (MH+)

RMN ¹H (400 MHz, DMSO-*d₆*) : δ 0,86 (m, 12H), 1,21-1,75 (m,12 H), 2,36 (m, 2H), 2,81 (m, 2H), 3,05 (m, 6H), 3,91(s, 3H), 4,33 (m, 2H), 4,47 (d, 2H), 7,46 (AB, 1H), 7,79 (AB, 1H), 7,98 (AB, 1H), 8,16 (AB, 1H), 8,36 (s, 1H), 10,18 (s, 1H), 10,73 (s, 1H).

Selon le schéma réactionnel C et de façon analogue à la procédure décrite pour la synthèse du dichlorhydrate de 4-[6-{[bis(3-méthylbutyl)amino]méthyl}-1-(3-pipéridin-1-ylpropyl)-1*H-*benzimidazol-2-yl]benzoate de méthyle, les composés suivants ont été préparés : 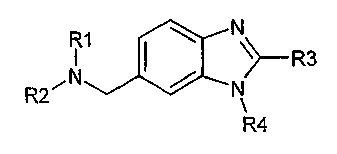 dans lesquels R₁R₂N représente l'un des radicaux ci-après :

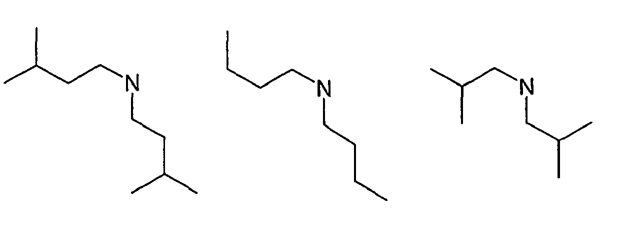

R₃ représente l'un des radicaux ci-après : 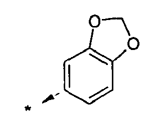 1 ou plusieurs substitutions choisies parmi : 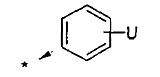 U = H, F, CI, Br, I, NO₂, OMe, SMe, Me, Et, iPr, tBu, CF₃, OCF₃, C(O)OMe, C(O)OEt,
C(O)Me, C(O)Et, C(O)NHMe, C(O)NH₂ 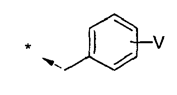 V = H, F, Cl, Br, I, NO₂, OMe, Me, Et, iPr, CF₃,OCF₃ 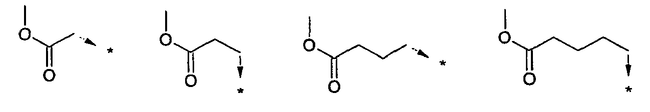 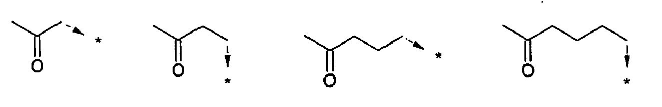 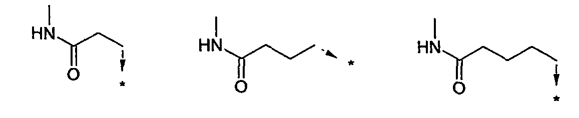 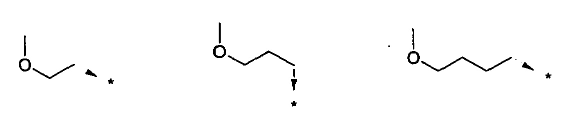 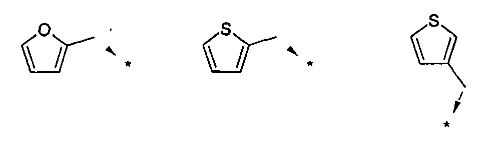 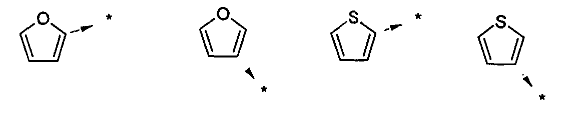 et R₄ représente le radical ci-après :

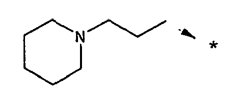

### D. Préparation selon le schéma réactionnel D :

Les composés de formule (I) selon l'invention dans laquelle A représente -C(O)-C(Rₐ)(R_{b})-, peuvent être préparés selon le schéma D suivant :

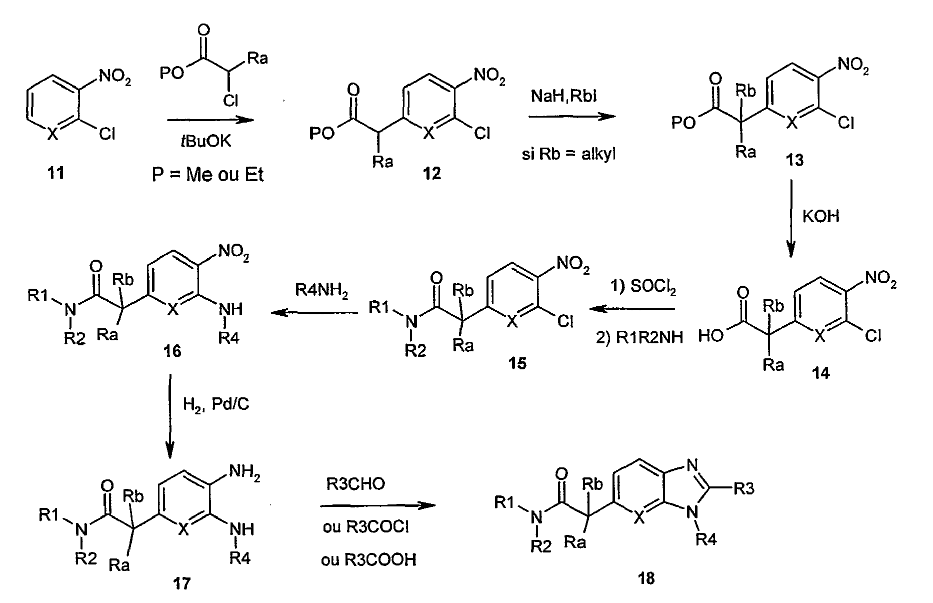

Comme décrit dans le schéma D, le dérivé (11) peut être alkylé en présence d'une base forte telle que le *tert*butylate de potassium, par un dérivé α-chloroester, dans un solvant aprotique polaire tel que le diméthylformamide à une température de 0-20° C pendant 0,5-2 heures, pour conduire au composé (12). Le dérivé (13) peut être éventuellement alkylé en présence d'une base forte telle que l'hydrure de sodium et d'un agent alkylant tel qu'un iodure d'alkyle dans un solvant aprotique tel que le diméthylformamide à une température de 0-20° C pendant 1-4 heures, pour conduire au composé (13). L'ester (13) peut-être saponifié en présence d'une base inorganique telle que l'hydroxyde de lithium ou potassium dans un mélange de solvants polaires tels que l'eau et le méthanol à une température de 20-80° C pendant 1-6 heures. L'acide carboxylique résultant (14) peut être couplé avec une amine primaire ou secondaire en présence d'un agent de couplage tel que le diisopropylcarbodiimide (DIC), le dicyclohexylcarbodiimide (DCC), le chlorhydrate de 1-(3-dimethylaminopropyl)-3-éthylcarbodiimide (EDC) ou le carbonyldiimidazole (CDI), avec ou sans du 1-hydroxybenzotriazole (HOBt) dans un solvant organique inerte tel que du chlorure de méthylène, tétrahydrofuranne ou diméthylformamide à température voisine de 20° C pendant 3 à 24 heures. Alternativement l'acide (14) peut-être traité avec du chlorure de thionyle ou d'oxalyle dans un solvant aprotique tel que le dichlorométhane ou toluène à une température de 40-60° C pendant 2-16 heures puis le chlorure d'acide ainsi obtenu peut réagir avec une amine primaire ou secondaire, en présence d'une base tertiaire telle que la triéthylamine, la diisopropyléthylamine dans un solvant aprotique tel que le dichlorométhane ou tétrahydrofuranne à une température de 0-20° C pendant 0,5-4 heures pour conduire à l'amide (15). Le traitement du dérivé fluoré ou chloré (15) par une amine primaire en présence d'une base inorganique telle que du carbonate de césium ou de potassium dans un solvant organique inerte tel que le diméthylformamide ou l'acétonitrile à une température de 20-100° C pendant 2 à 48 heures conduit au dérivé (16). La fonction nitro du composé (16) est réduite par traitement avec du chlorure d'étain dihydraté dans un solvant inerte tel que l'acétate d'éthyle ou le diméthylformamide à une température de 60-80° C pendant 3 à 15 heures, ou par hydrogénation catalytique en présence de palladium sur charbon 10 % dans un solvant inerte tel que le méthanol, éthanol, acétate d'éthyle ou un mélange de ces solvants, à une température de 18-25° C, pendant 2 à 8 heures pour conduire à la dianiline (17). La dianiline (17) peut ensuite être traitée par un aldéhyde en présence d'un oxydant tel que le nitrobenzène, ou le DDQ dans un solvant aprotique tel que le diméthylformamide, à une température de 60-140° C pendant 2 à 24 heures pour conduire au benzimidazole (18). Alternativement, le dérivé (17) peut réagir soit avec un chlorure d'acide, soit avec un acide carboxylique en présence d'un agent de couplage tel que le diisopropylcarbodiimide (DIC), le dicyclohexylcarbodiimide (DCC), le chlorhydrate de 1-(3-diméthylaminopropyl)-3-éthylcarbodiimide (EDC), le carbonyldiimidazole (CDI), le 2-(1H-benzotriazole-1-yl)-1,1,3,3-tétramethyluronium hexafluorophosphate (HBTU), le 2-(1H-benzotriazole-1-yl)-1,1,3,3-tétramethyluronium tetrafluoroborate (TBTU) ou le O-(7-azobenzotriazol-1-yl)-1,1,3,3-tétraméthyluronium hexafluorophosphate (HATU) dans un solvant organique inerte tel que du chlorure de méthylène, tétrahydrofuranne ou diméthylformamide à température ambiante pendant 3 à 24 heures pour conduire à l'amide correspondante. L'amide ainsi obtenue conduit au benzimidazole (18) par traitement avec un acide, tel que l'acide acétique, l'acide chlorhydrique, l'acide polyphosphorique à une température de 20-100° C pendant 2 à 24 heures. Le dérivé (17) peut également réagir avec un imidate d'ester ou un dérivé chloroacétamide dans un solvant organique inerte tel que du diméthylformamide à une température de 20-100° C pendant 3 à 24 heures pour conduire au dérivé benzimidazole (18).

### Exemple D1 : N,N-diisobutyl-2-[2-(4-méthoxyphényl)-1-(3-pipéridin-1-ylpropyl)-1H-benzimidazol-6-yl]-2-méthylpropanamide

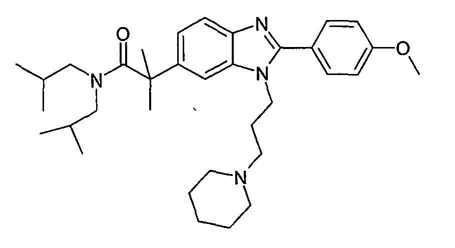

### Etape 1 : 2-(3-chloro-4-nitrophényl)propanoate d'éthyle

A une solution de DMF (80 ml) refroidie à 0° C, est additionné le *tert*-butylate de potassium (11,22 g, 2 eq). Une solution de 1-chloro-2-nitrobenzene (7,87 g, 1 eq) et de 2-chloropropanoate d'éthyle (7 ml, 1,1 eq) est additionnée goutte à goutte en 45 min au mélange en maintenant la température réactionnelle inférieure à 5° C. A la fin de l'addition, l'agitation est maintenue 2 heures à 0° C puis le mélange est hydrolysé à cette température par une solution d'acide chlorhydrique 1N et additionné d'acétate d'éthyle. Après décantation et extractions, les phases organiques réunies sont lavées avec de la saumure, séchées sur Na₂SO₄ et concentrées sous pression réduite. La purification par chromatographie éclair sur gel de silice (éluant : heptane / dichlorométhane 8:2 à 6:4) donne le composé attendu sous forme d'une huile jaune (8,28 g ; 64 % rendement).

RMN ¹H (400 MHz, DMSO-*d₆*) : δ 1,14 (t, 3H), 1,42 (d, 3H), 3,99 (q, 1H), 4,08 (m, 2H), 7,52 (AB, 1H), 7,71 (s, 1H), 8,05 (AB, 1H).

### Etape 2 : 2-(3-chloro-4-nitrophényl)-2-méthylpropanoate d'éthyle

A une suspension d'hydrure de sodium (60 % dans l'huile, 2,4 g, 1,1 eq) dans le DMF (15 ml), refroidie à 0° C, est additionnée goutte à goutte une solution de 2-(3-chloro-4-nitrophényl)propanoate d'éthyle (14,1 g). Après 1 heure d'agitation à cette température, une solution d'iodure de méthyle (3,72 ml, 1,1 eq) dans le DMF (40 ml) est additionnée goutte à goutte au mélange. L'agitation est poursuivie 3 heures à température ambiante. Le milieu réactionnel est refroidi à 0° C puis additionné d'acétate d'éthyle, d'eau saturée en hydrogénocarbonate de sodium goutte à goutte, puis d'eau. Après décantation et extractions, les phases organiques réunies sont lavées avec de la saumure, séchées sur Na₂SO₄ et concentrées sous pression réduite pour donner le composé attendu sous forme d'une huile qui cristallise. Les cristaux sont lavés à l'heptane et séchés (13,8 g ; 94 % rendement).

RMN ¹H (400 MHz, DMSO-*d₆*) : δ 1,12 (t, 3H), 1,54 (s, 6H), 4,09 (q, 1H), 7,50 (AB, 1H), 7,66 (s, 1H), 8,04 (AB, 1H).

### Etape 3 : acide 2-(3-chloro-4-nitrophényl)-2-méthylpropanoique

A une solution de 2-(3-chloro-4-nitrophényl)-2-méthylpropanoate d'éthyle (1 g) dans le méthanol (20 ml) est additionnée à une température voisine de 20° C, une solution d'hydroxyde de potassium 2N (18 ml). Le mélange est ensuite chauffé à 80° C pendant 1,5 heures puis refroidi à température ambiante. Le méthanol est évaporé par concentration du mélange sous pression réduite. La phase aqueuse restante est lavée au dichlorométhane puis refroidie à 0° C et acidifiée par de l'acide acétique. Après addition de dichlorométhane, décantation et extractions, les phases organiques réunies sont lavées avec de la saumure, séchées sur Na₂SO₄ et concentrées sous pression réduite pour donner le composé attendu sous forme d'une huile qui cristallise (852 mg, 95 % rendement).

RMN ¹H (400 MHz, DMSO-*d₆*): δ 1,52 (s, 6H), 7,53 (AB, 1H), 7,66 (s, 1H), 8,04 (AB, 1H), 12,72 (s, 1H).

### Etape 4 : 2-(3-chloro-4-nitrophényl)-N,N-diisobutyl-2-méthylpropanamide

A une solution d'acide 2-(3-chloro-4-nitrophényl)-2-méthylpropanoique (500 mg) dans le dichlorométhane (1 ml) est additionné le chlorure de thionyle (0,54 ml, 4 eq). Le mélange est chauffé au reflux pendant 16 heures puis refroidi à température ambiante. Le solvant est évaporé sous pression réduite à 40° C (co-évaporation avec du toluène). A une solution du chlorure d'acide ainsi obtenu dans le dichlorométhane (1 ml), refroidie à 0° C, sont successivement additionnées la diisopropyléthylamine (0,42 ml, 1,2 eq) et la diisobutylamine (0,36 ml, 1 eq). A la fin de l'addition, l'agitation est poursuivie 3 heures à température ambiante puis le mélange est concentré sous pression réduite à 40° C. Le résidu est dissous dans l'éther éthylique et la phase organique est lavée successivement avec de la soude 1N, une solution saturée en hydrogénocarbonate de sodium, de la saumure puis séchée sur Na₂SO₄ et concentrée sous pression réduite à 40° C. La purification par chromatographie éclair sur gel de silice (éluant : heptane / acétate d'éthyle 8:2 à 7:3) donne le composé attendu sous forme d'une huile qui cristallise (0,585 g ; 82 % rendement).

SM/CL : MM calculée = 354,9 ; m/z = 355,2 (MH+)

RMN ¹H (400 MHz, CDCl₃) : δ 0,58 (d, 6H), 0,90 (d, 6H), 1,58 (m, 6H), 1,74 (m, 1H), 1,95 (m, 1H), 2,65 (d, 2H), 3,27 (d, 2H), 7,30 (AB, 1H), 7,44 (s, 1H), 7,91 (AB, 1H).

### Etape 5 : N,N-diisobutyl-2-méthyl-2-{4-nitro-3-[(3-pipéridin-1-ylpropyl)amino]phényl} propanamide

Un mélange de 2-(3-chloro-4-nitrophényl)-*N,N*-diisobutyl-2-méthylpropanamide (2,39 g), de 3-pipéridino-propylamine (1,9 g, 2 eq) et de carbonate de potassium (1,8 g, 2 eq) dans le DMF (40 mL) est chauffé à 100° C pendant 24 heures puis refroidi à température ambiante. Le milieu est additionné d'eau et d'acétate d'éthyle. Après décantation et extractions, les phases organiques réunies sont lavées avec de la saumure, séchées sur Na₂SO₄ et concentrées sous pression réduite. La purification du résidu obtenu par chromatographie éclair sur gel de silice (éluant : dichlorométhane 100 % à dichlorométhane / méthanol 6:4) donne le composé attendu sous forme d'une huile jaune (1,6 g, 51 % rendement).

SM/CL : MM calculée = 460,7 ; m/z = 461,4 (MH+)

RMN¹H (400 MHz, CDCl₃): δ 0,57 (d, 6H), 0,89 (d, 6H), 1,50 (m, 2H), 1,56 (m, 6H), 1,63 (m, 4H), 1,77 (m, 1H), 1,89 (m, 3H), 2,43 (m, 6H), 2,75 (d, 2H), 3,29 (d, 2H), 3,32 (m, 2H), 6,58 (AB, 1H), 6,67 (s, 1H), 8,15 (AB, 1H), 8,29 (t, 1H).

### Etape 6 : 2-{4-amino-3-[(3-pipéridin-1-ylpropyl)amino]phényl}-N,N-diisobutyl-2-méthylpropanamide

Dans un autoclave sont additionnés le *N,N*-diisobutyl-2-méthyl-2-{4-nitro-3-[(3-pipéridin-1-ylpropyl)amino]phényl}propanamide (1,6 g) en solution dans un mélange d'acétate d'éthyle/ éthanol 2:1 (100 mL) et le palladium sur charbon 10 % (160 mg). Après 4 heures d'agitation sous atmosphère d'hydrogène (3 bars) à une température d'environ 20° C, le catalyseur est éliminé par filtration sur célite et le filtrat est concentré sous pression réduite à 40° C pour donner le composé attendu sous forme d'une huile (1,4 g, 94 % rendement).

SM/CL : MM calculée = 430,7 ; m/z = 431,4 (MH+)

RMN ¹H (400 MHz, CDCl₃) : δ 0,45 (d, 6H), 0,79 (d, 6H), 1,35 (m, 8H), 1,49 (m, 4H), 1,70 (m, 3H), 1,85 (m, 1H), 1,89 (m, 3H), 2,33 (m, 6H), 2,79 (d, 2H), 2,97 (t, 2H), 3,11 (m, 2H), 4,45 (m, 2H), 6,18 (s, 1H), 6,30 (AB, 1H), 6,48 (AB, 1H).

### Etape 7 : chlorhydrate de N,N-diisobutyl-2-[2-(4-méthoxyphényl)-1-(3-pipéridin-1-ylpropyl)-1H-benzimidazol-6-yl]-2-méthylpropanamide

Un mélange de 2-{4-amino-3-[(3-pipéridin-1-ylpropyl)amino]phényl}-*N,N*-diisobutyl-2-méthylpropanamide (34 mg) et de *p-*anisaldéhyde (13 mg) dans le nitrobenzène (1 mL) est chauffé à 120° C pendant 24 heures puis refroidi à température ambiante. La purification du mélange par chromatographie éclair sur gel de silice (éluant : dichlorométhane 100 % à dichlorométhane / méthanol 85:15) donne le composé attendu sous forme de base libre. Le sel de chlorhydrate correspondant est formé par addition d'une solution d'acide chlorhydrique 1N dans l'éther diéthylique. Le précipité obtenu est filtré, lavé à l'éther diéthylique et séché pour donner le composé monochlorhydrate attendu (12 mg, 60 % rendement).

SM/CL : MM calculée = 546,8 ; m/z = 547,4 (MH+)

RMN¹H (400 MHz, CDCl₃): δ 0,44 (d, 6H), 0,83 (d, 6H), 1,30 (m, 2H), 1,58 (s, 6H), 1,71 (m, 6H), 2,18 (m, 2H), 2,72 (m, 4H), 3,01 (m, 2H), 3,21 (m, 4H), 3,89 (s, 3H), 4,54 (t, 2H), 7,23 (AB, 2H), 7,28 (m, 1H), 7,76 (AB, 1H), 7,86 (m, 3H), 10,41 (s, 1H).

Selon le schéma réactionnel D et de façon analogue à la procédure décrite pour la synthèse du chlorhydrate de *N,N*-diisobutyl-2-[2-(4-méthoxyphényl)-1-(3-pipéridin-1-ylpropyl)-1*H-*benzimidazol-6-yl]-2-méthylpropanamide, les composés suivants ont été préparés : 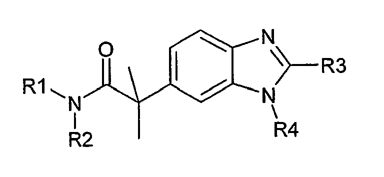 dans lesquels R₁R₂N représente l'un des radicaux ci-après : 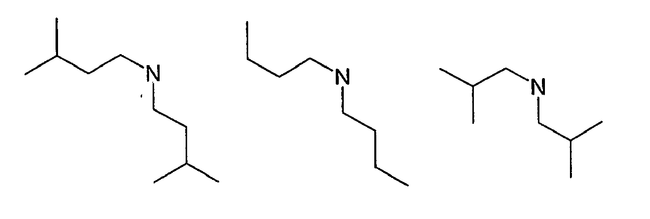 R₃ représente l'un des radicaux ci-après : 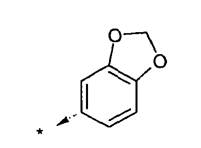 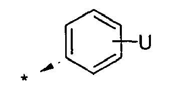 1 ou plusieurs substitutions choisies parmi :
U = H, F, Cl, Br, I, NO₂, OMe, Me, Et, iPr, tBu, CF₃, OCF₃, C(O)OMe, 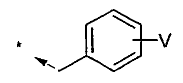 V = H, NO₂, OMe 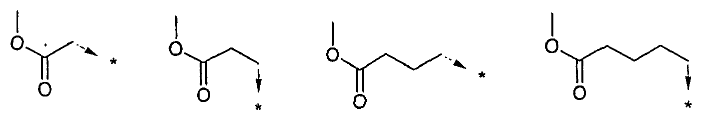 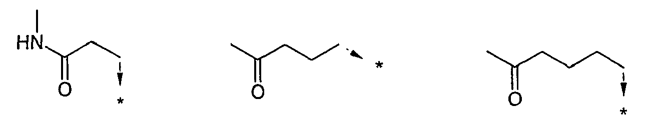 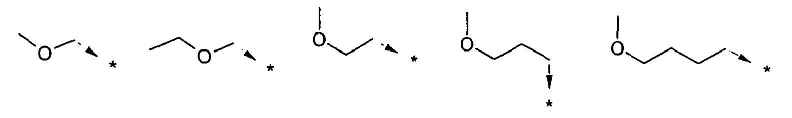 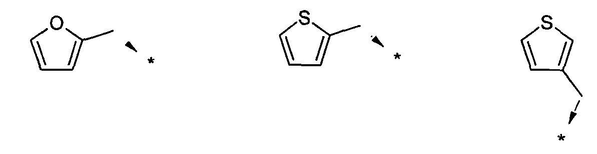 et R₄ représente l'un des radicaux ci-après :

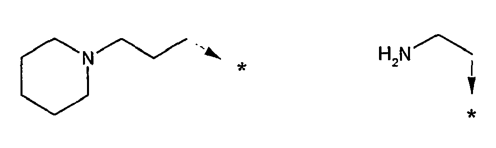

La présente demande a également pour objet un procédé de préparation d'un composé de formule (I) tel que défini ci-dessus, caractérisé en ce que l'on traite le composé de formule générale : 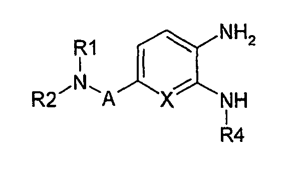 dans laquelle A, X, R₁, R₂, R₄ ont la signification indiquée ci-dessus,
i) soit par un aldéhyde de formule générale R₃CHO dans laquelle R₃ a la signification indiquée ci-dessus, en présence d'un oxydant ;
ii) soit par un chlorure d'acide de formule générale R₃COCl dans laquelle R₃ a la signification indiquée ci-dessus, en présence d'un acide ;
iii) soit par un acide carboxylique de formule générale R₃C(O)OH dans laquelle R₃ a la signification indiquée ci-dessus, en présence d'un agent de couplage suivi du traitement de l'amide ainsi formée par un acide.
iv) soit par un dérivé chloroacétamide de formule générale Z"₃-NH-C(O)CH₂Cl dans laquelle Z"₃ a la signification indiquée ci-dessus, en présence d'une base tertiaire et de soufre.

Lors du traitement par l'aldéhyde R₃CHO, l'oxydant utilisé peut être par exemple le nitrobenzène. Lors du traitement par un chlorure d'acide R₃COCl, l'acide utilisé peut être l'acide acétique. De même, lors du traitement par l'acide carboxylique R₃C(O)OH, puis le traitement de l'amide ainsi formée, l'acide utilisé peut être l'acide acétique.

L'invention a également pour objet un composé de formule générale **(I)** 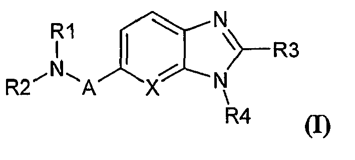 sous forme racémique, d'énantiomère ou toutes combinaisons de ces formes et dans laquelle :
A représente -CH₂- ,-C(O)-, -C(O)-C(Rₐ)(R_{b})- ;
X représente -CH- ou un atome d'azote ;
Rₐ et R_{b} représentent, indépendamment, l'atome d'hydrogène ou un radical (C₁-C₆)alkyle;
R₁ représente l'atome d'hydrogène ou un radical (C₁-C₈)alkyle;
R₂ représente un radical (C₁-C₈)alkyle ;
ou bien R₁ et R₂ forment ensemble, avec l'atome d'azote auquel ils sont rattachés, un hétérobicycloalkyle ou un hétérocycloalkyle éventuellement substitué par un ou
plusieurs substituants (C₁-C₆)alkyle identiques ou différents ;
R₃ représente -(CH₂)ₚ-Z₃, -C(O)-Z'₃, -CH(OH)-Z'₃ ou -C(O)-NH-Z"₃ ;
   Z₃ représente un radical (C₁-C₆)alkyle, (C₁-C₆)alkoxy, (C₁-C₆)alkyl-carbonyle, (C₁-C₆)alkoxy-carbonyle, (C₁-C₆)alkyl-aminocarbonyle, (C₃-C₇)cycloalkyle, hétérocycloalkyle, aryle ou hétéroaryle,
   les radicaux (C₃₋C₇) cycloalkyle et hétérocycloalkyle étant éventuellement substitués par un ou plusieurs radicaux identiques ou différents choisis parmi (C₁-C₆)alkyle et oxy ;
   le radical aryle étant éventuellement substitué par un ou plusieurs substituants identiques ou différents choisis parmi : halo, nitro ou -(CH₂)ₚ,-V₃-Y₃ ;
   V₃ représente -O-, -S-, -C(O)-, -C(O)-O-, -NH-C(O)-, -C(O)-NR'₃-, -NH-C(O)-NR'₃- ou une liaison covalente ;
   Y₃ représente l'atome d'hydrogène ou un radical (C₁-C₆)alkyle éventuellement substitué par un ou plusieurs radicaux halo identiques ou différents ;
   ou bien Z₃ représente un radical de formule 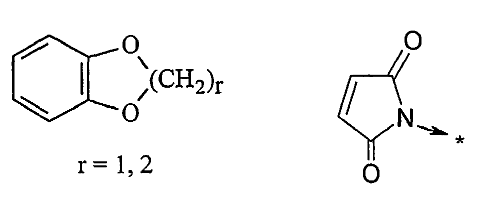 Z'₃ représente un radical aryle éventuellement substitué par un ou plusieurs substituants identiques ou différents choisis parmi : halo, nitro et -(CH₂)_{p"}-V'₃-Y'₃;
   V'₃ représente -O-, -C(O)-, -C(O)-O-, -NH-C(O)-, -C(O)-NR'₃- ou une liaison covalente ;
   Y'₃ représente l'atome d'hydrogène ou un radical (C₁-C₆)alkyle éventuellement substitué par un ou plusieurs radicaux halo identiques ou différents ;
   R'₃ représente l'atome d'hydrogène, un radical (C₁-C₆)alkyle ou (C₁-C₆)alkoxy
   Z"₃ représente l'atome d'hydrogène ou un radical -A₃-C(O)-O-((C₁-C₆)alkyle), -A₃-C(O)-NH-((C₁-C₆)alkyle) ou -A₃-O-((C₁-C₆)alkyle) ;
   A₃ représente une chaîne hydrocarbonée linéaire ou ramifiée contenant de 1 à 6 atomes de carbone, ou un radical arylène ;
   p, p' et p" représentent, indépendamment, un entier de 0 à 4 ;
R₄ représente un radical de formule-(CH₂)ₛ-R'₄ ;
R'₄ représente un hétérocycloalkyle contenant au moins un atome d'azote et éventuellement substitué par (C₁-C₆)alkyle ; un hétéroaryle contenant au moins un atome d'azote et éventuellement substitué par (C₁-C₆)alkyle ; ou un radical de formule -NW₄W'₄ ;
   W₄ représente l'atome d'hydrogène ou (C₁-C₈)alkyle ;
   W'₄ représente un radical de formule -(CH₂)_{s'}-Z₄ dans laquelle Z₄ représente l'atome d'hydrogène, un radical (C₁-C₈)alkyle ou (C₃-C₇)cycloalkyle ;
   s et s' représentent, indépendamment, un entier de 0 à 6 ; ou un sel pharmaceutiquement acceptable de ce dernier.

De préférence, l'invention concerne des composés de formule I telle que définie ci-dessus et caractérisés en ce que R₁ représente l'atome d'hydrogène ou un radical (C₁-C₈)alkyle, et R₂ représente un radical (C₁-C₈)alkyle ; ou un sel pharmaceutiquement acceptable de ce dernier.

L'invention concerne de préférence également des composés de formule I telle que définie ci-dessus et caractérisés en ce que X représente -CH- ; ou un sel pharmaceutiquement acceptable de ce dernier.

De manière préférentielle, l'invention concerne aussi des composés de formule I telle que définie ci-dessus et caractérisés en ce que A représente -CH₂- ; ou un sel pharmaceutiquement acceptable de ce dernier.

De manière préférentielle, l'invention concerne aussi des composés de formule I telle que définie ci-dessus et caractérisés en ce que A représente -C(O)-C(Rₐ)(R_{b})- et Rₐ et R_{b} représentent, indépendamment, le radical méthyle ; ou un sel pharmaceutiquement acceptable de ce dernier.

De manière préférentielle également, l'invention concerne des composés de formule I telle que définie ci-dessus et caractérisés en ce que A représente -C(O)- ; ou un sel pharmaceutiquement acceptable de ce dernier.

De manière très préférentielle, l'invention concerne des composés de formule I telle que définie ci-dessus et caractérisés en ce que
R₄ représente un radical de formule-(CH₂)ₛ-R'₄ ;
R'₄ représente un hétérocycloalkyle contenant au moins un atome d'azote choisi parmi pipéridine et pyrrolidine, hétérocycle éventuellement substitué par (C₁-C₆)alkyle ; ou un radical de formule -NW₄W'₄ ;
W₄ représente l'atome d'hydrogène ou (C₁-C₈)alkyle ;
W'₄ représente un radical de formule -(CH₂)_{s'}-Z₄ dans laquelle Z₄ représente l'atome d'hydrogène ou un radical (C₁-C₈)alkyle ;
s et s' représentent, indépendamment, un entier de 0 à 6 ; ou un sel pharmaceutiquement acceptable de ce dernier.

De manière très préférentielle également, l'invention concerne des composés de formule I telle que définie ci-dessus et caractérisés en ce que R₃ représente -(CH₂)ₚ-Z₃ ou -C(O)-Z'₃ ;
Z₃ représente un radical (C₁-C₆)alkoxy, (C₁-C₆)alkyl-carbonyle, (C₁-C₆)alkoxy-carbonyle, (C₁-C₆)alkyl-aminocarbonyle, aryle ou hétéroaryle,
le radical aryle étant éventuellement substitué par un ou plusieurs substituants identiques ou différents de formule -(CH₂)_{p'}-V₃-Y₃;
V₃ représente -O-, -C(O)-, -C(O)-O- ou -C(O)-NH- ;
Y₃ représente un radical (C₁-C₆)alkyle ;
Z'₃ représente un radical aryle éventuellement substitué par un ou plusieurs substituants identiques ou différents de formule -(CH₂)_{p"}- V'₃-Y'₃ ;
V'₃ représente -O- ;
Y'₃ représente un radical (C₁-C₆)alkyle ;
p, p' et p" représentent, indépendamment, un entier de 0 à 4 ; ou un sel pharmaceutiquement acceptable de ce dernier ;
et plus particulièrement le radical aryle est le radical phényle et le radical hétéroaryle est choisi parmi thiényle et furyle.

Les composés I de la présente invention possèdent d'intéressantes propriétés pharmacologiques. C'est ainsi que l'on a découvert que les composés 1 de la présente invention possèdent une bonne affinité pour certains sous-types de récepteurs des mélanocortines, en particulier des récepteurs MC4.

Les composés de la présente invention peuvent ainsi être utilisés dans différentes applications thérapeutiques. Ils peuvent avantageusement être utilisés pour traiter les états pathologiques ou les maladies métaboliques, du système nerveux ou dermatologiques dans lesquels un ou plusieurs récepteurs des mélanocortines sont impliqués tels que : les états inflammatoires, les troubles de l'homéostasie énergétique, de la prise de nourriture, les désordres pondéraux (l'obésité, la cachexie, l'anorexie), les désordres de l'activité sexuelle (les troubles de l'érection), la douleur neuropathique, mais également les troubles mentaux (l'anxiété, la dépression), les toxicomanies, les maladies de la peau (l'acné, les dermatoses, les cancers cutanés, les mélanomes). Ils peuvent également être utilisés pour stimuler la régénération nerveuse. On trouvera ci-après, dans la partie expérimentale, une illustration des propriétés pharmacologiques des composés de l'invention.

La présente demande a également pour objet des compositions pharmaceutiques contenant, à titre de principe actif, au moins un produit de formule I telle que définie ci-dessus, ainsi que les sels pharmaceutiquement acceptables dudit produit de formule I, en association avec un support pharmaceutiquement acceptable.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

La présente demande a également pour objet l'utilisation des composés selon la présente invention, pour la préparation d'un médicament pour le traitement des désordres pondéraux tels que l'obésité, la cachexie et l'anorexie, des troubles mentaux tels que l'anxiété et la dépression, de la douleur neuropathique, des désordres de l'activité sexuelle tels que les troubles de l'érection.

La composition pharmaceutique peut être sous forme d'un solide, par exemple, des poudres, des granules, des comprimés, des gélules ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrées par intraveineuse.

Tous les termes techniques et scientifiques utilisés dans le présent texte ont la signification connue de l'homme de l'art. Par ailleurs, tous les brevets (ou demandes de brevet) ainsi que les autres références bibliographiques sont incorporés par référence.

### Partie expérimentale :

Les composés selon l'invention obtenus selon les procédures des exemples A, B, C, C' et D précédemment décrites, sont rassemblés dans le tableau ci-dessous.

Les composés sont caractérisés par leur temps de rétention (tr) et leur pic moléculaire déterminé par spectrométrie de masse (MH+).

Pour la spectrométrie de masse, un spectromètre de masse simple quadripôle (Micromass, modèle Platform) équipé d'une source *electrospray* est utilisé avec une résolution de 0,8 Da à 50 % de vallée. Un calibrage est effectué mensuellement entre les masses 80 et 1000 Da à l'aide d'un mélange calibrant d'iodure de sodium et d'iodure de rubidium en solution dans un mélange isopropanol / eau (1/1 Vol.).

Pour la chromatographie liquide, un système Waters incluant un dégazeur en ligne, une pompe quaternaire Waters 600, un injecteur plaque Gilson 233 et un détecteur UV Waters PAD 996, est utilisé.

Les conditions d'élution employées sont les suivantes :
Eluant : **A** eau + 0,04 % acide trifluoroacétique ; **B** acétonitrile

| **T (min)** | **A %** | **B %** |
|---|---|---|
| 1 | 95 | 5 |
| 8,5 | 5 | 95 |
| 10,5 | 5 | 95 |
| 10,6 | 95 | 5 |
| 14,9 | 95 | 5 |
| 15,0 | 95 | 5 |

Débit : 1 ml/min ; Injection : 10 µl ; Colonne : Uptisphere ODS 3 µm 75*4,6 mm i.d.

Ces exemples sont présentés pour illustrer les procédures ci-dessus et ne doivent en aucun cas être considérés comme une limite à la portée de l'invention.

| **Exemples** | **Structures moléculaires** | **[M+H]+** | **tr (min)** |
|---|---|---|---|
| 1 | 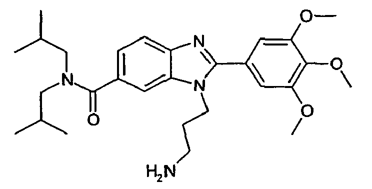 | 497,2 | 8,0 |
| 2 | 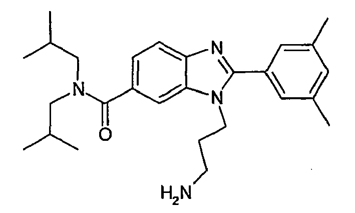 | 435,2 | 8,1 |
| 3 | 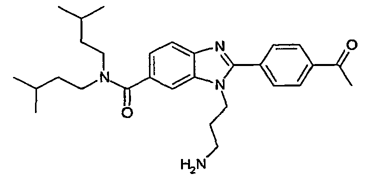 | 477,2 | 8,6 |
| 4 | 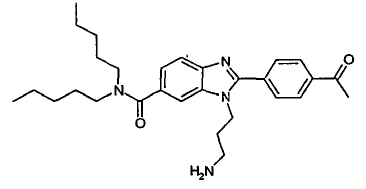 | 477,3 | 8,6 |
| 5 | 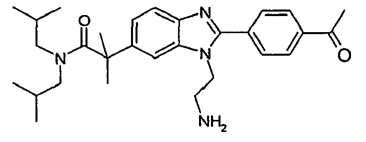 | 477,3 | 7,7 |
| 6 | 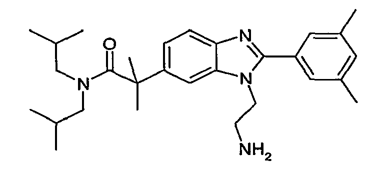 | 463,3 | 7,6 |
| 7 | 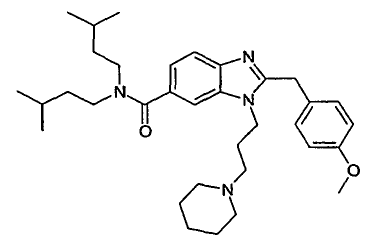 | 547,5 | 8,5 |
| 8 | 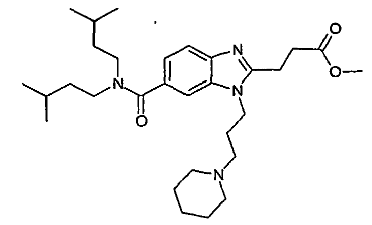 | 513,5 | 8,3 |
| 9 | 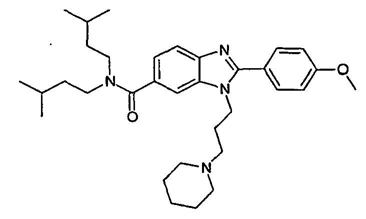 | 533,5 | 8,5 |
| 10 | 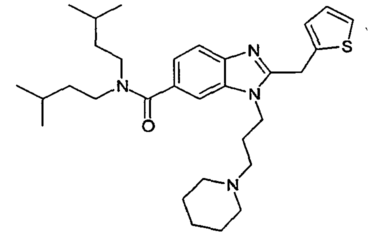 | 523,4 | 8,6 |
| 11 | 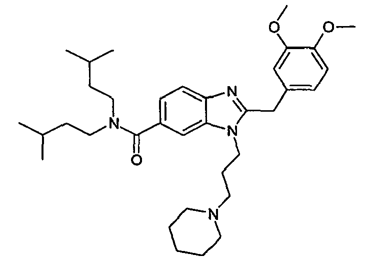 | 577,4 | 8,3 |
| 12 | 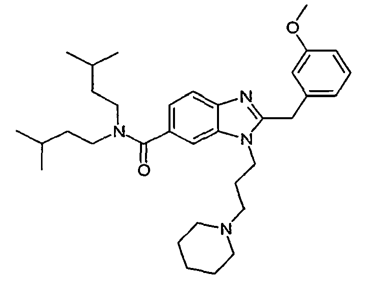 | 547,4 | 8,6 |
| 13 | 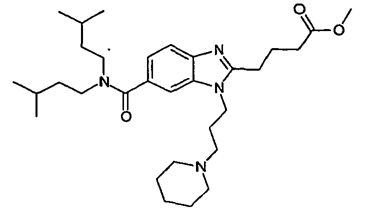 | 527,4 | 8,1 |
| 14 | 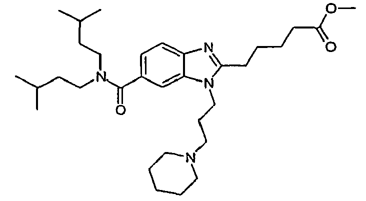 | 541,4 | 8,1 |
| 15 | 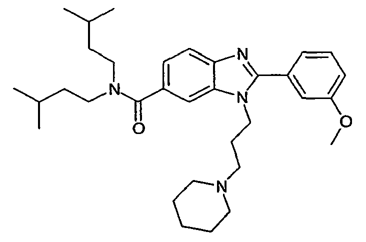 | 533,4 | 8,7 |
| 16 | 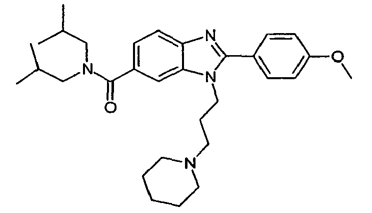 | 505,4 | 8,0 |
| 17 | 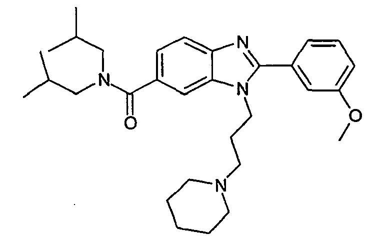 | 505,4 | 8,2 |
| 18 | 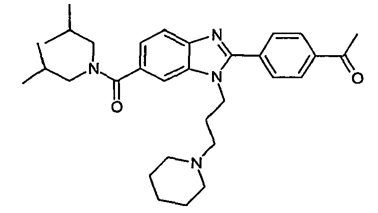 | 517,4 | 8,3 |
| 19 | 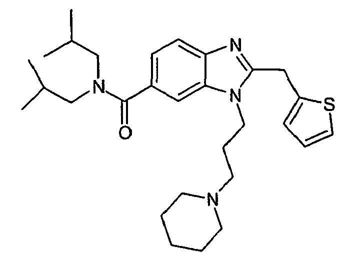 | 495,3 | 8,6 |
| 20 | 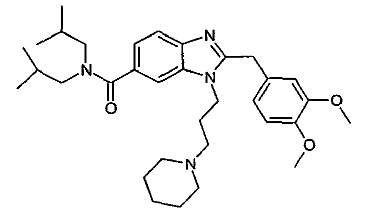 | 549,4 | 8,3 |
| 21 | 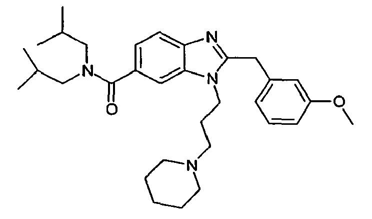 | 519,3 | 8,6 |
| 22 | 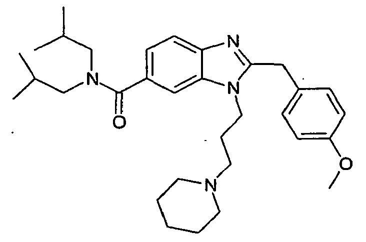 | 519,4 | 8,5 |
| 23 | 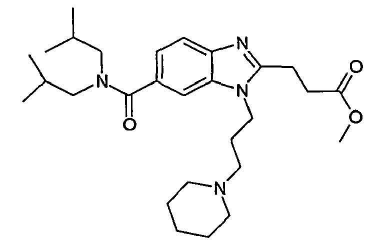 | 485,4 | 8,2 |
| 24 | 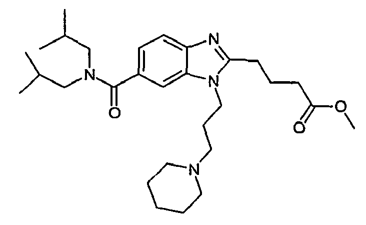 | 499,4 | 8,2 |
| 25 | 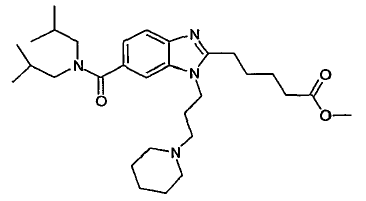 | 513,4 | 8,2 |
| 26 | 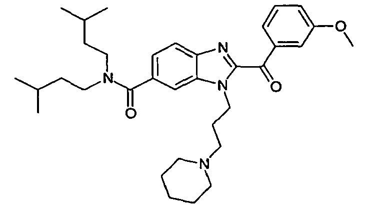 | 561,4 | 10,3 |
| 27 | 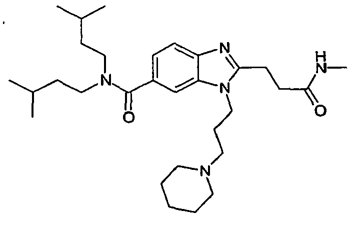 | 512,4 | 8,5 |
| 28 | 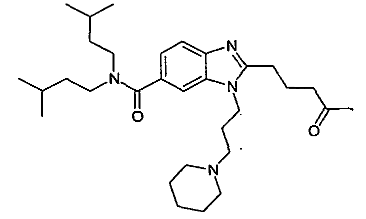 | 511,4 | 8,5 |
| 29 | 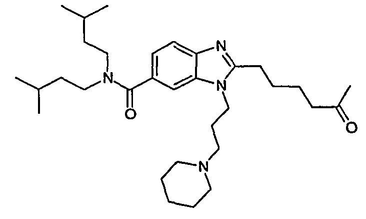 | 525,4 | 8,6 |
| 30 | 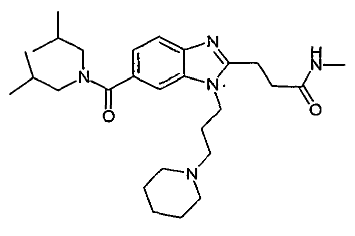 | 484,4 | 8,1 |
| 31 | 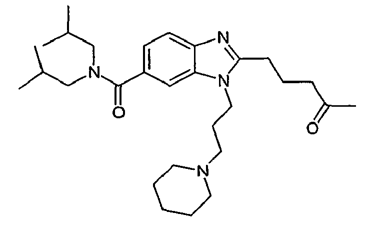 | 483,4 | 8,1 |
| 32 | 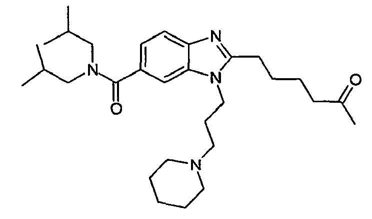 | 497,4 | 8,1 |
| 33 | 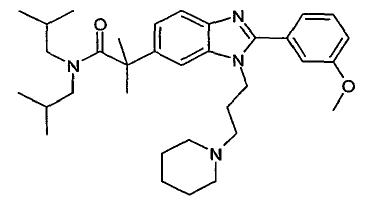 | 547,4 | 8,7 |
| 34 | 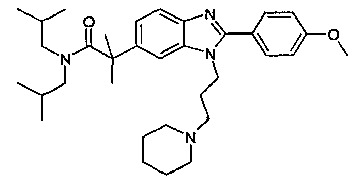 | 547,4 | 8,5 |
| 35 | 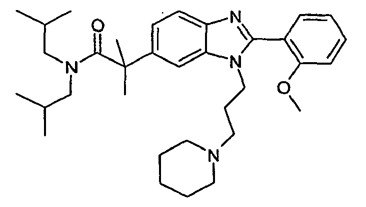 | 547,4 | 8,5 |
| 36 | 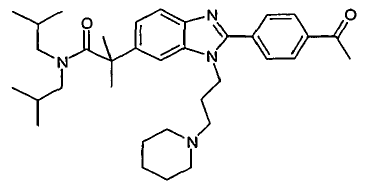 | 559,4 | 8,8 |
| 37 | 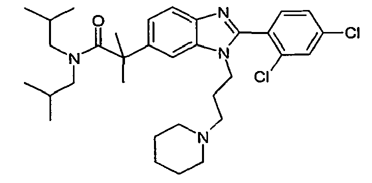 | 585,3 | 9,5 |
| 38 | 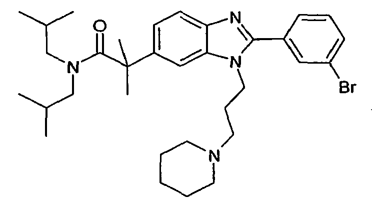 | 595,3 | 9,2 |
| 39 | 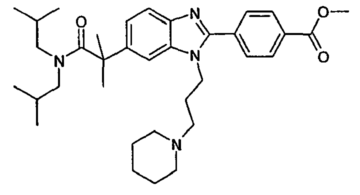 | 575,3 | 9,0 |
| 40 | 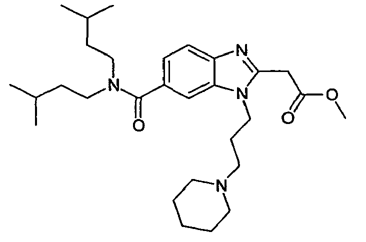 | 499,4 | 8,9 |
| 41 | 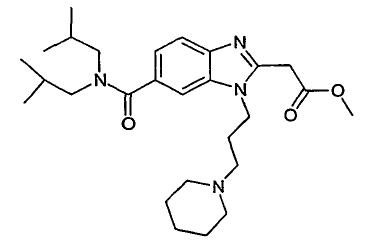 | 471,4 | 8,4 |
| 42 | 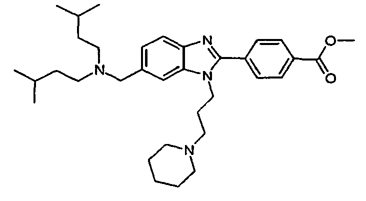 | 547,4 | 8,1 |
| 43 | 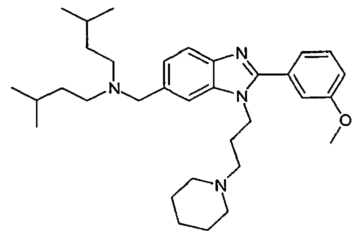 | 519,4 | 8,0 |
| 44 | 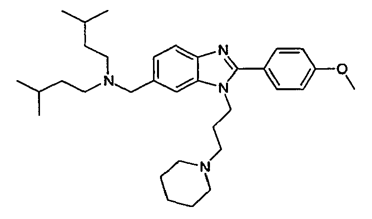 | 519,4 | 8,3 |
| 45 | 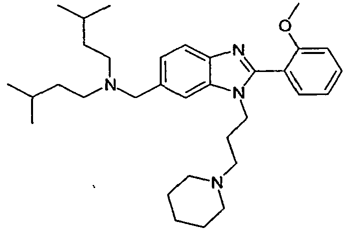 | 519,4 | 7,9 |
| 46 | 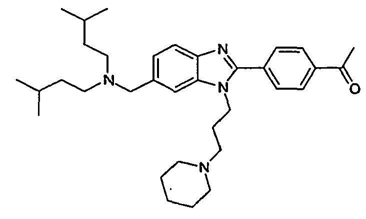 | 531,4 | 8,0 |
| 47 | 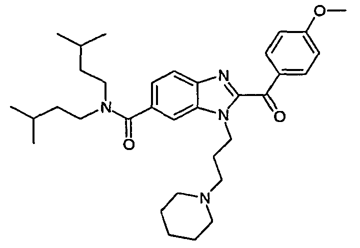 | 561,4 | 10,1 |
| 48 | 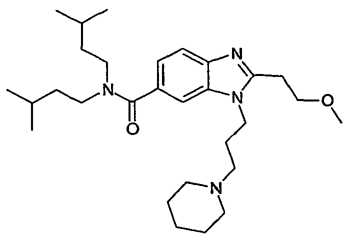 | 485,4 | 8,5 |
| 49 | 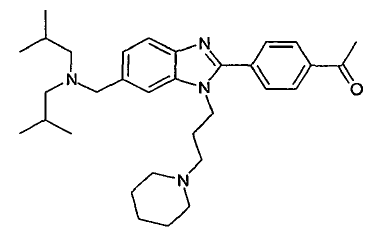 | 503,3 | 7,4 |
| 50 | 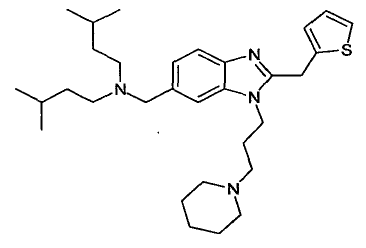 | 509,4 | 7,7 |
| 51 | 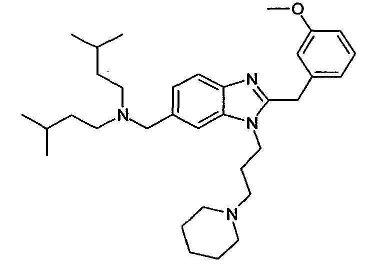 | 533,4 | 7,8 |
| 52 | 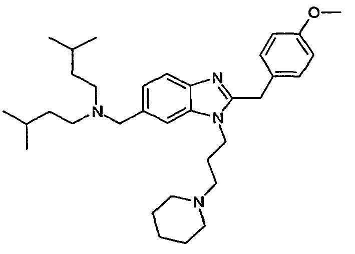 | 533,4 | 7,7 |
| 53 | 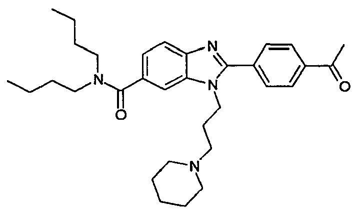 | 517,4 | 8,5 |
| 54 | 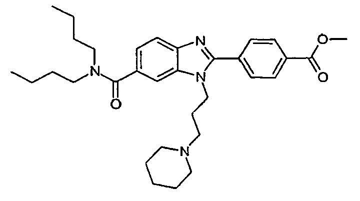 | 533,4 | 8,7 |
| 55 | 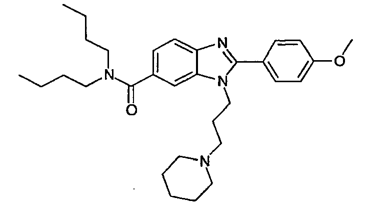 | 505,4 | 8,2 |
| 56 | 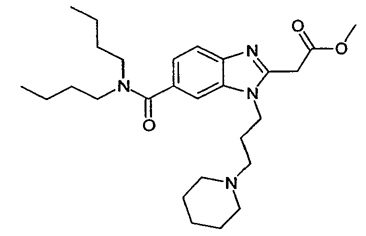 | 471,4 | 8,2 |
| 57 | 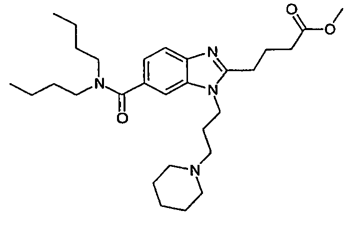 | 499,5 | 7,9 |
| 58 | 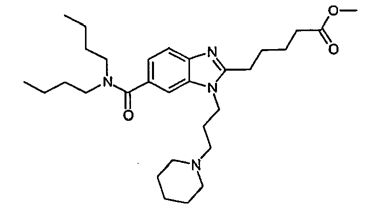 | 513,5 | 8,0 |
| 59 | 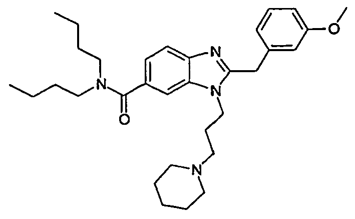 | 519,4 | 8,3 |
| 60 | 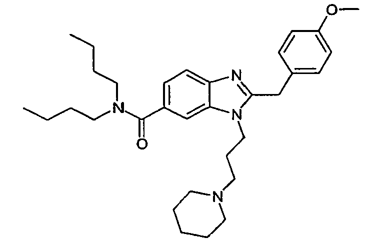 | 519,4 | 8,3 |
| 61 | 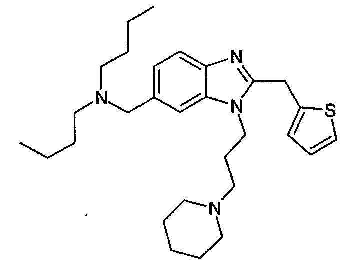 | 481,4 | 7,5 |
| 62 | 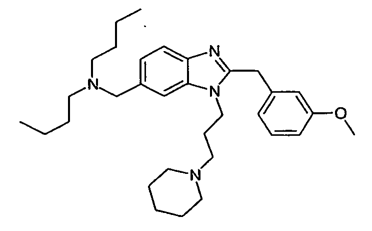 | 505,5 | 7,5 |
| 63 | 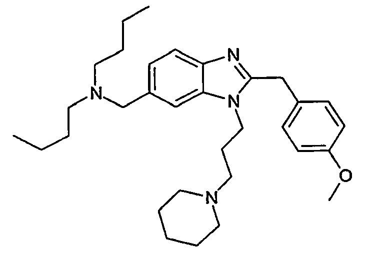 | 505,5 | 7,5 |
| 64 | 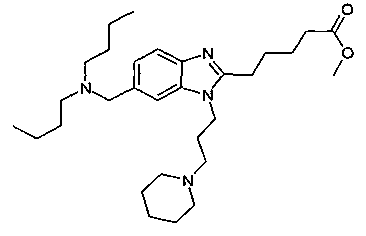 | 499,5 | 7,3 |
| 65 | 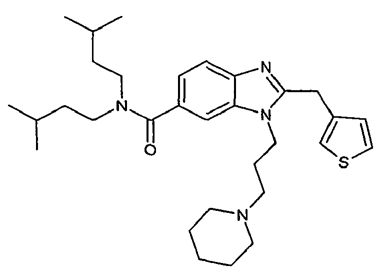 | 523,2 | 7,8 |
| 66 | 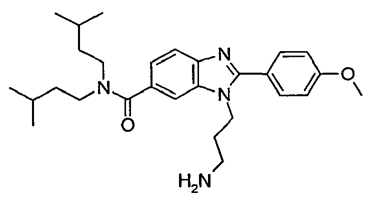 | 465,4 | 8,2 |
| 67 | 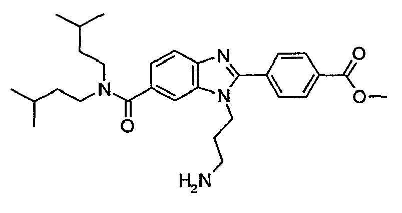 | 493,4 | 8,7 |
| 68 | 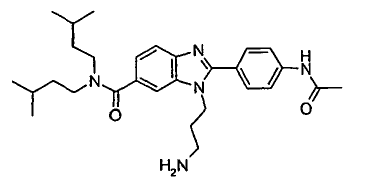 | 492,4 | 8,1 |
| 69 | 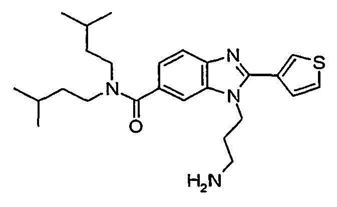 | 441,4 | 8,3 |
| 70 | 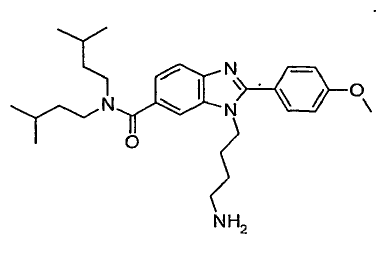 | 479,4 | 8,2 |
| 71 | 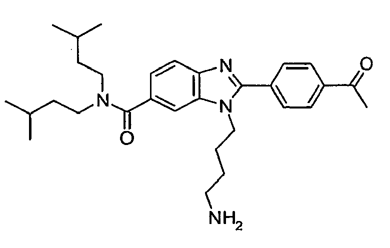 | 491,4 | 8,6 |
| 72 | 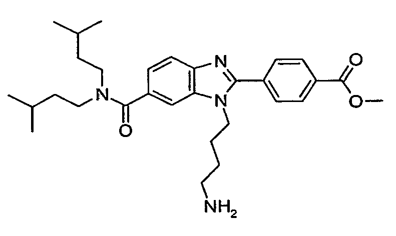 | 507,4 | 8,7 |
| 73 | 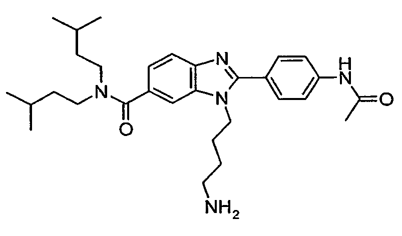 | 506,5 | 8,1 |
| 74 | 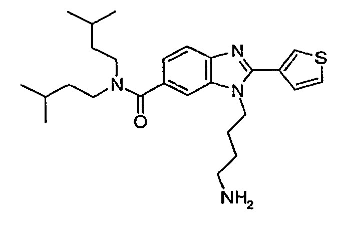 | 455,4 | 8,2 |
| 75 | 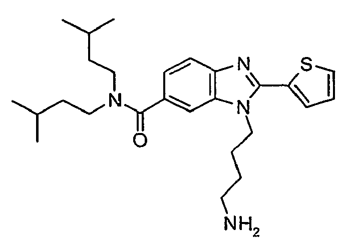 | 455,4 | 8,6 |
| 76 | 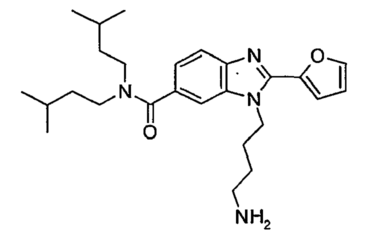 | 439,4 | 8,4 |
| 77 | 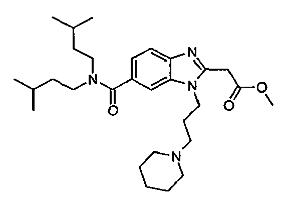 | 499,4 | 8,9 |
| 78 | 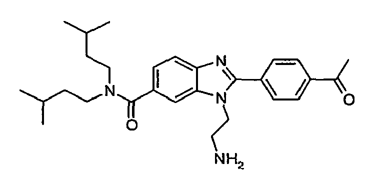 | 463,4 | 8,6 |
| 79 | 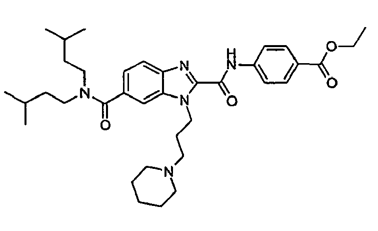 | 618,5 | 10,6 |
| 80 | 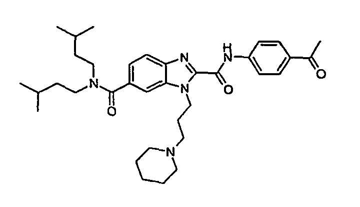 | 588,4 | 9,7 |
| 81 | 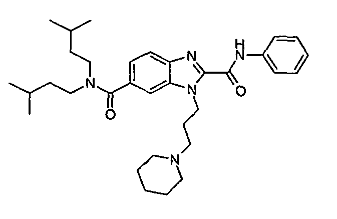 | 546,4 | 9,9 |
| 82 | 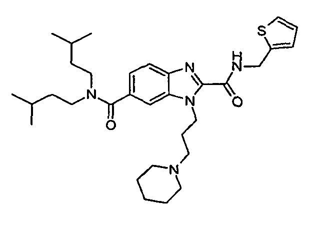 | 566,4 | 9,7 |
| 83 | 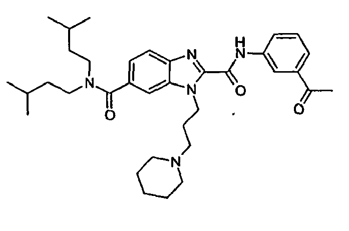 | 588,4 | 10,0 |
| 84 | 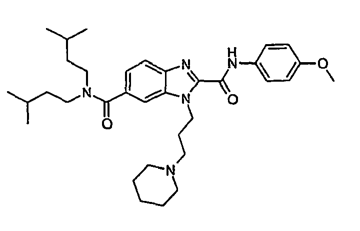 | 576,4 | 10,1 |
| 85 | 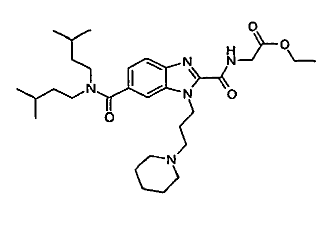 | 556,4 | 9,5 |
| 86 | 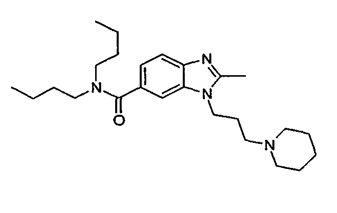 | 413,4 | 7,8 |
| 87 | 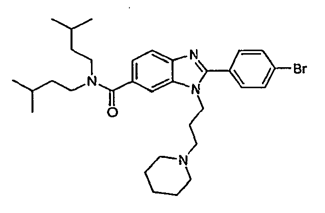 | 581,2 | 9,3 |
| 88 | 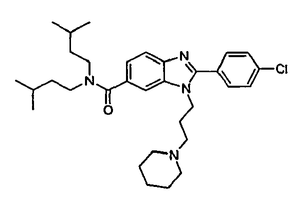 | 537,3 | 9,2 |
| 89 | 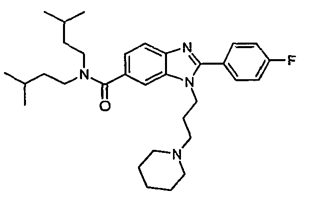 | 521,3 | 8,9 |
| 90 | 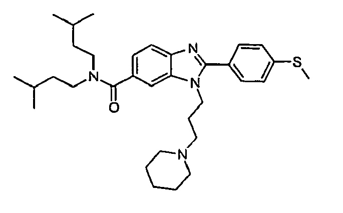 | 549,3 | 8,9 |
| 91 | 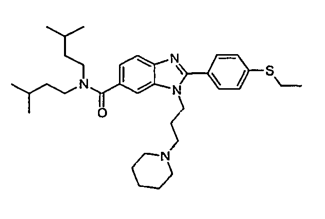 | 563,3 | 9,1 |
| 92 | 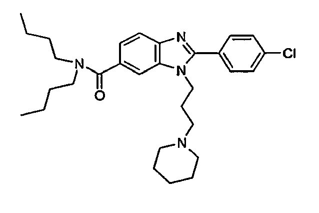 | 509,3 | 8,7 |
| 93 | 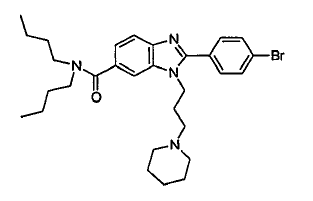 | 555,2 | 8,8 |
| 94 | 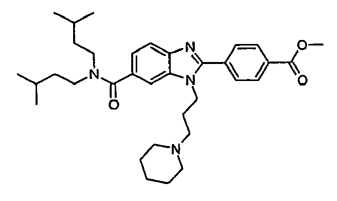 | 561,3 | 9,2 |
| 95 | 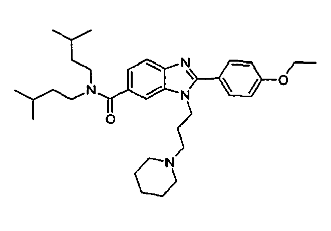 | 547,3 | 8,7 |
| 96 | 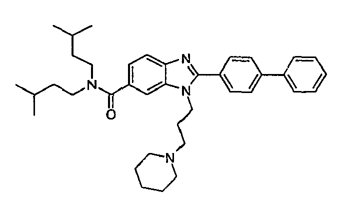 | 579,3 | 9,5 |
| 97 | 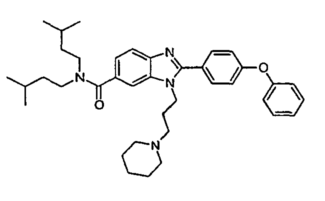 | 595,3 | 9,4 |
| 98 | 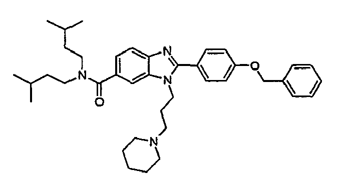 | 609,3 | 9,3 |
| 99 | 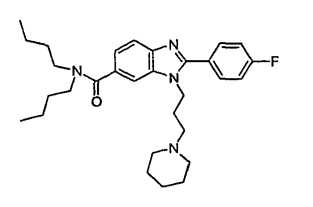 | 493,3 | 8,4 |
| 100 | 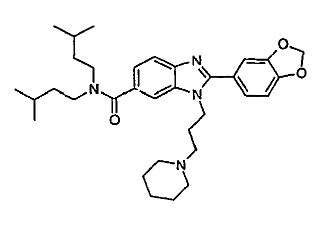 | 547,3 | 8,7 |
| 101 | 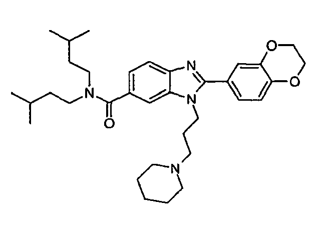 | 561,3 | 8,6 |
| 102 | 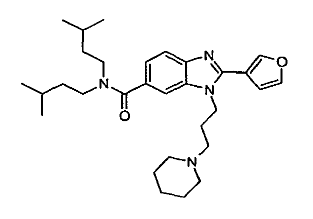 | 493,3 | 8,6 |
| 103 | 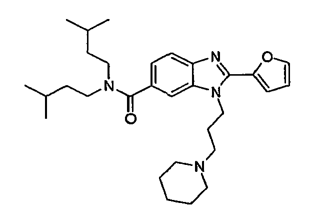 | 493,4 | 8,9 |
| 104 | 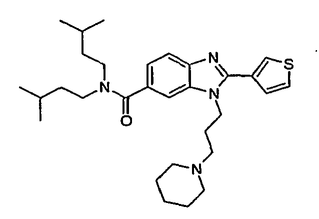 | 509,3 | 8,6 |
| 105 | 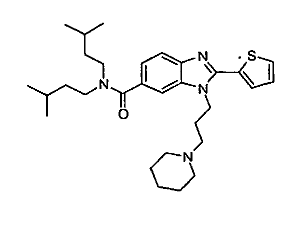 | 509,3 | 8,6 |
| 106 | 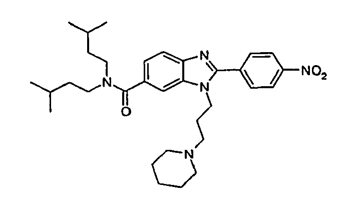 | 548,3 | 9,2 |
| 107 | 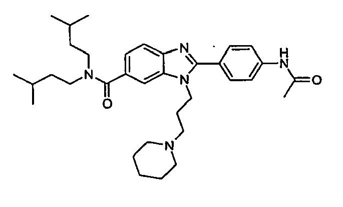 | 560,4 | 8,3 |
| 108 | 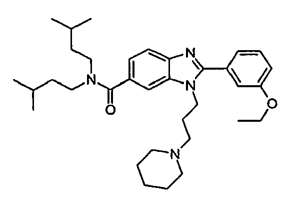 | 547,4 | 8,9 |
| 109 | 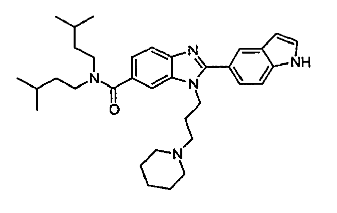 | 542,4 | 8,3 |
| 110 | 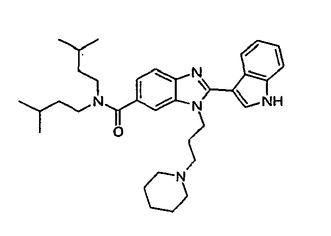 | 542,3 | 8,3 |
| 111 | 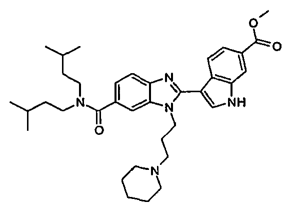 | 600,3 | 8,5 |
| 112 | 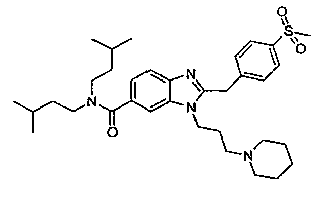 | 595,4 | 8,5 |
| 113 | 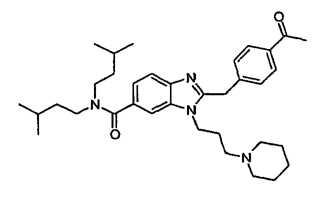 | 559,4 | 8,5 |
| 114 | 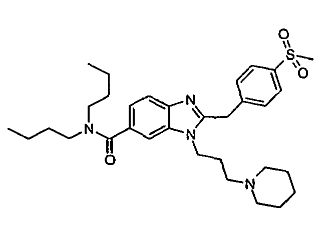 | 567,3 | 8,1 |
| 115 | 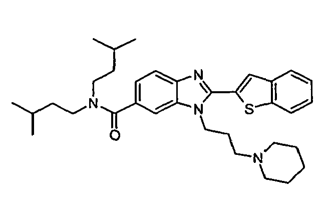 | 559,3 | 9,5 |
| 116 | 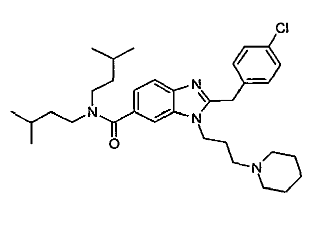 | 551,4 | 8,8 |
| 117 | 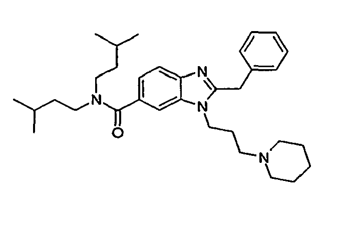 | 517,4 | 8,4 |
| 118 | 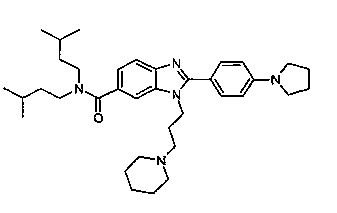 | 572,4 | 8,3 |
| 119 | 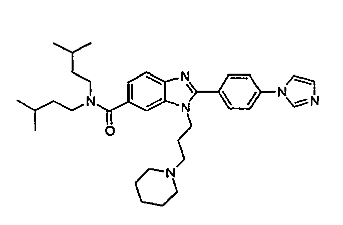 | 569,4 | 8,0 |
| 120 | 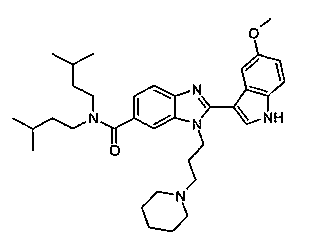 | 572,4 | 8,2 |
| 121 | 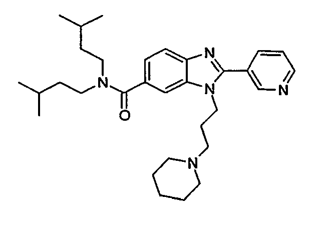 | 504,4 | 8,4 |
| 122 | 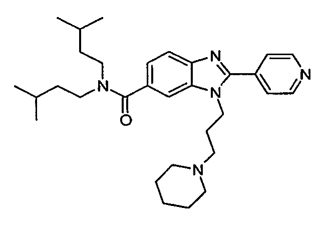 | 504,4 | 8,4 |
| 123 | 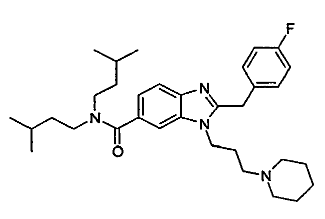 | 535,4 | 8,6 |
| 124 | 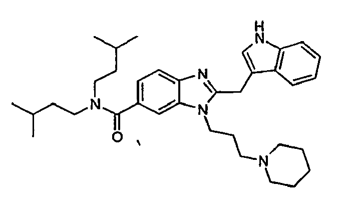 | 556,3 | 8,4 |
| 125 | 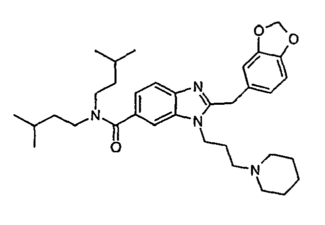 | 561,4 | 8,4 |
| 126 | 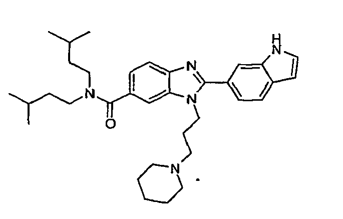 | 542,4 | 8,3 |
| 127 | 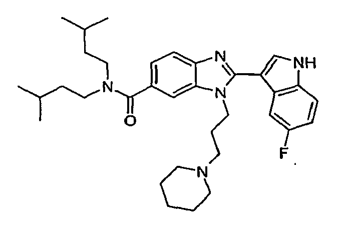 | 560,4 | 8,4 |
| 128 | 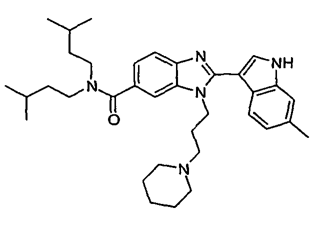 | 556,4 | 8,3 |
| 129 | 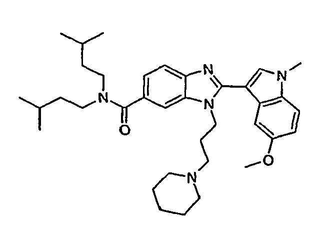 | 586,4 | 8,3 |
| 130 | 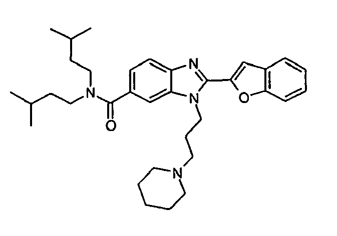 | 543,4 | 9,4 |
| 131 | 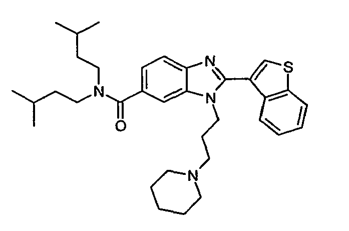 | 559,3 | 9,1 |
| 132 | 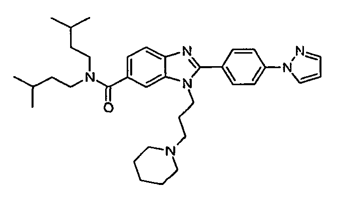 | 569,4 | 8,7 |
| 133 | 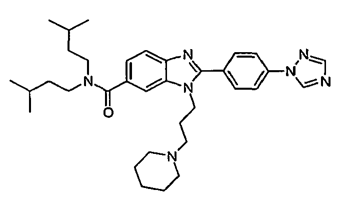 | 570,4 | 8,5 |
| 134 | 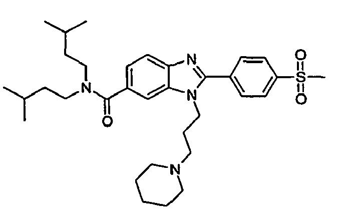 | 581,4 | 8,7 |
| 135 | 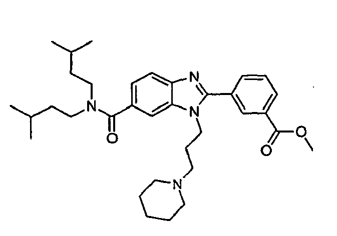 | 561,4 | 8,8 |
| 136 | 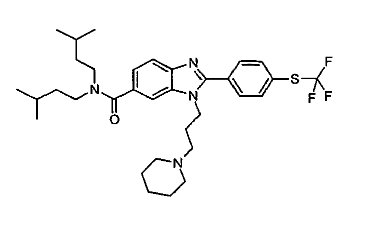 | 603,4 | 9,5 |
| 137 | 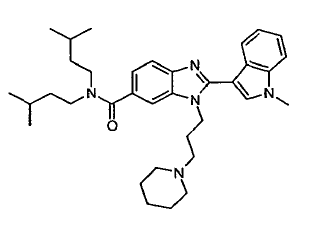 | 556,5 | 8,3 |
| 138 | 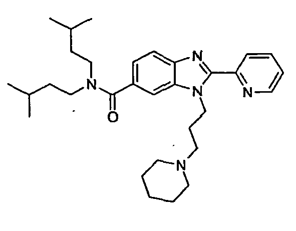 | 504,4 | 8,9 |
| 139 | 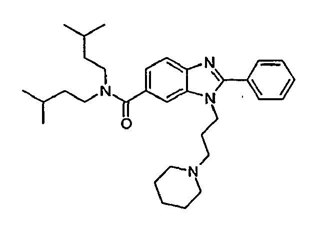 | 503,4 | 8,5 |
| 140 | 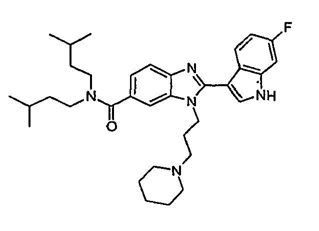 | 560,4 | 8,4 |
| 141 | 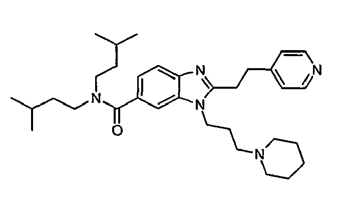 | 532,5 | 7,8 |
| 142 | 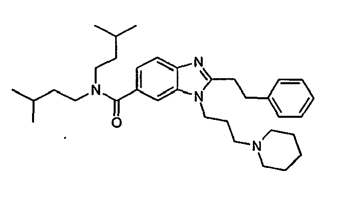 | 531,5 | 8,4 |
| 143 | 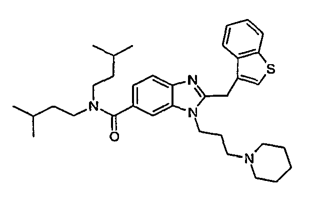 | 573,4 | 8,9 |
| 144 | 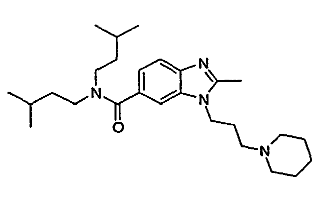 | 441,4 | 7,8 |
| 145 | 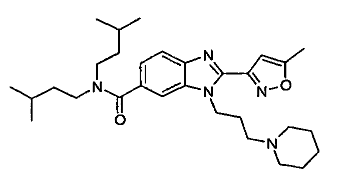 | 508,3 | 9,2 |
| 146 | 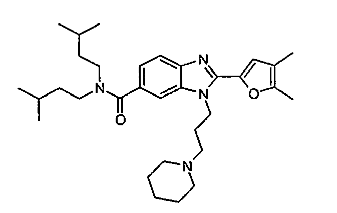 | 521,3 | 8,9 |
| 147 | 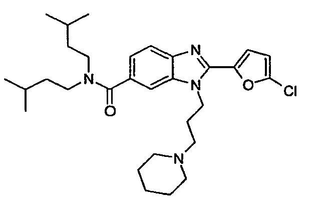 | 527,3 | 9,2 |
| 148 | 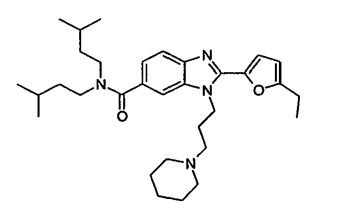 | 521,3 | 9,0 |
| 149 | 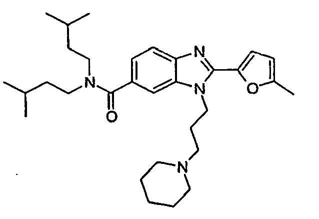 | 507,3 | 8,8 |
| 150 | 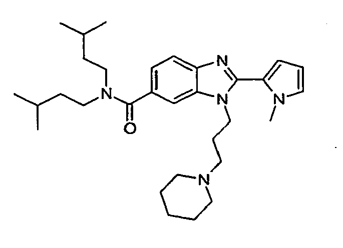 | 506,3 | 8,8 |
| 151 | 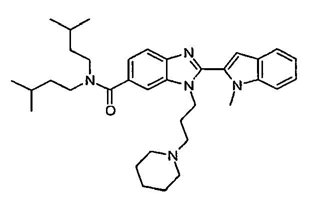 | 556,3 | 9,6 |
| 152 | 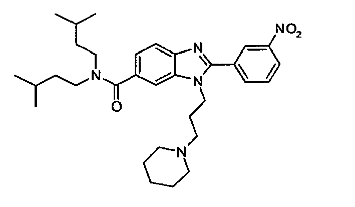 | 548,3 | 9,2 |
| 153 | 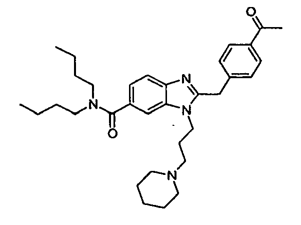 | 531,3 | 8,3 |
| 154 | 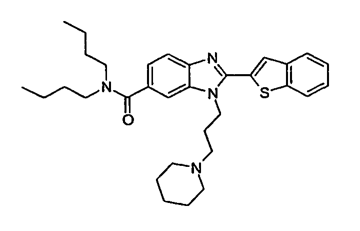 | 531,3 | 9,3 |
| 155 | 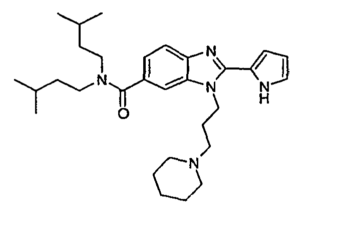 | 492,3 | 8,6 |
| 156 | 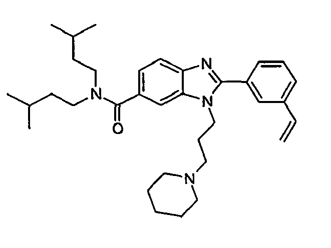 | 529,4 | 9,0 |
| 157 | 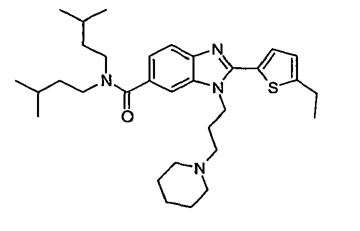 | 537,3 | 9,3 |
| 158 | 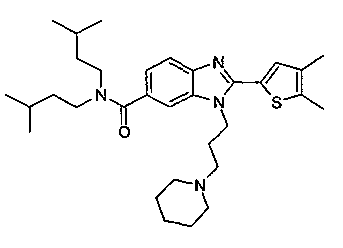 | 537,3 | 9,2 |
| 159 | 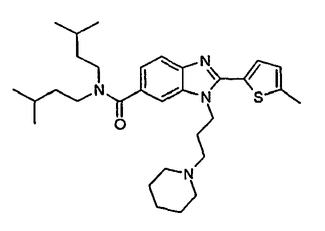 | 523,3 | 9,1 |
| 160 | 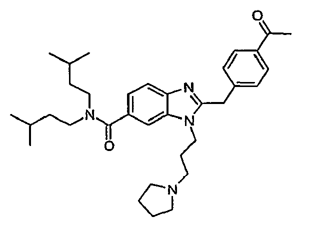 | 545,3 | 8,6 |
| 161 | 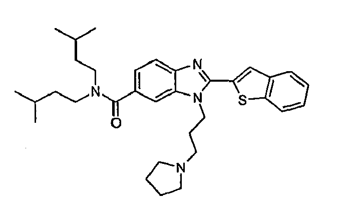 | 545,3 | 9,6 |
| 162 | 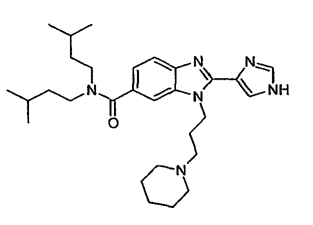 | 493,3 | 8,2 |
| 163 | 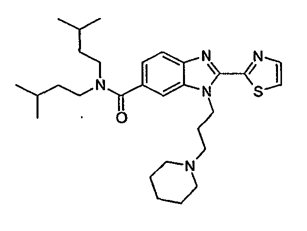 | 510,3 | 9,3 |
| 164 | 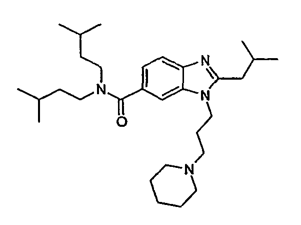 | 483,4 | 8,2 |
| 165 | 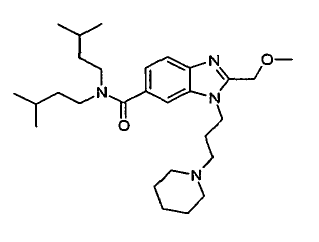 | 471,3 | 8,4 |
| 166 | 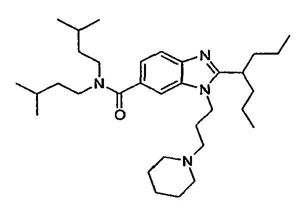 | 525,4 | 8,6 |
| 167 | 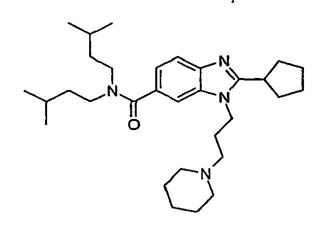 | 495,4 | 8,2 |
| 168 | 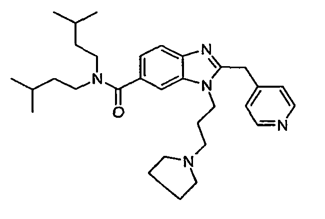 | 504,3 | 8,0 |
| 169 | 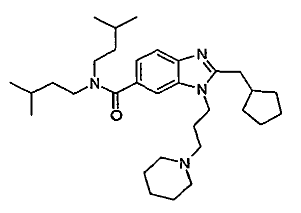 | 509,4 | 8,3 |
| 170 | 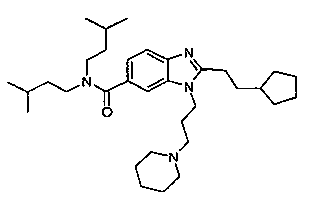 | 523,4 | 8,5 |
| 171 | 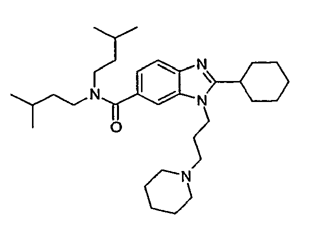 | 509,4 | 8,3 |
| 172 | 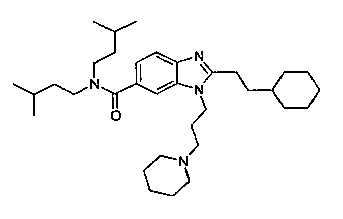 | 537,4 | 8,6 |
| 173 | 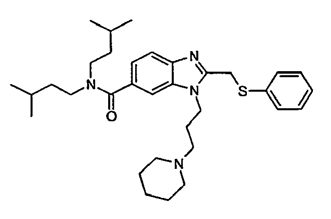 | 549,3 | 9,0 |
| 174 | 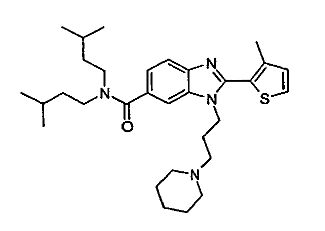 | 523,3 | 8,9 |
| 175 | 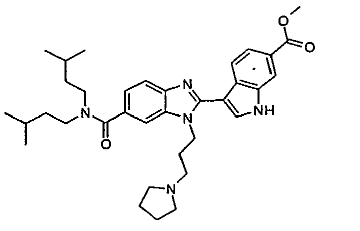 | 586,3 | 8,4 |
| 176 | 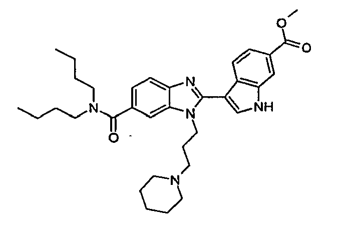 | 572,3 | 8,1 |
| 177 | 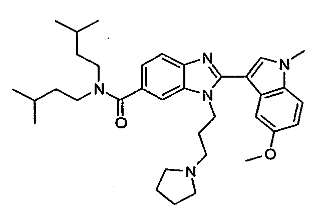 | 572,3 | 8,3 |
| 178 | 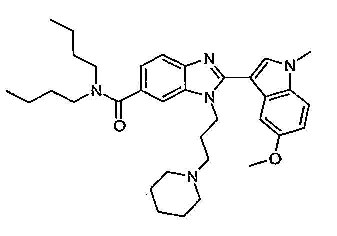 | 558, 3 | 8,1 |
| 179 | 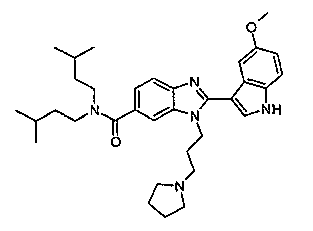 | 558,3 | 8,2 |
| 180 | 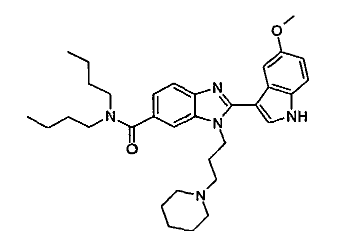 | 544,3 | 8,0 |
| 181 | 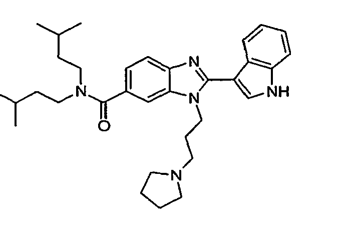 | 528,3 | 8,2 |
| 182 | 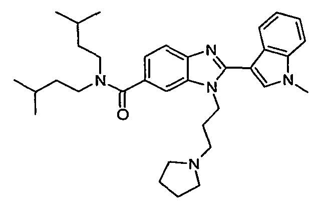 | 542,3 | 8,4 |
| 183 | 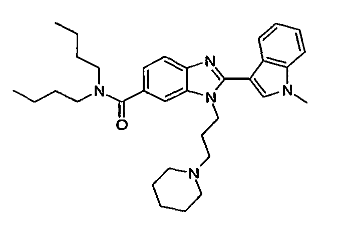 | 528,3 | 8,2 |
| 184 | 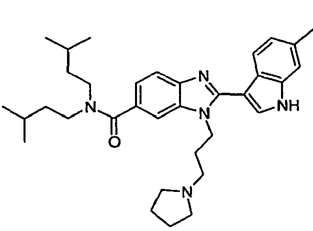 | 542,3 | 8,4 |
| 185 | 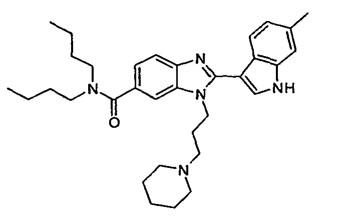 | 528,3 | 8,2 |
| 186 | 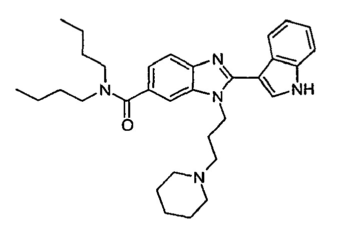 | 514,3 | 8,1 |
| 187 | 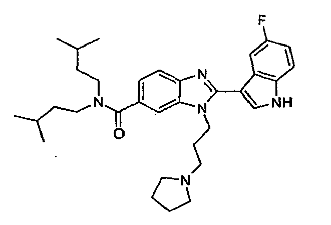 | 546,3 | 8,5 |
| 188 | 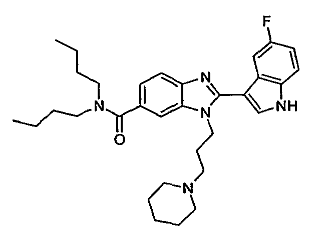 | 532,3 | 8,2 |
| 189 | 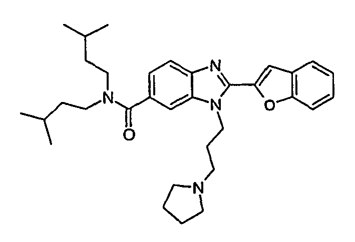 | 529,3 | 9,5 |
| 190 | 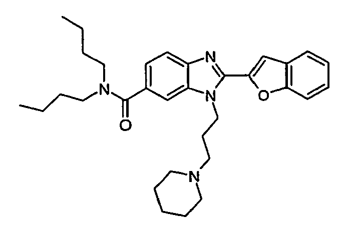 | 515,3 | 9,1 |
| 191 | 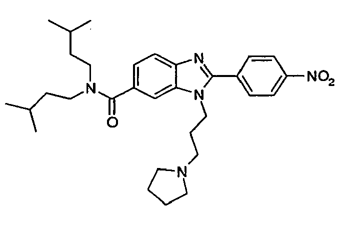 | 534,3 | 9,2 |
| 192 | 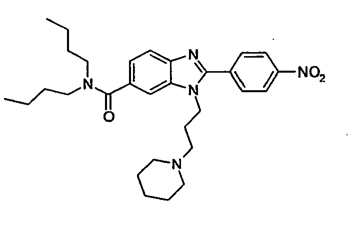 | 520,3 | 8,8 |
| 193 | 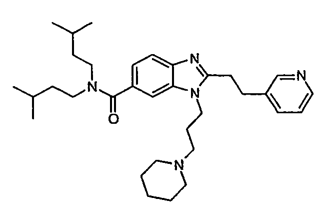 | 532,3 | 8,0 |
| 194 | 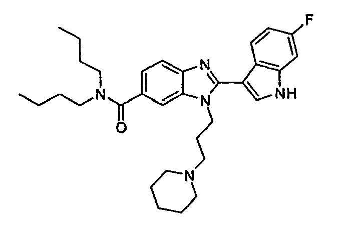 | 532,3 | 8,2 |
| 195 | 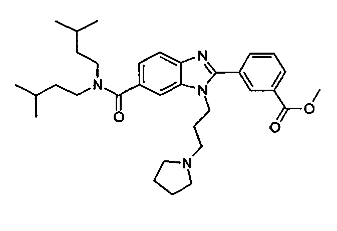 | 547,3 | 8,9 |
| 196 | 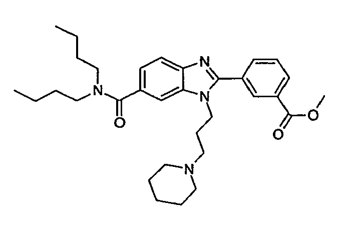 | 533,3 | 8,6 |
| 197 | 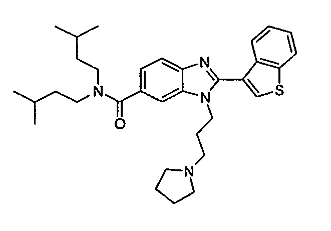 | 545,2 | 9,2 |
| 198 | 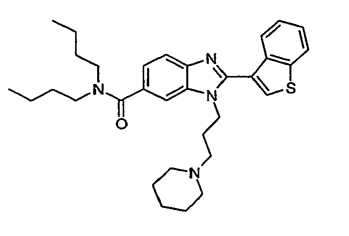 | 531,2 | 8,8 |
| 199 | 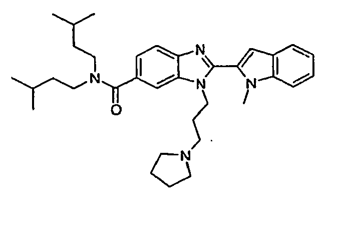 | 542,3 | 9,5 |
| 200 | 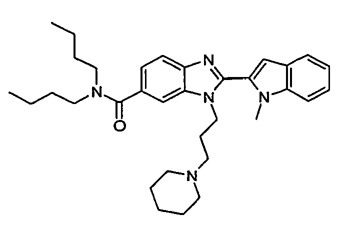 | 528,3 | 9,2 |
| 201 | 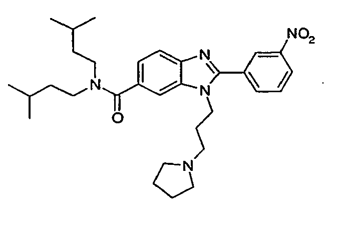 | 534,2 | 9,1 |
| 202 | 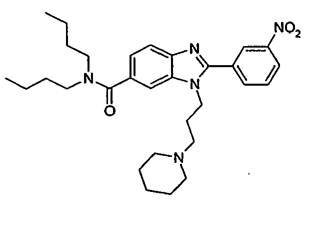 | 520,2 | 8,8 |
| 203 | 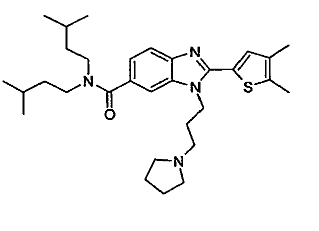 | 523,3 | 9,1 |
| 204 | 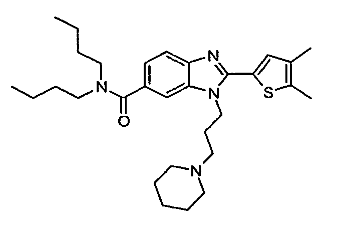 | 509,3 | 8,7 |
| 205 | 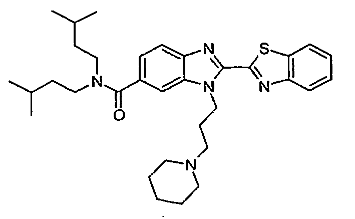 | 560,2 | 10,1 |
| 206 | 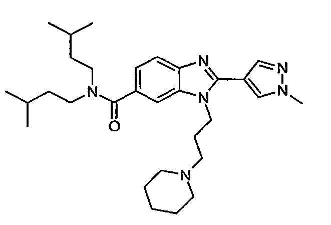 | 507,3 | 8,3 |
| 207 | 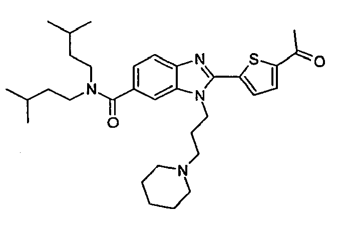 | 551,3 | 9,2 |
| 208 | 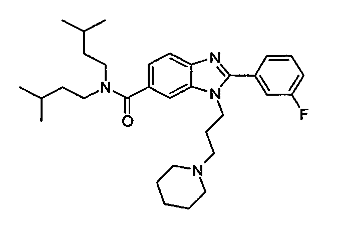 | 521,3 | 9,1 |
| 209 | 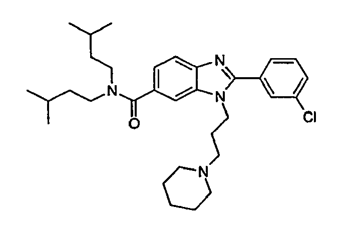 | 537,3 | 9,3 |
| 210 | 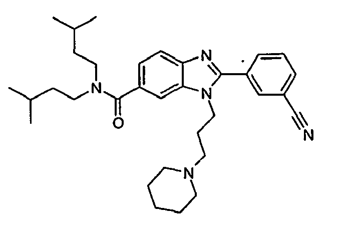 | 528,3 | 9,1 |
| 211 | 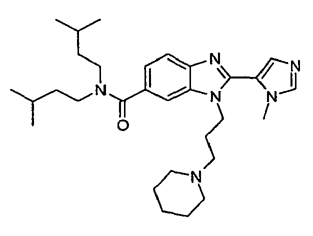 | 507,5 | 8,1 |
| 212 | 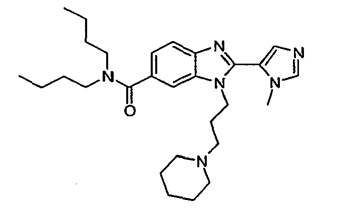 | 479,4 | 7,8 |
| 213 | 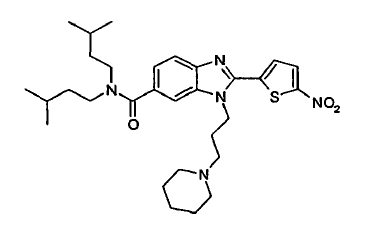 | 554,4 | 9,7 |
| 214 | 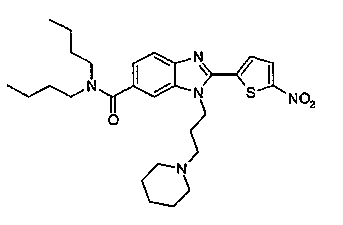 | 526,4 | 9,2 |
| 215 | 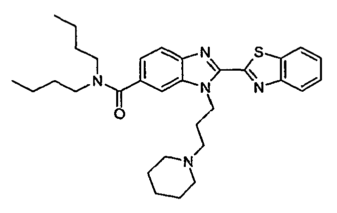 | 532,4 | 9,7 |
| 216 | 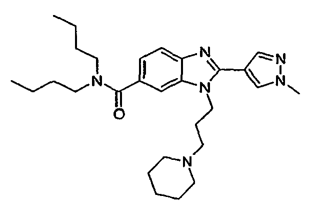 | 479,4 | 7,9 |
| 217 | 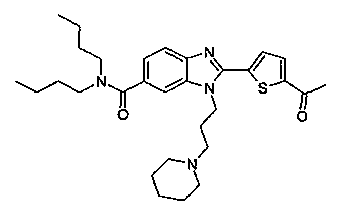 | 523,4 | 8,8 |
| 218 | 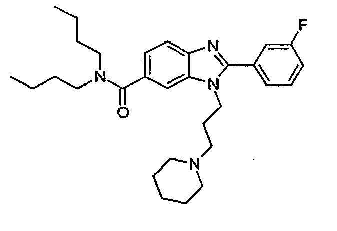 | 493,4 | 8,6 |
| 219 | 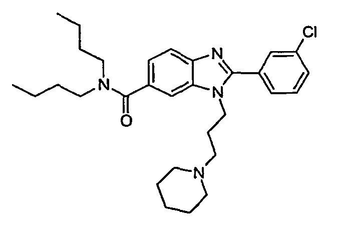 | 509,4 | 8,8 |
| 220 | 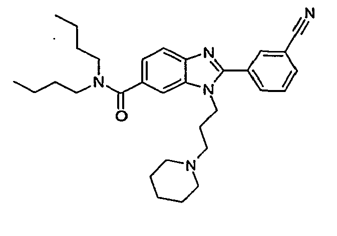 | 500,5 | 8,7 |
| 221 | 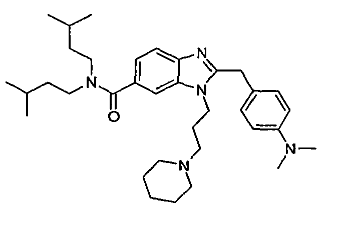 | 560,5 | 8,4 |
| 222 | 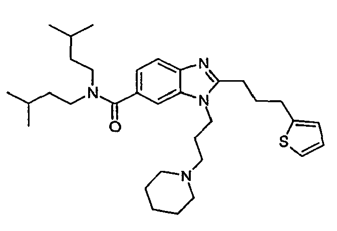 | 551,5 | 8,7 |
| 223 | 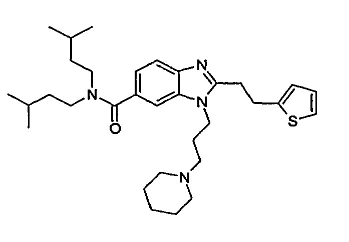 | 537,5 | 8,7 |
| 224 | 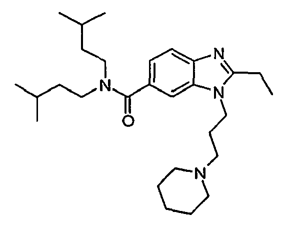 | 455,5 | 8,0 |
| 225 | 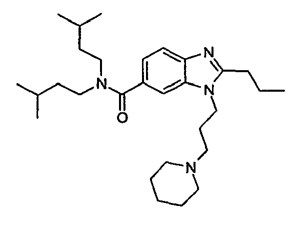 | 469,5 | 8,1 |
| 226 | 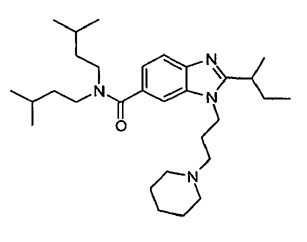 | 483,5 | 8,2 |
| 227 | 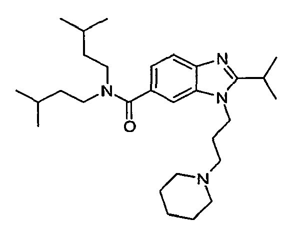 | 469,5 | 8,1 |
| 228 | 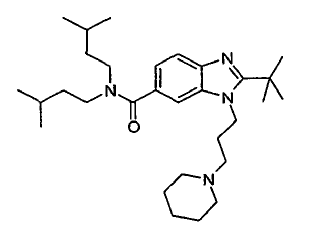 | 483,5 | 8,2 |
| 229 | 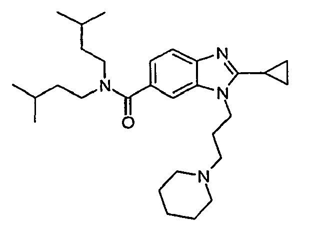 | 467,5 | 8,1 |
| 230 | 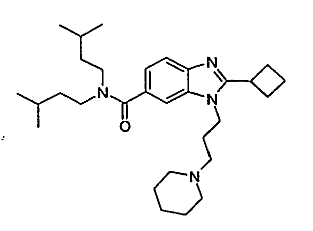 | 481,4 | 8,2 |
| 231 | 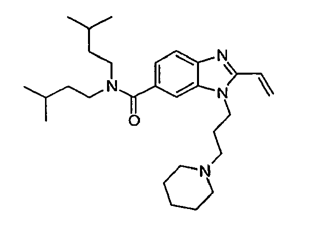 | 453,4 | 8,3 |
| 232 | 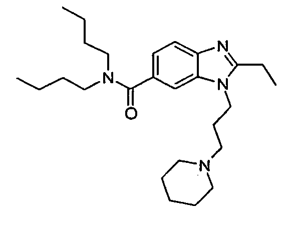 | 427,4 | 7,8 |
| 233 | 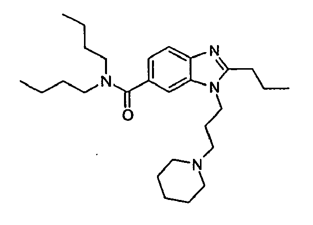 | 441,4 | 7,8 |
| 234 | 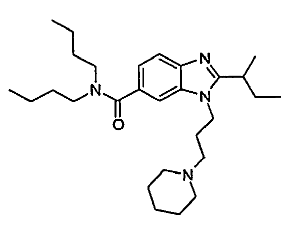 | 455,4 | 7,9 |
| 235 | 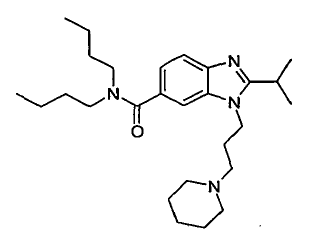 | 441,4 | 7,8 |
| 236 | 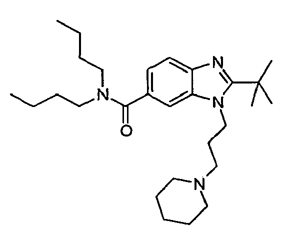 | 455,4 | 7,9 |
| 237 | 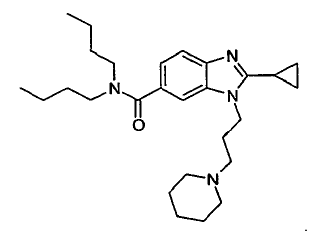 | 439,4 | 7,8 |
| 238 | 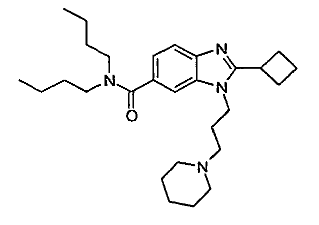 | 453,4 | 7,9 |
| 239 | 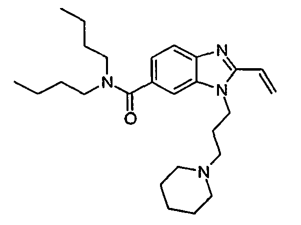 | 425,3 | 8,0 |
| 240 | 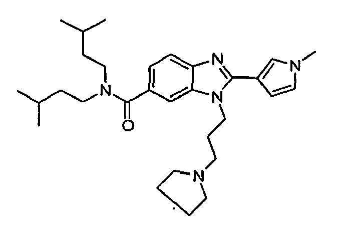 | 492,4 | 8,1 |
| 241 | 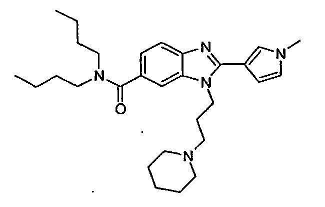 | 478,4 | 7,8 |
| 242 | 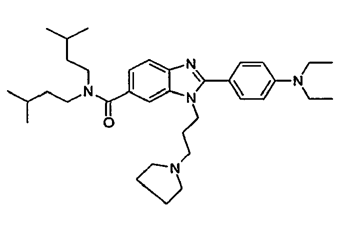 | 560,5 | 8,4 |
| 243 | 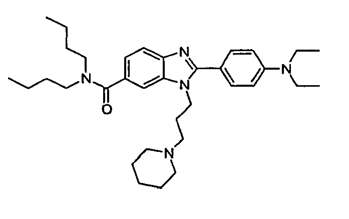 | 546,4 | 8,2 |
| 244 | 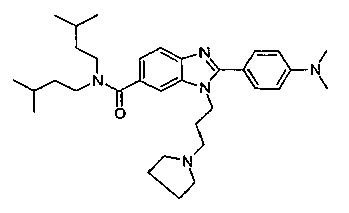 | 532,4 | 8,2 |
| 245 | 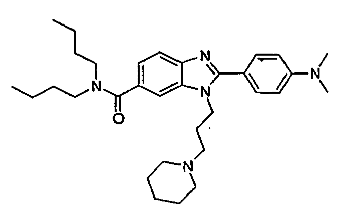 | 518,4 | 8,0 |
| 246 | 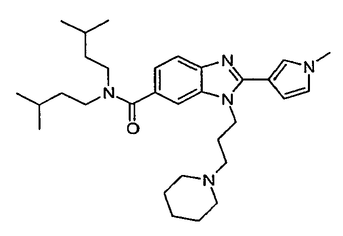 | 506,4 | 8,2 |
| 247 | 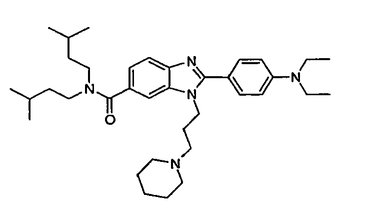 | 574,4 | 8,5 |
| 248 | 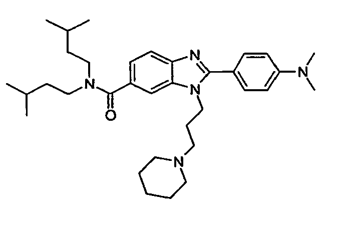 | 546,4 | 8,3 |
| 249 | 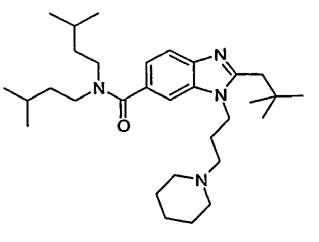 | 497,5 | 8,4 |
| 250 | 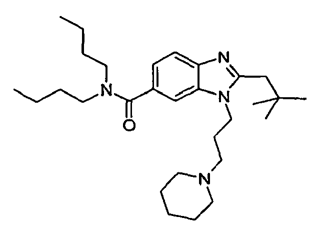 | 469,5 | 8,1 |

### Etude pharmacologique

L'affinité des composés de la présente invention pour les différents sous-types de récepteurs des mélanocortines a été mesurée selon les procédures analogues à celles décrites ci-après pour les récepteurs MC4.

### Etude de l'affinité des composés pour les récepteurs MC4 des mélanocortines :

L'affinité des composés de l'invention pour les récepteurs MC4 est déterminée par la mesure de l'inhibition de la liaison de la [¹²⁵I]-[Nle⁴, D-Phe⁷]-α-MSH à des préparations membranaires de cellules CHO-K1 transfectées.

Les cellules CHO-K1 exprimant de façon stable les récepteurs MC4 humains sont cultivées dans un milieu RPMI 1640 contenant 10 % de sérum foetal de veau, 2 mM de glutamine, 100 U/ml de pénicilline, 0,1 mg/ml de streptomycine et 0,5 mg/ml de G418. Les cellules sont collectées avec 0,5 mM d'EDTA et centrifugées à 500 g pendant 5 min à 4° C. Le culot est re-suspendu dans un milieu salin avec tampon phosphate (PBS) et centrifugé à 500 g pendant 5 min à 4° C. Le culot est re-suspendu dans un milieu tampon Tris 50 mM à pH 7,4 et centrifugé à 500 g pendant 5 min à 4° C. Les cellules sont lysées par sonication et centrifugées à 39 000 g pendant 10 min à 4° C. Le culot est re-suspendu dans le milieu tampon Tris 50 mM à pH 7,4 et centrifugé à 50 000 g pendant 10 min à 4° C. Les membranes obtenues dans ce dernier culot sont stockées à -80° C.

La mesure de l'inhibition compétitive de la liaison de la [¹²⁵I]-[Nle⁴, D-Phe⁷]-α-MSH (Amersham Biosciences) sur les récepteurs MC4 est effectuée en duplicats à l'aide de plaques en polypropylène de 96 puits. Les membranes cellulaires (50 µg de protéines / puits) sont incubées avec la [¹²⁵I]-[Nle⁴, D-Phe⁷]-α-MSH (0,5 nM) pendant 90 min à 37° C dans un milieu tampon Tris-HCl 50 mM, pH 7,4, comprenant 0,2 % d'albumine bovine de sérum (BSA), 5 mM de MgCl₂, et 0,1 mg/ml de bacitracine.

La [¹²⁵I]-[Nle⁴, D-Phe⁷]-α-MSH liée est séparée de la [¹²⁵I]-[Nle⁴, D-Phe⁷]-α-MSH libre par filtration à travers des plaques de filtres en fibre de verre GF/C (Unifilter, PerkinElmer) pré-imprégné avec 0,1 % de polyéthylènimine (P.E.I.), en utilisant un Filtermate 196 (PerkinElmer). Les filtres sont lavés avec du tampon Tris-HCl 50 mM, pH 7,4 à 0-4° C et la radioactivité présente est déterminée à l'aide d'un compteur (Top Count, PerkinElmer).

La liaison spécifique est obtenue en soustrayant la liaison non spécifique (déterminée en présence de 0,1 µM de Nle⁴, D-Phe⁷-α-MSH) de la liaison totale. Les données sont analysées par régression non-linéaire assistée par ordinateur (MDL) et les valeurs des constantes d'inhibition (Ki) sont déterminées.

L'activité agoniste ou antagoniste des récepteurs MC4 des composés de la présente invention a été déterminée en mesurant la production d'AMP cyclique par les cellules CHO-K1 transfectées par le récepteur MC4.

### Mesure de la production d'AMP cyclique intracellulaire via les récepteurs MC4 :

Les cellules CHO-K1 exprimant les récepteurs MC4 des mélanocortines sont cultivées dans des plaques à 384 puits dans un milieu RPMI 1640 avec 10 % de sérum foetal de veau et 0,5 mg/ml de G418. Les cellules sont lavées 2 fois avec 50 µl de milieu RPMI comprenant 0,2 % BSA et 0,5 mM de 3-isobutyl-1-méthylxanthine (IBMX).

Pour mesurer l'effet agoniste d'un composé, les cellules sont incubées pendant 5 min à 37° C en présence de 0,5 mM d'IBMX, puis la stimulation de la production d'AMP cyclique est obtenue en ajoutant le composé à des concentrations comprises entre 1 pM et 10 µM pendant 20 min à 37° C. L'effet antagoniste d'un composé est mesuré en inhibant la stimulation de la production d'AMP cyclique induite par la Nle⁴, D-Phe⁷-α-MSH, à des concentrations comprises entre 1 pM et 10 µM, en présence du composé à tester, à des concentrations comprises entre 1 nM et 10 µM pendant 20 min à 37° C.

Le milieu réactionnel est éliminé et 80 µl de tampon de lyse sont ajoutés. Le taux d'AMP cyclique intracellulaire est mesuré par un test de compétition avec de l'AMP cyclique fluorescent (CatchPoint, Molecular Devices).

Les tests effectués selon les protocoles décrits ci-dessus ont permis de montrer que les produits selon la présente invention ont une bonne affinité sur les récepteurs MC4, la constante d'inhibition Ki sur ces récepteurs étant inférieure au micromolaire pour la majorité des composés exemplifiés.

## Revendications

1. Composé de formule générale (**I**) 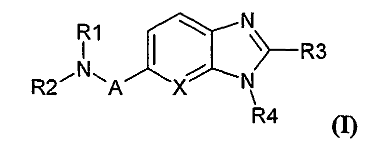 sous forme racémique, d'énantiomère ou toutes combinaisons de ces formes et dans laquelle :
A représente -CH₂-, -C(O)-, -C(O)-C(Rₐ)(R_{b})- ;
X représente le radical -CH- ou l'atome d'azote ;
Rₐ et R_{b} représentent, indépendamment, l'atome d'hydrogène ou un radical (C₁-C₆)alkyle ;
R₁ représente l'atome d'hydrogène ou un radical (C₁-C₈)alkyle ;
R₂ représente un radical (C₁-C₈)alkyle ;
ou bien R₁ et R₂ forment ensemble, avec l'atome d'azote auquel ils sont rattachés, un hétérobicycloalkyle ou un hétérocycloalkyle éventuellement substitué par un ou
plusieurs substituants (C₁-C₆)alkyle identiques ou différents ;
R₃ représente -(CH₂)ₚ-Z₃, -C(O)-Z'₃, -CH(OH)-Z'₃ ou -C(O)-NH-Z"₃ ;
Z₃ représente un radical (C₁-C₆)alkyle, (C₂-C₆)alkényle, (C₁-C₆)alkoxy, (C₁-C₆)alkyl-carbonyle, (C₁-C₆)alkoxy-carbonyle, (C₁-C₆)alkyl-N(R_{N})-carbonyle, (C₃-C₇)cycloalkyle, hétérocycloalkyle, aryle, aryl-thio ou hétéroaryle, Z₃ étant lié au radical -(CH₂)ₚ- par un atome de carbone,
les radicaux (C₃-C₇) cycloalkyle et hétérocycloalkyle étant éventuellement substitués par un ou plusieurs radicaux identiques ou différents choisis parmi (C₁-C₆)alkyle et oxy ;
le radical hétéroaryle étant éventuellement substitué par un ou plusieurs substituants identiques ou différents choisis parmi : halo, nitro ou -(CH₂)_{p'}-V₃₀-Y₃ ;
le radical aryle étant éventuellement substitué par un ou plusieurs substituants identiques ou différents choisis parmi : halo, nitro, cyano, (C₂-C₆)alkényle, hétérocycloalkyle, aryle, aryloxy, aralkyl-oxy, hétéroaryle et -(CH₂)_{p'}-V₃₁-Y₃ ;
V₃₀ représente -O-, -C(O)-, -C(O)-O- ou une liaison covalente ;
V₃₁ représente -O-, -S-, -SO₂- -C(O)-, -C(O)-O-, -N(R_{N})-, -NH-C(O)-, -C(O)-NR'₃-, -NH-C(O)-NR'₃- ou une liaison covalente ;
Y₃ représente l'atome d'hydrogène ou un radical (C₁-C₆)alkyle éventuellement substitué par un ou plusieurs radicaux halo identiques ou différents ;
R_{N} représente un atome d'hydrogène ou un radical (C₁-C₆)alkyle ;
ou bien Z₃ représente un radical de formule 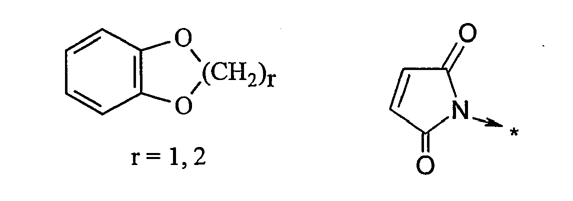 Z'₃ représente un radical aryle éventuellement substitué par un ou plusieurs substituants identiques ou différents choisis parmi : halo, nitro et -(CH₂)ₚ"-V'₃-Y'₃ ;
V'₃ représente -O-, -C(O)-, -C(O)-O-, -NH-C(O)-, -C(O)-NR'₃- ou une liaison covalente ;
Y'₃ représente l'atome d'hydrogène ou un radical (C₁-C₆)alkyle éventuellement substitué par un ou plusieurs radicaux halo identiques ou différents ;
R'₃ représente l'atome d'hydrogène, un radical (C₁-C₆)alkyle ou (C₁-C₆)alkoxy ;
Z"₃ représente l'atome d'hydrogène ou un radical -(CH₂)_{q} A"₃ ;
A"₃ représente un radical (C₁-C₆)alkyle, aryle ou hétéroaryle ;
les radicaux alkyle et aryle étant éventuellement substitués par un ou plusieurs substituants identiques ou différents choisis parmi halo et -V"₃-Y"₃ ;
V"₃ représente -O-, -C(O)-, -C(O)-O-, -C(O)-NH- ou une liaison covalente ; Y"₃ représente l'atome d'hydrogène ou un radical (C₁-C₆)alkyle éventuellement substitué par un ou plusieurs radicaux halo identiques ou différents ;
p représente un entier de 0 à 6 ; p' et p" représentent, indépendamment, un entier de 0 à 4 ; q représente un entier de 0 à 2 ;
R₄ représente un radical de formule-(CH₂)ₛ-R'₄ ;
R'₄ représente un hétérocycloalkyle contenant au moins un atome d'azote et éventuellement substitué par (C₁-C₆)alkyle ; un hétéroaryle contenant au moins un atome d'azote et éventuellement substitué par (C₁-C₆)alkyle ; ou un radical de formule -NW₄W'₄ ;
W₄ représente l'atome d'hydrogène ou (C₁-C₈)alkyle ;
W'₄ représente un radical de formule -(CH₂)_{s'}-Z₄ dans laquelle Z₄ représente l'atome d'hydrogène, un radical (C₁-C₈)alkyle ou (C₃-C₇)cycloalkyle ;
s et s' représentent, indépendamment, un entier de 0 à 6 ;
ou un sel pharmaceutiquement acceptable de ce dernier.

2. Composé selon la revendication 1, **caractérisé en ce que** X représente le radical -CH- ; ou un sel pharmaceutiquement acceptable de ce dernier.

3. Composé selon l'une des revendications précédentes, **caractérisé en ce que** R₁ représente l'atome d'hydrogène ou un radical (C₁-C₈)alkyle, et R₂ représente un radical (C₁-C₈)alkyle ; ou un sel pharmaceutiquement acceptable de ce dernier.

4. Composé selon l'une des revendications 1 à 3, **caractérisé en ce que** R₁ représente un radical (C₁-C₆)alkyle ; R₂ représente un radical (C₁-C₆)alkyle; ou un sel pharmaceutiquement acceptable de ce dernier.

5. Composé selon l'une des revendications 1 à 4, **caractérisé en ce que** A représente -CH₂- ; ou un sel pharmaceutiquement acceptable de ce dernier.

6. Composé selon l'une des revendications 1 à 4, **caractérisé en ce que** A représente -C(O)-C(Rₐ)(R_{b})- et Rₐ et R_{b} représentent, indépendamment, un radical méthyle ; ou un sel pharmaceutiquement acceptable de ce dernier.

7. Composé selon l'une des revendications 1 à 4, **caractérisé en ce que** A représente -C(O)- ; ou un sel pharmaceutiquement acceptable de ce dernier.

8. Composé selon l'une des revendications précédentes, **caractérisé en ce que** R₄ représente un radical de formule-(CH₂)ₛ-R'₄ ;
R'₄ représente un hétérocycloalkyle contenant au moins un atome d'azote et éventuellement substitué par (C₁-C₆)alkyle ; ou un radical de formule -NW₄W'₄ ;
W₄ représente l'atome d'hydrogène ou (C₁-C₈)alkyle ;
W'₄ représente un radical de formule -(CH₂)_{s'}-Z₄ dans laquelle Z₄ représente l'atome d'hydrogène ou un radical (C₁-C₈)alkyle ;
s et s' représentent, indépendamment, un entier de 1 à 6 ; ou un sel pharmaceutiquement acceptable de ce dernier.

9. Composé selon l'une des revendications précédentes, **caractérisé en ce que** R₄ représente un radical de formule-(CH₂)ₛ-R'₄ ;
R'₄ représente un radical de formule -NW₄W'₄ ;
W₄ représente un radical (C₁-C₈)alkyle ;
W'₄ représente un radical de formule -(CH₂)_{s'}-Z₄ dans laquelle Z₄ représente l'atome d'hydrogène ou un radical (C₁-C₈)alkyle ;
s et s' représentent, indépendamment, un entier de 2 à 6 ; ou un sel pharmaceutiquement acceptable de ce dernier.

10. Composé selon l'une des revendications 1 à 8, **caractérisé en ce que** R₄ représente un radical de formule-(CH₂)ₛ-R'₄ ;
R'₄ représente un hétérocycloalkyle contenant au moins un atome d'azote et éventuellement substitué par (C₁-C₆)alkyle ; et s représente un entier de 2 à 6 ; ou un sel pharmaceutiquement acceptable de ce dernier.

11. Composé selon la revendication 10, **caractérisé en ce que** R'₄ représente le cycle pipéridine ou pyrrolidine ; s représente un entier de 1 à 4 ; ou un sel pharmaceutiquement acceptable de ce dernier.

12. Composé selon l'une des revendications précédentes, **caractérisé en ce que** R₃ représente -(CH₂)ₚ-Z₃ et
Z₃ représente un radical (C₁-C₆)alkyle, (C₂-C₆)alkényle, (C₁-C₆)alkoxy, (C₁-C₆)alkyl-carbonyle, (C₁-C₆)alkoxy-carbonyle, (C₁-C₆)alkyl-N(R_{N})-carbonyle, (C₃-C₇)cycloalkyle, hétérocycloalkyle, aryle, aryl-thio ou hétéroaryle,
les radicaux (C₃-C₇) cycloalkyle et hétérocycloalkyle étant éventuellement substitués par un ou plusieurs radicaux identiques ou différents choisis parmi (C₁-C₆)alkyle et oxy ;
le radical hétéroaryle étant éventuellement substitué par un ou plusieurs substituants identiques ou différents choisis parmi : halo, nitro ou -(CH₂)ₚ'-V₃₀-Y₃;
le radical aryle étant éventuellement substitué par un ou plusieurs substituants identiques ou différents choisis parmi : halo, nitro, (C₂-C₆)alkényle, hétérocycloalkyle, aryle, aryloxy, aralkyl-oxy, hétéroaryle et -(CH₂)_{p'}- V₃₁- Y₃ ;
V₃₀ représente -O-, -C(O)-, -C(O)-O- ou une liaison covalente ;
V₃₁ représente -O-, -S-, -SO₂- -C(O)-, -C(O)-O-, -N(R_{N})-, -NH-C(O)-, -C(O)-NR'₃- ou une liaison covalente ;
Y₃ représente l'atome d'hydrogène ou un radical (C₁-C₆)alkyle éventuellement substitué par un ou plusieurs radicaux halo identiques ou différents ;
R_{N} représente un atome d'hydrogène ou un radical (C₁-C₆)alkyle ;
ou bien Z₃ représente un radical de formule 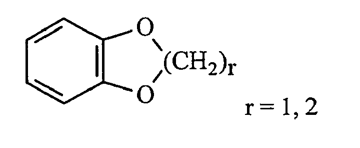 ou un sel pharmaceutiquement acceptable de ce dernier.

13. Composé selon l'une des revendications précédentes, **caractérisé en ce que**
Z₃ représente un radical (C₁-C₆)alkyle, (C₁-C₆)alkyl-carbonyle, (C₁-C₆)alkoxy-carbonyle, (C₁-C₆)alkyl-N(R_{N})-carbonyle, (C₃-C₇)cycloalkyle, aryle ou hétéroaryle, les radicaux aryle et hétéroaryle étant éventuellement substitués ; ou un sel pharmaceutiquement acceptable de ce dernier.

14. Composé selon la revendication 13, **caractérisé en ce que**
le radical hétéroaryle est éventuellement substitué par un ou plusieurs substituants identiques ou différents choisis parmi : halo et -(CH₂)_{p'}-V₃₀-Y₃ ;
le radical aryle est éventuellement substitué par un ou plusieurs substituants identiques ou différents choisi parmi : nitro et -(CH₂)_{p'}-V₃₁ -Y₃ ;
V₃₀ représente -O-, -C(O)-, -C(O)-O- ou une liaison covalente ;
V₃₁ représente -O-, -C(O)-, -C(O)-O- ou -SO₂- ;
Y₃ représente un radical (C₁-C₆)alkyle ;
p et p' représentent, indépendamment, un entier de 0 à 4 ; ou un sel pharmaceutiquement acceptable de ce dernier.

15. Composé selon la revendication 13, **caractérisé en ce que** Z₃ représente un radical (C₁-C₆)alkyle ; ou un sel pharmaceutiquement acceptable de ce dernier.

16. Composé selon la revendication 13, **caractérisé en ce que** Z₃ représente un radical (C₁-C₆)alkyl-carbonyle, (C₁-C₆)alkoxy-carbonyle ou (C₁-C₆)alkyl-N(R_{N})-carbonyle ; ou un sel pharmaceutiquement acceptable de ce dernier.

17. Composé selon la revendication 13, **caractérisé en ce que** Z₃ représente un hétéroaryle éventuellement substitué par un ou plusieurs substituants identiques ou différents choisis parmi : halo et -(CH₂)_{p'}-V₃₀-Y₃ ;
V₃₀ représente -O-, -C(O)-, -C(O)-O- ou une liaison covalente ;
Y₃ représente un radical (C₁-C₆)alkyle ;
p' représente un entier de 0 à 4 ; ou un sel pharmaceutiquement acceptable de ce dernier.

18. Composé selon la revendication 17, **caractérisé en ce que** Z₃ représente le radical thiényle, furyle, benzofuryle, benzothiényle, thiazolyle, pyrazolyle, imidazolyle, pyridinyle, indolyle ; ou un sel pharmaceutiquement acceptable de ce dernier.

19. Composé selon la revendication 13, **caractérisé en ce que** Z₃ représente un radical (C₃-C₇)cycloalkyle ou aryle, le radical aryle étant éventuellement substitué par un ou plusieurs substituants identiques ou différents choisi parmi : nitro ou -(CH₂)_{p'}-V₃₁-Y₃ ;
V₃₁ représente -O-, -C(O)-, -C(O)-O- ou -SO₂- ;
Y₃ représente un radical (C₁-C₆)alkyle ;
p' représente un entier de 0 à 4 ; ou un sel pharmaceutiquement acceptable de ce dernier.

20. Composé selon la revendication 19, **caractérisé en ce que** le radical (C₃-C₇)cycloalkyle est choisi parmi cyclopentyle et cyclohexyle ; le radical aryle est le radical phényle ; ou un sel pharmaceutiquement acceptable de ce dernier.

21. Composé selon l'une des revendications 1 à 11, **caractérisé en ce que** R₃ représente -C(O)-Z'₃ ; ou un sel pharmaceutiquement acceptable de ce dernier.

22. Composé selon la revendication 21, **caractérisé en ce que** Z'₃ représente un radical phényle éventuellement substitué par un ou plusieurs substituants identiques ou différents de formule -(CH₂)ₚ"- V'₃-Y'₃ ;
V'₃ représente -O- ;
Y'₃ représente un radical (C₁-C₆)alkyle ;
p" représente un entier de 0 à 4 ; ou un sel pharmaceutiquement acceptable de ce dernier.

23. Composé selon l'une des revendications 1 à 11, **caractérisé en ce que** R₃ représente -C(O)-NH-Z"₃
Z"₃ représente un radical -(CH₂)_{q} A"₃ ;
A"₃ représente un radical (C₁-C₆)alkyle, phényle ou thiényle ;
les radicaux alkyle et aryle étant éventuellement substitués par un ou plusieurs substituants identiques ou différents de formule -V"₃-Y"₃ ;
V"₃ représente -O-, -C(O)-, -C(O)-O- ou une liaison covalente ;
Y"₃ représente l'atome d'hydrogène ou un radical (C₁-C₆)alkyle ;
q représente un entier de 0 à 1 ; ou un sel pharmaceutiquement acceptable de ce dernier.

24. Procédé de préparation d'un composé de formule (I) selon l'une des revendications précédentes **caractérisé en ce que** l'on traite le composé de formule générale : 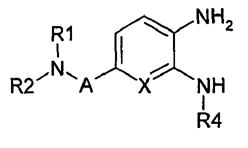 dans laquelle A, X, R₁, R₂, R₄ ont la signification indiquée dans la revendication 1,
i) soit par un aldéhyde de formule générale R₃CHO dans laquelle R₃ a la signification indiquée dans la revendication 1, en présence d'un oxydant ;
ii) soit par un chlorure d'acide de formule générale R₃COCl dans laquelle R₃ a la signification indiquée dans la revendication 1, en présence d'un acide ;
iii) soit par un acide carboxylique de formule générale R₃C(O)OH dans laquelle R₃ a la signification indiquée dans la revendication 1, en présence d'un agent de couplage suivi du traitement de l'amide ainsi formée par un acide ;
iv) soit par un dérivé chloroacétamide de formule générale Z"₃-NH-C(O)CH₂Cl dans laquelle Z"₃ a la signification indiquée ci-dessus, en présence d'une base tertiaire et de soufre.

25. Composition pharmaceutique contenant, à titre de principe actif, au moins un composé selon l'une des revendications 1 à 23, en association avec un support pharmaceutiquement acceptable.

26. Utilisation d'un composé selon l'une des revendications 1 à 23, pour la préparation d'un médicament pour le traitement des désordres pondéraux, des troubles mentaux, de la douleur neuropathique, des désordres de l'activité sexuelle.

27. Utilisation selon la revendication 26, pour la préparation d'un médicament pour le traitement des désordres pondéraux tels que l'obésité, la cachexie et l'anorexie.

28. Utilisation selon la revendication 26, pour la préparation d'un médicament pour le traitement des troubles mentaux tels que l'anxiété et la dépression

29. Utilisation selon la revendication 26, pour la préparation d'un médicament pour le traitement de la douleur neuropathique.

30. Utilisation selon la revendication 26, pour la préparation d'un médicament pour le traitement des désordres de l'activité sexuelle tels que les troubles de l'érection.

## Claims

1. Compound of general formula **(I)** 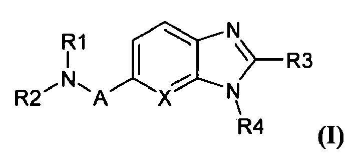 in racemic, enantiomeric form or any combinations of these forms and in which:
A represents -CH₂-, -C(O)-, -C(O)-C(Rₐ)(R_{b})-;
X represents the -CH- radical or the nitrogen atom;
Rₐ and R_{b} represent, independently, the hydrogen atom or a (C₁-C₆)alkyl radical;
R₁ represents the hydrogen atom or a (C₁-C₈)alkyl radical;
R₂ represents a (C₁-C₈)alkyl radical;
or R₁ and R₂ form together with the nitrogen atom to which they are attached, a heterobicycloalkyl or a heterocycloalkyl optionally substituted by one or more identical or different (C₁-C₆)alkyl substituents;
R₃ represents -(CH₂)ₚ-Z₃, -C(O)-Z'₃, -CH(OH)-Z'₃ or -C(O)-NH-Z"₃;
Z₃ represents a (C₁-C₆)alkyl, (C₂-C₆)alkenyl, (C₁-C₆)alkoxy, (C₁-C₆)alkylcarbonyl, (C₁-C₆)alkoxy-carbonyl, (C₁-C₆)alkyl-N(R_{N})-carbonyl, (C₃-C₇)cycloalkyl, heterocycloalkyl, aryl, aryl-thio or heteroaryl radical, Z₃ being linked to the -(CH₂)ₚ- radical by a carbon atom,
the (C₃-C₇) cycloalkyl and heterocycloalkyl radicals being optionally substituted by one or more identical or different radicals chosen from (C₁-C₆)alkyl and oxy;
the heteroaryl radical being optionally substituted by one or more identical or different substituents chosen from: halo, nitro or -(CH₂)_{p'}-V₃₀-Y₃;
the aryl radical being optionally substituted by one or more identical or different substituents chosen from: halo, nitro, cyano, (C₂-C₆)alkenyl, heterocycloalkyl, aryl, aryloxy, aralkyl-oxy, heteroaryl and -(CH₂)_{p'}-V₃₁ -Y₃ ;
V₃₀ represents -O-, -C(O)-, -C(O)-O- or a covalent bond;
V₃₁ represents -O-, -S-, -SO₂-, -C(O)-, -C(O)-O-, -N(R_{N})-, -NH-C(O)-, -C(O)-NR'₃-, -NH-C(O)-NR'₃- or a covalent bond;
Y₃ represents the hydrogen atom or a (C₁-C₆)alkyl radical optionally substituted by one or more identical or different halo radicals;
R_{N} represents a hydrogen atom or a (C₁-C₆)alkyl radical;
or Z₃ represents a radical of formula 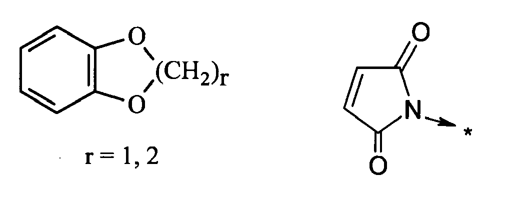 Z'₃ represents an aryl radical optionally substituted by one or more identical or different substituents chosen from: halo, nitro and -(CH₂)ₚ"-V'₃-Y'_{3;}
V'₃ represents -O-, -C(O)-, -C(O)-O-, -NH-C(O)-, -C(O)-NR'₃- or a covalent bond;
Y'₃ represents the hydrogen atom or a (C₁-C₆)alkyl radical optionally substituted by one or more identical or different halo radicals;
R'₃ represents the hydrogen atom, a (C₁-C₆)alkyl or (C₁-C₆)alkoxy radical;
Z"₃ represents the hydrogen atom or a -(CH₂)_{q}-A"₃ radical;
A"₃ represents a (C₁-C₆)alkyl, aryl or heteroaryl radical;
the alkyl and aryl radicals being optionally substituted by one or more identical or different substituents chosen from halo and -V"₃-Y"₃;
V"₃ represents -O-, -C(O)-, -C(O)-O-, -C(O)-NH- or a covalent bond;
Y"₃ represents the hydrogen atom or a (C₁-C₆)alkyl radical optionally substituted by one or more identical or different halo radicals;
p represents an integer from 0 to 6; p' and p" represent, independently, an integer from 0 to 4; q represents an integer from 0 to 2;
R₄ represents a radical of formula-(CH₂)ₛ-R'₄;
R'₄ represents a heterocycloalkyl containing at least one nitrogen atom and optionally substituted by (C₁-C₆)alkyl; a heteroaryl containing at least one nitrogen atom and optionally substituted by (C₁-C₆)alkyl; or a radical of formula -NW₄W'₄;
W₄ represents the hydrogen atom or (C₁-C₈)alkyl;
W'₄ represents a radical of formula -(CH₂)_{s'}-Z₄ in which Z₄ represents the hydrogen atom, a (C₁-C₈)alkyl or (C₃-C₇)cycloalkyl radical;
s and s' represent, independently, an integer from 0 to 6;
or a pharmaceutically acceptable salt of the latter.

2. Compound according to claim 1, **characterized in that** X represents the -CH- radical; or a pharmaceutically acceptable salt of the latter.

3. Compound according to one of the preceding claims, **characterized in that** R₁ represents the hydrogen atom or a (C₁-C₈)alkyl radical, and R₂ represents a (C₁-C₈)alkyl radical; or a pharmaceutically acceptable salt of the latter.

4. Compound according to one of claims 1 to 3, **characterized in that** R₁ represents a (C₁-C₆)alkyl radical; R₂ represents a (C₁-C₆)alkyl radical; or a pharmaceutically acceptable salt of the latter.

5. Compound according to one of claims 1 to 4, **characterized in that** A represents -CH₂-; or a pharmaceutically acceptable salt of the latter.

6. Compound according to one of claims 1 to 4, **characterized in that** A represents -C(O)-C(Rₐ)(R_{b})- and Rₐ and R_{b} represent, independently, a methyl radical; or a pharmaceutically acceptable salt of the latter.

7. Compound according to one of claims 1 to 4, **characterized in that** A represents -C(O)-; or a pharmaceutically acceptable salt of the latter.

8. Compound according to one of the preceding claims, **characterized in that** R₄ represents a radical of formula-(CH₂)ₛ-R'₄;
R'₄ represents a heterocycloalkyl containing at least one nitrogen atom and optionally substituted by (C₁-C₆)alkyl; or a radical of formula -NW₄W'₄;
W₄ represents the hydrogen atom or (C₁-C₈)alkyl;
W'₄ represents a radical of formula -(CH₂)_{s'}-Z₄ in which Z₄ represents the hydrogen atom or a (C₁-C₈)alkyl radical;
s and s' represent, independently, an integer from 1 to 6; or a pharmaceutically acceptable salt of the latter.

9. Compound according to one of the preceding claims, **characterized in that** R₄ represents a radical of formula-(CH₂)ₛ-R'₄;
R'₄ represents a radical of formula -NW₄W'₄;
W₄ represents a (C₁-C₈)alkyl radical;
W'₄ represents a radical of formula -(CH₂)_{s'}-Z₄ in which Z₄ represents the hydrogen atom or a (C₁-C₈)alkyl radical;
s and s' represent, independently, an integer from 2 to 6; or a pharmaceutically acceptable salt of the latter.

10. Compound according to one of claims 1 to 8, **characterized in that** R₄ represents a radical of formula-(CH₂)ₛ- R'₄;
R'₄ represents a heterocycloalkyl containing at least one nitrogen atom and optionally substituted by (C₁-C₆)alkyl; and s represents an integer from 2 to 6; or a pharmaceutically acceptable salt of the latter.

11. Compound according to claim 10, **characterized in that** R'₄ represents the piperidine or pyrrolidine ring; s represents an integer from 1 to 4; or a pharmaceutically acceptable salt of the latter.

12. Compound according to one of the preceding claims, **characterized in that** R₃ represents -(CH₂)ₚ-Z₃ and
Z₃ represents a (C₁-C₆)alkyl, (C₂-C₆)alkenyl, (C₁-C₆)alkoxy, (C₁-C₆)alkylcarbonyl, (C₁-C₆)alkoxy-carbonyl, (C₁-C₆)alkyl-N(R_{N})-carbonyl, (C₃-C₇)cycloalkyl, heterocycloalkyl, aryl, aryl-thio or heteroaryl radical,
the (C₃-C₇) cycloalkyl and heterocycloalkyl radicals being optionally substituted by one or more identical or different radicals chosen from (C₁-C₆)alkyl and oxy;
the heteroaryl radical being optionally substituted by one or more identical or different substituents chosen from: halo, nitro or -(CH₂)_{p'}-V₃₀-Y_{3;}
the aryl radical being optionally substituted by one or more identical or different substituents chosen from: halo, nitro, (C₂-C₆)alkenyl, heterocycloalkyl, aryl, aryloxy, aralkyl-oxy, heteroaryl and -(CH₂)ₚ'-V₃₁-Y₃;
V₃₀ represents -O-, -C(O)-, -C(O)-O- or a covalent bond;
V₃₁ represents -O-, -S-, -SO₂-, -C(O)-, -C(O)-O-, -N(R_{N})-, -NH-C(O)-, -C(O)-NR'₃- or a covalent bond;
Y₃ represents the hydrogen atom or a (C₁-C₆)alkyl radical optionally substituted by one or more identical or different halo radicals;
R_{N} represents a hydrogen atom or a (C₁-C₆)alkyl radical;
or Z₃ represents a radical of formula 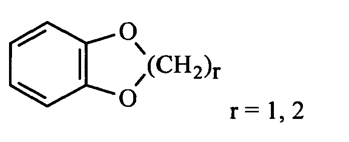 or a pharmaceutically acceptable salt of the latter.

13. Compound according to one of the preceding claims, **characterized in that**
Z₃ represents a (C₁-C₆)alkyl radical, (C₁-C₆)alkyl-carbonyl, (C₁-C₆)alkoxycarbonyl, (C₁-C₆)alkyl-N(R_{N})-carbonyl, (C₃-C₇)cycloalkyl, aryl or heteroaryl radical, the aryl and heteroaryl radicals being optionally substituted; or a pharmaceutically acceptable salt of the latter.

14. Compound according to claim 13, **characterized in that**
the heteroaryl radical is optionally substituted by one or more identical or different substituents chosen from: halo and -(CH₂)_{p'}-V₃₀-Y₃;
the aryl radical is optionally substituted by one or more identical or different substituents chosen from: nitro and -(CH₂)_{p'}-V₃₁ -Y₃;
V₃₀ represents -O-, -C(O)-, -C(O)-O- or a covalent bond;
V₃₁ represents -O-, -C(O)-, -C(O)-O- or -SO₂-;
Y₃ represents a (C₁-C₆)alkyl radical;
p and p' represent, independently, an integer from 0 to 4; or a pharmaceutically acceptable salt of the latter.

15. Compound according to claim 13, **characterized in that** Z₃ represents a (C₁-C₆)alkyl radical; or a pharmaceutically acceptable salt of the latter.

16. Compound according to claim 13, **characterized in that** Z₃ represents a (C₁-C₆)alkylcarbonyl, (C₁-C₆)alkoxy-carbonyl or (C₁-C₆)alkyl-N(R_{N})-carbonyl radical; or a pharmaceutically acceptable salt of the latter.

17. Compound according to claim 13, **characterized in that** Z₃ represents a heteroaryl optionally substituted by one or more identical or different substituents chosen from: halo and -(CH₂)_{p'}-V₃₀-Y₃;
V₃₀ represents -O-, -C(O)-, -C(O)-O- or a covalent bond;
Y₃ represents a (C₁-C₆)alkyl radical;
p' represents an integer from 0 to 4; or a pharmaceutically acceptable salt of the latter.

18. Compound according to claim 17, **characterized in that** Z₃ represents the thienyl, furyl, benzofuryl, benzothienyl, thiazolyl, pyrazolyl, imidazolyl, pyridinyl, indolyl radical; or a pharmaceutically acceptable salt of the latter.

19. Compound according to claim 13, **characterized in that** Z₃ represents a (C₃-C₇)cycloalkyl or aryl radical, the aryl radical being optionally substituted by one or more identical or different substituents chosen from: nitro or -(CH₂)_{p'}-V₃₁ -Y₃ ;
V₃₁ represents -O-, -C(O)-, -C(O)-O- or -SO₂-;
Y₃ represents a (C₁-C₆)alkyl radical;
p' represents an integer from 0 to 4; or a pharmaceutically acceptable salt of the latter.

20. Compound according to claim 19, **characterized in that** the (C₃-C₇)cycloalkyl radical is chosen from cyclopentyl and cyclohexyl; the aryl radical is the phenyl radical; or a pharmaceutically acceptable salt of the latter.

21. Compound according to one of claims 1 to 11, **characterized in that** R₃ represents -C(O)-Z'₃; or a pharmaceutically acceptable salt of the latter.

22. Compound according to claim 21, **characterized in that** Z'₃ represents a phenyl radical optionally substituted by one or more identical or different substituents of formula -(CH₂)ₚ"- V'₃-Y'₃;
V'₃ represents -O-;
Y'₃ represents a (C₁-C₆)alkyl radical;
p" represents an integer from 0 to 4; or a pharmaceutically acceptable salt of the latter.

23. Compound according to one of claims 1 to 11, **characterized in that** R₃ represents -C(O)-NH-Z"₃
Z"₃ represents a -(CH₂)_{q}-A"₃ radical;
A"₃ represents a (C₁-C₆)alkyl, phenyl or thienyl radical;
the alkyl and aryl radicals being optionally substituted by one or more identical or different substituents of formula -V"₃-Y"₃;
V"₃ represents -O-, -C(O)-, -C(O)-O- or a covalent bond;
Y"₃ represents the hydrogen atom or a (C₁-C₆)alkyl radical;
q represents an integer from 0 to 1; or a pharmaceutically acceptable salt of the latter.

24. Process for the preparation of a compound of formula (I) according to one of the preceding claims **characterized in that** the compound of general formula: 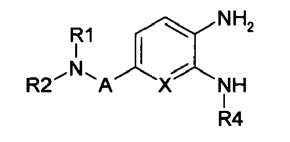 in which A, X, R₁, R₂, R₄ have the meaning indicated in claim 1, is treated
i) either by an aldehyde of general formula R₃CHO in which R₃ has the meaning indicated in claim 1, in the presence of an oxidizing agent ;
ii) or by an acid chloride of general formula R₃COCl in which R₃ has the meaning indicated in claim 1, in the presence of an acid;
iii) or by a carboxylic acid of general formula R₃C(O)OH in which R₃ has the meaning indicated in claim 1, in the presence of a coupling agent followed by treatment of the amide thus formed by an acid;
iv) or by a chloroacetamide derivative of general formula Z"₃-NH-C(O)CH₂Cl in which Z"₃ has the meaning indicated above, in the presence of a tertiary base and sulphur.

25. Pharmaceutical composition containing, as active ingredient, at least one compound according to one of claims 1 to 23, in combination with a pharmaceutically acceptable support.

26. Use of a compound according to one of claims 1 to 23, for the preparation of a medicament for the treatment of weight disorders, mental disorders, neuropathic pain, sexual activity disorders.

27. Use according to claim 26, for the preparation of a medicament for the treatment of weight disorders such as obesity, cachexia and anorexia.

28. Use according to claim 26, for the preparation of a medicament for the treatment of mental disorders such as anxiety and depression

29. Use according to claim 26, for the preparation of a medicament for the treatment of neuropathic pain.

30. Use according to claim 26, for the preparation of a medicament for the treatment of sexual activity disorders such as erectile disorders.

## Patentansprüche

1. Verbindung der allgemeinen Formel (I) 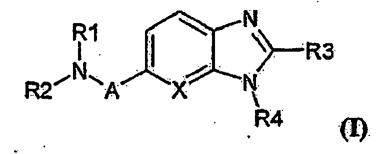 in racemischer Form, in enantiomerer Form oder in allen Kombinationen dieser Formen, wobei:
A -CH₂-, -C (O) -, -C(O)-C(Rₐ)(R_{b})- darstellt;
X den Rest -CH- oder ein Stickstoffatom darstellt;
Rₐ und R_{b} unabhängig ein Wasserstoffatom oder einen (C₁-C₆)-Alkyl-Rest darstellen;
R₁ ein Wasserstoffatom oder einen (C₁-C₈)-Alkyl-Rest darstellt;
R₂ einen (C₁-C₈) -Alkyl-Rest darstellt;
oder R₁ und R₂ zusammen mit dem Stickstoffatom, an das sie gebunden sind, ein Heterobicycloalkyl oder ein Heterocycloalkyl bilden, das gegebenenfalls mit einem oder mehreren (C₁-C₆)-Alkyl-Substituenten, die gleich oder verschieden sind, substituiert ist;
R₃ - (CH₂) p-Z₃, -C(O)-Z'₃, -CH(OH)-Z'₃ oder -C(O)-NH-Z"₃ darstellt;
Z₃ einen Rest -(C₁-C₆)-Alkyl, (C₂-C₆) -Alkenyl, (C₁-C₆)-Alkoxy, (C₁-C₆) -Alkylcarbonyl, (C₁-C₆)-Alkoxycarbonyl, (C₁-C₆) -Alkyl-N (R_{N}) -carbonyl, (C₃-C₇)-Cycloalkyl, Heterocycloalkyl, Aryl, Arylthio oder Heteroaryl darstellt, wobei Z₃ durch ein Kohlenstoffatom an den Rest -(CH₂)ₚ-gebunden ist,
die Reste (C₃-C₇)-Cycloalkyl und -Heterocycloalkyl gegebenenfalls mit einem oder mehreren gleichen oder verschiedenen Resten substituiert sind, ausgewählt aus (C₁-C₆)-Alkyl und Oxy;
der Rest Heteroaryl gegebenenfalls mit einem oder mehreren gleichen oder verschiedenen Substituenten substituiert ist, ausgewählt aus: Halogen, Nitro und -(CH₂)_{p'}-V₃₀-Y₃ ;
der Rest Aryl gegebenenfalls mit einem oder mehreren gleichen oder verschiedenen Substituenten substituiert ist, ausgewählt aus: Halogen, Nitro, Cyano, (C₂-C₆)-Alkenyl, Heterocycloalkyl, Aryl, Aryloxy, Aralkyloxy, Heteroaryl und -(CH₂)_{p'}-V₃₁-Y₃;
V₃₀ -O-, -C(O)-, -C(O)-O- oder eine kovalente Bindung darstellt;
V₃₁ -O-, -S-, -SO₂-, -C(O)-, -C (O) -O-, -N(R_{N})-, -NH-C (O) -, -C(O)-NR'₃-, -NH-C(O)-NR'₃ oder eine kovalente Bindung darstellt;
Y₃ ein Wasserstoffatom oder einen (C₁-C₆)-Alkyl-Rest, der gegebenenfalls mit einem oder mehreren gleichen oder verschiedenen Halogen-Resten substituiert ist, darstellt;
R_{N} ein Wasserstoffatom oder einen (C₁-C₆)-Alkyl-Rest darstellt;
oder Z₃ einen Rest der Formel 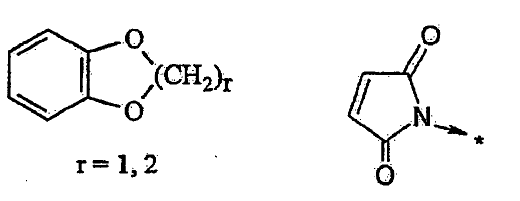 darstellt;
Z'₃ einen Aryl-Rest darstellt, der gegebenenfalls mit einem oder mehreren gleichen oder verschiedenen Substituenten substituiert ist, ausgewählt: Halogen, Nitro und -(CH₂)_{p"}-V'₃-Y'₃;
V'₃ -O-, -C(O)-, -C(O)-O-, -NH-C(O)-, -C((O)-NR'₃ oder eine kovalente Bindung darstellt;
Y'₃ ein Wasserstoffatom oder einen (C₁-C₆)-Alkyl-Rest, der gegebenenfalls mit einem oder mehreren gleichen oder verschiedenen Halogen-Resten substituiert ist, darstellt;
R'₃ ein Wasserstoffatom, einen (C₁-C₆)-Alkyl- oder (C₁-C₆)-Alkoxy-Rest darstellt;
Z"₃ ein Wasserstoffatom oder einen Rest -(CH₂)q-A"₃ darstellt;
A"₃ einen Rest (C₁-C₆)-Alkyl, Aryl oder Heteroaryl darstellt;
die Reste Alkyl und Aryl gegebenenfalls mit einem oder mehreren gleichen oder verschiedenen Substituenten substituiert sind, ausgewählt aus Halogen und -V"₃-Y"₃;
V"₃ -O-, -C (O) -, -C(O)-O-, -C(O)-NH- oder eine kovalente Bindung darstellt;
Y"₃ ein Wasserstoffatom oder einen (C₁-C₆)-Alkyl-Rest, der gegebenenfalls mit einem oder mehreren gleichen oder verschiedenen Halogen-Resten substituiert ist, darstellt;
p eine ganze Zahl von 0 bis 6 darstellt; p' und p" unabhängig eine ganze Zahl von 0 bis 4 darstellen; q eine ganze Zahl von 0 bis 2 darstellt;
R₄ einen Rest der Formel (CH₂)ₛ-R'₄ darstellt;
R'₄ ein Heterocycloalkyl, das wenigstens ein Stickstoffatom enthält und gegebenenfalls mit (C₁-C₆)-Alkyl substituiert ist; ein Heteroaryl, das wenigstens ein Stickstoffatom enthält und gegebenenfalls mit (C₁-C₆)-Alkyl substituiert ist; oder einen Rest der Formel -NW₄W'₄ darstellt;
W₄ ein Wasserstoffatom oder (C₁-C₈)-Alkyl darstellt;
W'₄ einen Rest der Formel -(CH₂)_{s'}-Z₄ darstellt, worin Z₄ ein Wasserstoffatom, einen Rest (C₁-C₈)-Alkyl oder (C₃-C₇)-Cycloalkyl darstellt;
s und s' unabhängig eine ganze Zahl von 0 bis 6 darstellen;
oder ein pharmazeutisch annehmbares Salz dieser Verbindung.

2. Verbindung nach Anspruch 1, **dadurch gekennzeichnet, dass** X den Rest -CH- darstellt; oder ein pharmazeutisch annehmbares Salz dieser Verbindung.

3. Verbindung nach einem der vorangehenden Ansprüche, **dadurch gekennzeichnet, dass** R₁ ein Wasserstoffatom oder einen (C₁-C₈) -Alkyl-Rest darstellt und R₂ einen (C₁-C₈) -Alkyl-Rest darstellt; oder ein pharmazeutisch annehmbares Salz dieser Verbindung.

4. Verbindung nach einem der Ansprüche 1 bis 3, **dadurch gekennzeichnet, dass** R₁ einen (C₁-C₆)-Alkyl-Rest darstellt; R₂ einen (C₁-C₆)-Alkyl-Rest darstellt; oder ein pharmazeutisch annehmbares Salz dieser Verbindung.

5. Verbindung nach einem der Ansprüche 1 bis 4, **dadurch gekennzeichnet, dass** A -CH₂- darstellt; oder ein pharmazeutisch annehmbares Salz dieser Verbindung.

6. Verbindung nach einem der Ansprüche 1 bis 4, **dadurch gekennzeichnet, dass** A -C(O)-C(Rₐ)(R_{b}) - darstellt und Rₐ und R_{b} unabhängig einen Methyl-Rest darstellen; oder ein pharmazeutisch annehmbares Salz dieser Verbindung.

7. Verbindung nach einem der Ansprüche 1 bis 4, **dadurch gekennzeichnet, dass** A -C(O)- darstellt; oder ein pharmazeutisch annehmbares Salz dieser Verbindung.

8. Verbindung nach einem der vorangehenden Ansprüche, **dadurch gekennzeichnet, dass**
R₄ einen Rest der Formel -(CH₂)ₛ-R'₄ darstellt;
R'₄ ein Heterocycloalkyl, das wenigstens ein Stickstoffatom enthält und gegebenenfalls mit (C₁-C₆)-Alkyl substituiert ist, oder einen Rest der Formel -NW₄W'₄ darstellt;
W₄ ein Wasserstoffatom oder (C₁-C₈)-Alkyl darstellt;
W'₄ einen Rest der Formel -(CH₂)_{s'}Z₄ darstellt, in der Z₄ ein Wasserstoffatom oder einen (C₁-C₈)-Alkyl-Rest darstellt;
s und s' unabhängig eine ganze Zahl von 1 bis 6 darstellen; oder ein pharmazeutisch annehmbares Salz dieser Verbindung.

9. Verbindung nach einem der vorangehenden Ansprüche, **dadurch gekennzeichnet, dass** R₄ einen Rest der Formel -(CH₂)ₛ-R'₄ darstellt;
R'₄ einen Rest der Formel -NW₄W'₄ darstellt;
W₄ einen (C₁-C₈)-Alkyl-Rest darstellt;
W'₄ einen Rest der Formel -(CH₂)_{s'}-Z₄ darstellt, in der Z₄ ein Wasserstoffatom oder einen (C₁-C₈)-Alkyl-Rest darstellt;
s und s' unabhängig eine ganze Zahl von 2 bis 6 darstellen; oder ein pharmazeutisch annehmbares Salz dieser Verbindung.

10. Verbindung nach einem der Ansprüche 1 bis 8, **dadurch gekennzeichnet, dass** R₄ einen Rest der Formel -(CH₂)ₛ-R'₄ darstellt;
R'₄ ein Heterocycloalkyl, das wenigstens ein Stickstoffatom enthält und gegebenenfalls mit (C₁-C₆)-Alkyl substituiert ist, darstellt und s eine ganze Zahl von 2 bis 6 darstellt; oder ein pharmazeutisch annehmbares Salz dieser Verbindung.

11. Verbindung nach Anspruch 10, **dadurch gekennzeichnet, dass** R'₄ einen Piperidin- oder Pyrrolidin-Ring darstellt; s eine ganze Zahl von 1 bis 4 darstellt; oder ein pharmazeutisch annehmbares Salz dieser Verbindung.

12. Verbindung nach einem der vorangehenden Ansprüche, **dadurch gekennzeichnet, dass** R₃ -(CH₂)ₚ-Z₃ darstellt und
Z₃ einen Rest (C₁-C₆) -Alkyl, (C₂-C₆)-Alkenyl, (C₁-C₆)-Alkoxy, (C₁-C₆)-Alkylcarbonyl, (C₁-C₆)-Alkoxycarbonyl, (C₁-C₆)-Alkyl-N(R_{N})-carbonyl, (C₃-C₇)-Cycloalkyl, Heterocycloalkyl, Aryl, Arylthio oder Heteroaryl darstellt;
die Reste (C₃-C₇)-Cycloalkyl und Heterocycloalkyl gegebenenfalls mit einem oder mehreren gleichen oder verschiedenen Substituenten substituiert sind, ausgewählt aus (C₁-C₆)-Alkyl und Oxy;
der Rest Heteroaryl gegebenenfalls mit einem oder mehreren gleichen oder verschiedenen Substituenten substituiert ist, ausgewählt aus: Halogen, Nitro und -(CH₂)_{p'}-V₃₀-Y₃;
der Rest Aryl gegebenenfalls mit einem oder mehreren gleichen oder verschiedenen Substituenten substituiert ist, ausgewählt aus: Halogen, Nitro, (C₂-C₆)-Alkenyl, Heterocycloalkyl, Aryl, Aryloxy, Aralkyloxy, Heteroaryl und (CH₂)_{p'}-V₃₁-Y₃;
V₃₀ -O-, -C(O)-, -C(O)-O- oder eine kovalente Bindung darstellt;
V₃₁ -O-, -S-, -SO₂-, -C(O)-, -C(O)-O-, -N(R_{N})-, -NH-C(O)-, -C(O)-NR'₃- oder eine kovalente Bindung darstellt;
Y₃ ein Wasserstoffatom oder einen (C₁-C₆) -Alkyl-Rest, der gegebenenfalls mit einem oder mehreren gleichen oder verschiedenen Halogenresten substituiert ist, darstellt; R_{N} ein Wasserstoffatom oder einen (C₁-C₆)-Alkyl-Rest darstellt;
oder Z₃ einen Rest der Formel 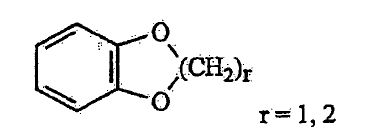 darstellt;
oder ein pharmazeutisch annehmbares Salz dieser Verbindung.

13. Verbindung nach einem der vorangehenden Ansprüche, **dadurch gekennzeichnet, dass**
Z₃ einen Rest (C₁-C₆)-Alkyl, (C₁-C₆) -Alkylcarbonyl, (C₁-C₆) -Alkoxycarbonyl, (C₁-C₆)-Alkyl-N(R_{N})-carbonyl, (C₃-C₇)-Cycloalkyl, Aryl oder Heteroaryl darstellt, wobei die Reste Aryl und Heteroaryl gegebenenfalls substituiert sind; oder ein pharmazeutisch annehmbares Salz dieser Verbindung.

14. Verbindung nach Anspruch 13, **dadurch gekennzeichnet, dass**
der Heteroaryl-Rest gegebenenfalls mit einem oder mehreren gleichen oder verschiedenen Substituenten substituiert ist, ausgewählt aus: Halogen und -(CH₂)_{p'}-V₃₀-Y₃;
der Rest Aryl gegebenenfalls mit einem oder mehreren gleichen oder verschiedenen Substituenten substituiert ist, ausgewählt aus: Nitro und -(CH₂)_{p'}-V₃₁-Y₃;
V₃₀ -O-, -C(O)-, -C(O)-O- oder eine kovalente Bindung darstellt;
V₃₁ -O-, -C(O)-, -C(O)-O- oder -SO₂- darstellt;
Y₃ einen (C₁-C₆) -Alkyl-Rest darstellt;
p und p' unabhängig eine ganze Zahl von 0 bis 4 darstellen; oder ein pharmazeutisch annehmbares Salz dieser Verbindung.

15. Verbindung nach Anspruch 13, **dadurch gekennzeichnet, dass** Z₃ einen (C₁-C₆)-Alkyl-Rest darstellt; oder ein pharmazeutisch annehmbares Salz dieser Verbindung.

16. Verbindung nach Anspruch 13, **dadurch gekennzeichnet, dass** Z₃ einen Rest (C₁-C₆)-Alkylcarbonyl, (C₁-C₆)-Alkoxycarbonyl oder (C₁-C₆)-Alkyl-N(R_{N})-carbonyl darstellt; oder ein pharmazeutisch annehmbares Salz dieser Verbindung.

17. Verbindung nach Anspruch 13, **dadurch gekennzeichnet, dass** Z₃ ein Heteroaryl darstellt, das gegebenenfalls mit einem oder mehreren gleichen oder verschiedenen Substituenten substituiert ist, ausgewählt aus: Halogen und -(CH₂)_{p'}-V₃₀-Y₃;
V₃₀ -O-, -C(O)-, -C(O)-O- oder eine kovalente Bindung darstellt;
Y₃ einen (C₁-C₆)-Alkyl-Rest darstellt;
p' eine ganze Zahl von 0 bis 4 darstellt; oder ein pharmazeutisch annehmbares Salz dieser Verbindung.

18. Verbindung nach Anspruch 17, **dadurch gekennzeichnet, dass** Z₃ den Rest Thienyl, Furyl, Benzofuryl, Benzothienyl, Thiazolyl, Pyrazolyl, Imidazolyl, Pyridinyl, Indolyl darstellt; oder ein pharmazeutisch annehmbares Salz dieser Verbindung.

19. Verbindung nach Anspruch 13, **dadurch gekennzeichnet, dass** Z₃ einen (C₃-C₇)-Cycloalkyl- oder Aryl-Rest darstellt, wobei der Aryl-Rest gegebenenfalls mit einem oder mehreren gleichen oder verschiedenen Substituenten substituiert ist, ausgewählt aus: Nitro und -(CH₂)_{p'}-V₃₁-Y₃;
V₃₁ -O-, -C (O) -, -C (O) -O oder -SO₂ darstellt;
Y₃ einen (C₁-C₆)-Alkyl-Rest darstellt;
p' eine ganze Zahl von 0 bis 4 darstellt; oder ein pharmazeutisch annehmbares Salz dieser Verbindung.

20. Verbindung nach Anspruch 19, **dadurch gekennzeichnet, dass** der Rest (C₃-C₇)-Cycloalkyl ausgewählt ist aus Cyclopentyl und Cyclohexyl; der Aryl-Rest der Phenyl-Rest ist; oder ein pharmazeutisch annehmbares Salz dieser Verbindung.

21. Verbindung nach einem der Ansprüche 1 bis 11, **dadurch gekennzeichnet, dass** R₃-C(O)-Z'₃ darstellt; oder ein pharmazeutisch annehmbares Salz dieser Verbindung.

22. Verbindung nach Anspruch 21, **dadurch gekennzeichnet, dass** Z'₃ einen Phenyl-Rest darstellt, der gegebenenfalls mit einem oder mehreren gleichen oder verschiedenen Substituenten der Formel -(CH₂)ₚ"-V'₃-Y'₃ substituiert ist;
V'₃ -O-darstellt;
Y'₃ einen (C₁-C₆)-Alkyl-Rest darstellt;
p" eine ganze Zahl von 0 bis 4 darstellt; oder ein pharmazeutisch annehmbares Salz dieser Verbindung.

23. Verbindung nach einem der Ansprüche 1 bis 11, **dadurch gekennzeichnet, dass** R₃ -C(O)-NH-Z" darstellt
Z"₃ einen Rest -(CH₂)q-A"₃ darstellt;
A"₃ einen Rest (C₁-C₆)-Alkyl, Phenyl oder Thienyl darstellt;
die Reste Alkyl oder Aryl gegebenenfalls mit einem oder mehreren gleichen oder verschiedenen Substituenten der Formel -V"₃-Y"₃ substituiert sind;
V"₃ -O-, -C(O)-, -C(O)-O- oder eine kovalente Bindung darstellt;
Y"₃ ein Wasserstoffatom oder einen (C₁-C₆)-Alkyl-Rest darstellt;
q eine ganze Zahl von 0 bis 1 darstellt; oder ein pharmazeutisch annehmbares Salz dieser Verbindung.

24. Verfahren zur Herstellung einer Verbindung der Formel (I) nach einem der vorangehenden Ansprüche, **dadurch gekennzeichnet, dass** man die Verbindung der allgemeinen Formel: 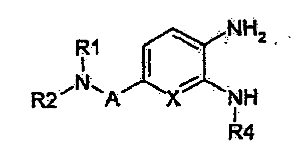 in der A, X, R₁, R₂, R₄ die in Anspruch 1 angegebene Bedeutung haben,
i) entweder mit einem Aldehyd der allgemeinen Formel R₃CHO, in der R₃ die in Anspruch 1 angegebene Bedeutung hat, in Gegenwart eines Oxidationsmittels;
ii) oder mit einem Säurechlorid der allgemeinen Formel R₃COCl, in der R₃ die in Anspruch 1 angegebene Bedeutung hat, in Gegenwart einer Säure;
iii) oder mit einer Carbonsäure der allgemeinen Formel R₃C(O)OH, in der R₃ die in Anspruch 1 angegebene Bedeutung hat, in Gegenwart eines Kupplungsmittels behandelt, gefolgt von der Behandlung des so gebildeten Amids mit einer Säure;
iv) oder mit einem Chloracetamid der allgemeinen Formel Z"₃-NH-C(O)CH₂Cl, in der Z"₃ die oben angegebene Bedeutung hat, in Gegenwart einer tertiären Base und Schwefel behandelt.

25. Pharmazeutische Zusammensetzung, die als Wirkstoff wenigstens eine Verbindung nach einem der Ansprüche 1 bis 23 in Kombination mit einem pharmazeutisch annehmbaren Träger enthält.

26. Verwendung einer Verbindung nach einem der Ansprüche 1 bis 23 für die Herstellung eines Medikaments für die Behandlung von Gewichtsstörungen, mentalen Störungen, neuropathischem Schmerz, Störungen der sexuellen Aktivität.

27. Verwendung nach Anspruch 26 für die Herstellung eines Medikaments zur Behandlung von Gewichtsstörungen wie Adipositas, Kachexie und Anorexie.

28. Verwendung nach Anspruch 26 für die Herstellung eines Medikaments zur Behandlung von mentalen Störungen wie Angst und Depression.

29. Verwendung nach Anspruch 26 für die Herstellung eines Medikaments zur Behandlung von neuropathischem Schmerz.

30. Verwendung nach Anspruch 26 für die Herstellung eines Medikaments zur Behandlung von Störungen der sexuellen Aktivität wie Erektionsstörungen.
